(19) Europäisches Patentamt
European Patent Office
Office européen des brevets

(11) **EP 3 825 736 B1**

(12) **EUROPÄISCHE PATENTSCHRIFT**

(45) Veröffentlichungstag und Bekanntmachung des Hinweises auf die Patenterteilung:
**06.08.2025 Patentblatt 2025/32**

(21) Anmeldenummer: **20209321.7**

(22) Anmeldetag: **23.11.2020**

(51) Internationale Patentklassifikation (IPC):
**G01T 1/29** (2006.01)

(52) Gemeinsame Patentklassifikation (CPC):
**G01T 1/2907**

(54) **BILDGEBENDES DETEKTORSYSTEM FÜR GAMMASTRAHLUNG UNTER NUTZUNG VON UNI- UND BIDIREKTIONALEN COMPTON-STREUPROZESSEN**

IMAGING DETECTOR SYSTEM FOR GAMMA RADIATION USING UNIDIRECTIONAL AND BIDIRECTIONAL COMPTON SCATTERING PROCESSES

SYSTÈME DE DÉTECTEUR IMAGEUR DESTINÉ AU RAYONNEMENT GAMMA À L'AIDE DES PROCESSUS DE DIFFUSION COMPTON UNI ET BIDIRECTIONNELS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **22.11.2019 DE 102019131695**
**22.11.2019 DE 102019131696**

(43) Veröffentlichungstag der Anmeldung:
**26.05.2021 Patentblatt 2021/21**

(73) Patentinhaber: **Hellma Materials GmbH**
**07745 Jena (DE)**

(72) Erfinder: **PETRAK, Sibylle**
**07743 Jena (DE)**

(74) Vertreter: **Fritzsche, Thomas**
**Fritzsche Patent**
**Naupliastraße 110**
**81545 München (DE)**

(56) Entgegenhaltungen:
**US-A1- 2009 256 080**

• **GUEORGUIEV A ET AL: "A novel method to determine the directionality of radiation sources with two detectors based on coincidence measurements", NUCLEAR SCIENCE SYMPOSIUM CONFERENCE RECORD (NSS/ MIC), 2010 IEEE, IEEE, 30 October 2010 (2010-10-30), pages 1525 - 1530, XP032054306, ISBN: 978-1-4244-9106-3, DOI: 10.1109/ NSSMIC.2010.5874031**

## EP 3 825 736 B1

**Beschreibung**

Die Erfindung betrifft ein System sowie ein Verfahren zur Strahlungsdetektion, insbesondere ein bildgebendes Detektorsystem zur Erstellung von tomographischen Schnittbildern von Aktivitätsverteilungen und zur Richtungsmessung von Strahlungsquellen. Das System und das Verfahren sind im Nah- und Fernfeld eines Quellvolumens einsetzbar, von dem die Strahlung ausgeht. Insbesondere können Aktivitätsverteilungen von Radiopharmaka in einem Patienten bestimmt werden. Das erfindungsgemäße System ermöglicht auch das Aufspüren von Gammastrahlungsquellen im Gelände durch Einsatzkräfte des ABC- und Strahlenschutzes sowie das Auffinden von Gammastrahlungsquellen kosmischen Ursprungs, wie sie in der Astronomie mit Gammastrahlen-Teleskopen beobachtet werden.

Auf dem Gebiet der Strahlungsdetektion ist es häufig von Interesse, die räumliche Verteilung einer Aktivitätsanreicherung in einem Quellvolumen zu bestimmen. Je nach Anwendungsbereich sind die Anforderungen an die Messsysteme und Verfahren sehr unterschiedlich. Die physikalischen Prinzipien, von denen die Messsysteme Gebrauch machen, können aber identisch sein.

Ein häufig eingesetztes richtungsabhängiges Detektionsprinzip ist die Compton-Streuung von Gammastrahlung an einem Elektron im Detektormaterial. Entsprechende Geräte und Systeme hierzu werden als Compton Kameras oder Compton Teleskope bezeichnet.

Compton Kameras und Compton Teleskope sind aus Anordnungen mehrerer Strahlungsdetektoren zusammengesetzt. Es werden Koinzidenzen von jeweils zwei Detektoren aufgezeichnet, wenn beide Detektoren gleichzeitig einen Energieeintrag aufweisen. Die Koinzidenz-Methode nutzt die Gleichzeitigkeit zweier Ionisationsprozesse aus, welche von dem Rückstoß-Elektron und - räumlich dazu versetzt - von der gestreuten Strahlung ausgehen, die einen zweiten Detektor ionisieren kann.

Compton Teleskope sind seit ca. 50 Jahren in der Gammaastronomie weit verbreitet. Die erstmalige Nutzung eines Compton Teleskops geht auf Schönfelder zurück [Nuclear Instruments & Methods 107 (1973) 385]. Der Erfolg dieser Technologie wird durch die vielen Ballon-Experimente und Satelliten demonstriert, die kosmische Gammastrahlungsquellen mit Compton Teleskopen erkundet haben und weiterhin erkunden.

1974 haben Todd, Nightingale und Everett vorgeschlagen, Compton Kameras in der Nuklearmedizin einzusetzen [Nature 251 (1974) 5471]. In medizinischen Anwendungen besteht eine besondere Herausforderung darin, dass sich der Patient in einem endlichen Abstand vor bzw. sogar in der Compton Kamera befindet, was einen Parallaxenfehler erzeugt und erhöhte Anforderungen an die Bildrekonstruktion stellt.

Im klinischen Bereich stehen Compton Kameras im Wettbewerb mit SPECT (Single Photon Emission Computed Tomography) und PET (Positron Emission Tomography), welche die beiden am weitesten verbreiteten Bildgebungstechniken in der Nuklearmedizin sind.

Die Vor- und Nachteile von SPECT und PET sind in der Fachliteratur ausführlich dargestellt worden und sollen hier nicht weiter erörtert werden. Es wird jedoch allgemein anerkannt, dass eine erhebliche Beschränkung von SPECT die sehr niedrige Nachweiseffizienz aufgrund der Kollimatoren ist. Außerdem gibt es eine Beschränkung im Energiebereich von SPECT Scannern. SPECT wird häufig in Verbindung mit dem Radionuklid Tc-99m genutzt, dessen Gammaenergie 141 keV beträgt. Für höherenergetische Nuklide ist SPECT nicht geeignet. Aus medizinischer Sicht ist aber der Energiebereich bis 640 keV interessant. Für den Energiebereich oberhalb von 200 keV gibt es derzeit keinen geeigneten Emissionstomographen für Single Photon Gammastrahlung.

Ein drittes Einsatzgebiet für Compton Kameras ist der ABC- und Strahlenschutz. Hier besteht die Aufgabe darin, versteckte oder verbotene radioaktive Quellen aufzuspüren. Es sollen Strahlungsquellen geortet werden, wenn z.B. größere Infrastrukturanlagen, Großveranstaltungen, Personen- und Güterbewegungen an Flughäfen, Grenzen und Bahnhöfen auf Strahlungsquellen überwacht werden. Sicherheitskräfte im ABC-Schutz benötigen Geräte, um radiologische und nukleare Gefahrenstoffe zu lokalisieren.

Es soll an dieser Stelle ein kurzer Überblick zu den Bauformen für Compton Kameras nach dem Stand der Technik gegeben werden. Bekannte Bauformen für Compton Kameras nutzen entweder eine Detektoranordnung in zwei Ebenen oder in einer Ebene.

Im klassischen 2-Ebenen Compton-Design besteht die erste Ebene aus Detektoren eines Materials mit niedriger Ordnungszahl Z und die zweite aus Detektoren eines Materials hoher Ordnungszahl. Die Detektoren der ersten Ebene mit niedrigem Z haben eine hohe Wahrscheinlichkeit für Compton-Streuung für Gammaenergien von 100 keV bis 3 MeV. Die gestreute Gammastrahlung fällt anschließend auf die zweite Ebene, deren Detektoren mit hohem Z eine hohe Wahrscheinlichkeit besitzen, Strahlung in diesem Energiebereich zu absorbieren. Beide Ebenen haben einen Abstand von ca. 30 cm bis 1 m zueinander.

Ein charakteristisches Merkmal der 2-Ebenen Compton Kamera ist, dass die Detektorelektronik nur solche Koinzidenzereignisse prozessiert und aufzeichnet, an denen ein Detektor niedriger Ordnungszahl und ein Detektor hoher Ordnungszahl beteiligt sind. Koinzidenzen zwischen zwei Detektoren niedriger Ordnungszahl oder zwischen zwei Detektoren hoher Ordnungszahl sind für die klassische 2-Ebenen Compton Kamera ohne Bedeutung.

Die US2012/0114100 beschreibt ein Messsystem aus zwei Strahlungsdetektoren und einer Koinzidenzschaltung, das zur Richtungsmessung einer Strahlungsquelle geeignet ist. Die beiden Detektoren können im Wesentlichen baugleich oder auch verschieden sein. In der US2012/0043467 wird eine Compton Kamera beschrieben, die einen komplanaren Aufbau von Strahlungsdetektoren zur Richtungsmessung nutzt. Diese als Single Plane bezeichnete Compton Kamera zeichnet sich durch ihre einfache und kompakte Bauform aus.

Die in der US2012/0114100 und der US2012/0043467 beschriebenen Geräte sind nicht auf Detektoren niedriger Ordnungszahl angewiesen. Eine Einteilung der Detektoren in Streu- und Absorptions-Detektor ist für diese Geräte nicht erforderlich. Die Single Plane Compton Kamera in der US2012/0043467 weist den Detektoren eine Doppelrolle zu. Jeder Detektor in einer Single Plane Compton Kamera kann die Strahlung sowohl streuen als auch absorbieren. Hierfür sind Detektormaterialien von mittlerem bis hohen Z geeignet, die ähnliche Wahrscheinlichkeiten für Compton-Streuung und für photoelektrische Absorption im Energiebereich von 100 keV bis 3 MeV besitzen.

Das erfindungsgemäße bildgebende Detektorsystem stellt eine Weiterentwicklung der bekannten Bauformen für Compton Kameras dar. Die klassische 2-Ebenen Compton Kamera und die Single Plane Compton Kamera aus der US2012/0043467 werden als Spezialfälle des erfindungsgemäßen bildgebenden Detektorsystems identifiziert. Beide Bauformen - die 2-Ebenen Compton Kamera und die Single Plane Compton Kamera - werden durch die Erfindung modifiziert und verbessert. Außerdem erschließt die Erfindung neue Bauformen für Compton Kameras, die bisher nicht bekannt waren.

In der US2012/0114100 und der US2012/0043467 werden auch Verfahren zur Richtungsmessung beschrieben. Diese Verfahren sind dafür ausgelegt, die Richtung einer Strahlungsquelle zu bestimmen. Es wird vorausgesetzt, dass es von jedem Nuklid maximal eine Strahlungsquelle gibt. Alle Strahlen eines Nuklids sollen unter einem Winkel auf das System einfallen. Der Einfallswinkel kann dann für 2-dimensionale und 3-dimensionale Messsituationen ermittelt werden.

Gemäß der US2012/0114100 und der US2012/0043467 wird die Richtungsmessung folgendermaßen ausgeführt: Die in Koinzidenzereignissen gemessenen Energien in je zwei Strahlungsdetektoren werden in zwei separaten Spektren erfasst. Anschließend werden die Energiemittelwerte der beiden Spektren berechnet. Mit Hilfe einer empirischen Beziehung, welche die Richtung als Funktion der Energiemittelwerte ausdrückt, kann die Einfallsrichtung bestimmt werden.

Die Verfahren in der US2012/0114100 und der US2012/0043467 sind die ersten Verfahren zur Richtungsmessung mit einer Compton Kamera, bei der Streu- und Absorptions-Detektor physikalisch nicht voneinander unterscheidbar sein müssen. Eine wesentliche Einschränkung der Verfahren darin ergibt sich jedoch aus ihrer Beschränkung auf eine Einzelquelle pro Nuklid, da lediglich die Energiemittelwerte beider Detektoren berücksichtigt werden. Bei Vorhandensein mehrerer Strahlungsquellen des gleichen Nuklids sind die Verfahren nicht anwendbar. Die beschriebenen Verfahren sind zur Bildgebung nicht geeignet. Sie sind auf die Richtungsmessung einer Strahlungsquelle beschränkt.

Aufgabe der Erfindung ist es, ein bildgebendes Detektorsystem für Gammastrahlung bereitzustellen, welches die Nachteile des Stands der Technik überwindet. Das System soll in der Astronomie, der Medizin und im ABC- und Strahlenschutz einsetzbar sein und den spezifischen gerätetechnischen Anforderungen in diesen Fachgebieten gerecht werden.

In der Astronomie werden leistungsstarke hochempfindliche Compton Teleskope nachgefragt. Zu den Anforderungen in der Medizintechnik zählt eine deutliche Verbesserung der Nachweiseffizienz von SPECT Scannern und eine Ausweitung des Einsatzbereiches auf hochenergetische Nuklide bis zu einer Energie von 640 keV. Im ABC- und Strahlenschutz werden kompakte Geräte benötigt, die in Echtzeit und unter Einsatz weniger Strahlungsdetektoren eine Richtungsverteilung von einem Strahlungsfeld messen können. Die Aufgaben werden durch ein erfindungsgemäßes bildgebendes Detektorsystem gemäß dem unabhängigen Anspruch 1 gelöst. In den Unteransprüchen sind vorteilhafte Weiterbildungen und bevorzugte Ausführungsformen angegeben.

Das erfindungsgemäße bildgebende Detektorsystem für Gammastrahlung im Nah- und Fernfeld gemäß Anspruch 1 umfasst eine Gruppe von mehreren synchronisierten Detektoren zur Erfassung von Strahlung. Hierbei weist mindestens ein Detektormaterial eine Ordnungszahl von $Z_{eff} > 30$ auf. Alle Detektoren des erfindungsgemäßen bildgebenden Detektorsystems messen die Energien $E$ und die Wechselwirkungspunkte $\underline{d}$, welche in Wechselwirkungen der Strahlung mit den Detektormaterialien auftreten. Die Detektormaterialien sind dabei virtuell oder physisch in Voxel segmentiert.

Die kleinsten unterscheidbaren Volumeneinheiten der Detektormaterialien werden als Voxel bzw. Detektorvoxel bezeichnet. Ein bestimmtes Voxel ist durch die x, y und z-Koordinaten seines räumlichen Mittelpunktes $\underline{d}$ identifiziert.

Weiter umfasst das erfindungsgemäße bildgebende Detektorsystem eine Systemelektronik, welche Koinzidenzereignisse registriert, wenn in je zwei Detektorvoxeln aus einer Liste von definierten Voxelpaaren gleichzeitig Wechselwirkungen stattfinden. Die Liste von definierten Voxelpaaren umfasst alle Paare, die kombinatorisch aus der Menge aller Detektorvoxel gebildet werden, wobei die definierten Voxelpaare mindestens ein Detektorvoxel aus einem Material mit einer Ordnungszahl von $Z_{eff} > 30$ enthalten.

Weiter weist das erfindungsgemäße bildgebende Detektorsystem ein Datenakquisitionssystem auf, das die Messdaten der Koinzidenzereignisse speichert. Für beide an einem Koinzidenzereignis beteiligte Detektorvoxel wird eine Rangordnung festgelegt, die einen ersten und einen zweiten Detektorvoxel definiert. In jedem definierten Voxelpaar erhält der Voxel mit der niedrigeren Ordnungszahl die Nummer 1 und derjenige mit der höheren Ordnungszahl die Nummer 2. Sollten beide Detektorvoxel eines Paares die gleiche Ordnungszahl haben, wird die Kennzeichnung als 1 bzw. 2 willkürlich getroffen. Die in Koinzidenzereignissen gemessenen Energien $(E_1, E_2)$ und die Wechselwirkungspunkte $(\underline{d}_1, \underline{d}_2)$ werden entsprechend ihrer Kennzeichnung 1,2 geordnet und in einer chronologischen Liste mit den Attributen y = $\{\underline{d}_1, E_1, \underline{d}_2, E_2\}$ und der Detektionszeit $t$ gespeichert.

Darüber hinaus umfasst das erfindungsgemäße bildgebende Detektorsystem eine Analyseeinheit gemäß Anspruch 1 auf.

Ferner ist Gegenstand der Erfindung auch ein Verfahren zur Detektion und Bildgebung von Gammastrahlung gemäß Anspruch 14.

Das erfindungsgemäße bildgebende Detektorsystem dient zur Abbildung von Aktivitäts- und Richtungsverteilungen von Gammastrahlung emittierenden Strahlungsfeldern. Es ist im Nah- und Fernfeld einsetzbar. Das erfindungsgemäße Verfahren dient zur Detektion und Bildgebung von Gammastrahlung unter Verwendung des erfindungsgemäßen bildgebenden Detektorsystems.

Bei dem erfindungsgemäßen Verfahren sind die Detektormaterialien virtuell oder physisch in Voxel segmentiert, wobei das Verfahren uni- und bidirektionale Compton Streuprozesse nutzt und eine Systemmatrix $\underline{H}$, einen definierten Funktionswert $\sigma(E_1, E_2)$ und eine Liste von definierten Voxelpaaren aufweist und für die Erfassung von Projektionsdaten $\underline{p}(\underline{y})$ der Messwerte und für die Berechnung von Bilddaten $f(\underline{x})$ eingerichtet ist.

Das erfindungsgemäße Verfahren umfasst dabei die folgenden Schritte:

- Erstellen einer Liste mit definierten Voxelpaaren, wobei die definierten Voxelpaare alle Paare umfassen, welche kombinatorisch aus der Menge der Detektorvoxel gebildet werden können und jedes Paar mindestens einen Detektorvoxel aus einem Material mit einer Ordnungszahl von $Z_{eff} > 30$ enthält;
- Verschalten aller Detektoren/Voxel in einer Koinzidenzschaltung derart, dass Koinzidenzereignisse in allen definierten Voxelpaaren erfasst werden;
- Kennzeichnen der beiden Detektorvoxel eines jeden Voxelpaares mit den Nummern 1 bzw. 2, wobei der Detektorvoxel mit der niedrigeren Ordnungszahl die Nummer 1 erhält, und derjenige mit der höheren Ordnungszahl die Nummer 2, wenn beide Detektorvoxel aus dem gleichen Material bestehen, wird die Kennzeichnung willkürlich getroffen;
- Definieren einer Funktion $\sigma(E_1, E_2)$ gemäß Anspruch 14, welche aus zwei Energiewerten $E_1$ und $E_2$ berechnet wird;
- Akquirieren von Messwerten $\underline{y} = \{\underline{d}_1, E_1, \underline{d}_2, E_2\}$ von Koinzidenzereignissen, wenn in je zwei Detektorvoxeln von allen definierten Voxelpaaren gleichzeitig Wechselwirkungen stattfinden, wobei die Messwerte einem Strahlungsnah- oder Fernfeld entstammen und die Messwerte die in den Detektorvoxeln gemessenen Energien $(E_1, E_2)$ und die Wechselwirkungspunkte $(\underline{d}_1, \underline{d}_2)$ sind,
- Assoziieren von Koinzidenzereignissen $\underline{y} = \{\underline{d}_1, E_1, \underline{d}_2, E_2\}$ mit einem ersten Detektorvoxel/Detektionsort $\underline{d}_1$ und einem zweiten Detektorvoxel/Detektionsort $\underline{d}_2$;
- Berechnen des Funktionswertes $\sigma(E_1, E_2)$ aus zwei Energiewerten $(E_1, E_2)$ pro Koinzidenzereignis;
- Erfassen der Koinzidenzereignisse entsprechend ihres ersten Detektorvoxels $\underline{d}_1$, ihres zweiten Detektorvoxels $\underline{d}_2$ und ihres $\sigma(E_1, E_2)$ Wertes in einem Element der Projektionsdaten $\underline{p}(\underline{y})$, wobei für jedes Radionuklid separate Projektionsdaten $\underline{p}(\underline{y})$ erfasst werden;
- Berechnen eines oder mehrerer Bilder $f(\underline{x})$ aus den Projektionsdaten $\underline{p}(\underline{y})$ mit einem statistischen Bildrekonstruktionsverfahren der Emissionstomographie unter Nutzung der Systemmatrix $\underline{H}$, wobei für jedes Radionuklid ein separates Bild $f(\underline{x})$ berechnet wird; die Bilder $f(\underline{x})$ repräsentieren eine Aktivitätsverteilung in einem Quellvolumen oder eine Flussdichteverteilung über die Einfallsrichtungen.

Im Sinne der Erfindung werden unter Nah- und Fernfeld die geometrischen Nah- und Fernfelder verstanden. Ein Nahfeld liegt vor, wenn sich das bildgebende Detektorsystem nah am Quellvolumen befindet, d.h. wenn mindestens eine Strahlungsquelle so nah am Detektorsystem liegt, dass ihr Abstand als endlich groß in Bezug auf die Abmaße des Detektorsystems einzuschätzen ist. In einem Fernfeld liegen alle Strahlungsquellen soweit vom Detektorsystem entfernt, dass ihre Abstände sehr viel größer als das Detektorsystem sind. Das Fernfeld ist dadurch ausgezeichnet, dass es keinen Parallaxenfehler gibt. Für die Bildrekonstruktion ist von Bedeutung, dass ein Fernfeld keine radiale Abhängigkeit besitzt und nur von der Einfallsrichtung abhängig ist. Im Fernfeld sind die Beiträge einzelner Quellen entlang einer Richtung nicht unterscheidbar; es kann nur die Gesamtintensität der Strahlung aus einer Richtung gemessen werden.

Soweit nicht anders angegeben, gelten für alle Verwendungen des erfindungsgemäßen Detektorsystems in der Nuklearmedizin die Bedingungen des Nahfeldes als zutreffend im Sinne der Erfindung. Alle Verwendungen des erfindungsgemäßen Detektorsystems in der Astronomie oder im ABC- und Strahlenschutz fallen unter die Bedingungen des Fernfeldes, wenn keine anderweitigen Aussagen getroffen werden.

Das erfindungsgemäße bildgebende Detektorsystem verfügt über eine Gruppe mehrerer synchronisierter Strahlungsdetektoren, die in einer beliebigen linearen, ebenen oder räumlichen Anordnung vorliegen können. Im Sinne der Erfindung werden auch Detektorsegmente als Strahlungsdetektoren aufgefasst, wenn sie individuell auslesbar sind. Dabei weist mindestens ein Strahlungsdetektor eine mittlere bis hohe Ordnungszahl Z auf.

Im Sinne der Erfindung wird unter mittlerer Ordnungszahl eine Zahl von größer als 30 und kleiner als 50 verstanden. Eine Zahl größer als 50 wird im Sinne der Erfindung als eine hohe Ordnungszahl verstanden. Liegen die Detektormaterialien als chemische Verbindungen vor, so wird unter Z die effektive Ordnungszahl $Z_{eff}$ verstanden, d.h. die mittlere Ordnungszahl aller in der Verbindung enthaltenen Elemente unter Berücksichtigung der Atommassen der Elemente und ihrer stöchiometrischen Zusammensetzung.

Das erfindungsgemäße Detektorsystem erhält seine bildgebenden Eigenschaften durch die Auswahl geeigneter Detektorpaare in der Gruppe von synchronisierten Strahlungsdetektoren. Soweit nicht anders beschrieben wird im Sinne der Erfindung unter einem Detektor ein Strahlungsdetektor verstanden. Erfindungsgemäß enthält ein geeignetes Detektorpaar mindestens einen Detektor aus einem Material mittlerer bis hoher Ordnungszahl, d.h. jedes ausgewählte Detektorpaar weist mindestens einen Detektor mit einer Ordnungszahl $Z_{eff}$ größer als 30 auf.

Zur Einrichtung des erfindungsgemäßen bildgebenden Detektorsystems ist ein Maximum an Paarkombinationen von Strahlungsdetektoren zu berücksichtigen, d.h. es wird versucht, möglichst jede Kombination von Strahlungsdetektoren zu nutzen, die für den jeweiligen Anwendungsfall sinnvoll ist. Durch die Nutzung möglichst vieler dieser Kombinationen kann eine effiziente Bildgebung unter optimalen Voraussetzungen für die anschließende Bildrekonstruktion erfolgen.

Erfindungsgemäß sind beide Strahlungsdetektoren eines jeden Detektorpaares eindeutig mit den Nummern 1 bzw. 2 gekennzeichnet. Der Detektor eines Paares mit der niedrigeren Ordnungszahl erhält die Nummer 1; derjenige mit der höheren Ordnungszahl die Nummer 2. Bestehen beide Detektoren aus dem gleichen Material, wird die Kennzeichnung als 1 bzw. 2 willkürlich getroffen, insofern sie konsistent beibehalten wird.

Die Bezeichnung der Detektoren eines jeden ausgewählten Detektorpaars als *erster* bzw. *zweiter* Detektor ist für das zur Erfindung gehörige Bildrekonstruktionsverfahren erforderlich, impliziert darüber hinaus aber nicht notwendigerweise, wie der Prozess tatsächlich abgelaufen ist. Im Sinne der Erfindung sollen die Bezeichnungen *erster* und *zweiter* Detektor nicht so verstanden werden, dass der Ionisationsprozess, welcher die (nahezu) gleichzeitige Ionisation beider Detektoren eines Paares ausgelöst hat, zwangsläufig im ersten Detektor seinen Anfang nahm und im zweiten Detektor sein Ende fand. Diese Interpretation, welche für die klassische 2-Ebenen Compton Kamera von grundlegender Bedeutung ist, kann für das erfindungsgemäße Detektorsystem zutreffend sein, muss aber nicht zwangsläufig richtig sein. Grundsätzlich sollen die Bezeichnungen *erster* und *zweiter* Detektor nur als für die Bildrekonstruktion notwendige Kennzeichnung verstanden werden. Die Kennzeichnung ist von geometrischer Bedeutung, da sie eine Richtung im Raum definiert.

In der klassischen 2-Ebenen Compton Kamera kann jeder Detektor entsprechend seiner Ordnungszahl $Z_{eff}$ eindeutig als Streu- oder Absorptionsdetektor identifiziert werden. Detektoren aus Materialien mit $Z_{eff} \leq 30$ sind Streudetektoren; Detektoren aus Materialien mit $Z_{eff} > 30$ sind Absorptionsdetektoren.

Das erfindungsgemäße bildgebende Detektorsystem besitzt nicht zwangsläufig eine solche Funktionseinteilung. Die Rollen, welche Streu- und Absorptionsdetektoren in der klassischen 2-Ebenen Compton Kamera innehaben, werden in dem erfindungsgemäßen bildgebenden Detektorsystem von einem *ersten* und einem *zweiten* Strahlungsdetektor ausgeübt, entsprechend der erfindungsgemäßen Definition, wie die Detektoren in einem Detektorpaar zu nummerieren sind.

Diejenigen Detektorpaare des erfindungsgemäßen bildgebenden Detektorsystems welche einen Detektor aus einem Material mit einer Ordnungszahl $Z_{eff} \leq 30$ und einen zweiten Detektor aus einem Material $Z_{eff} > 30$ enthalten, werden als *unidirektional* bezeichnet. Für die unidirektionalen Detektorpaare gilt die von der klassischen 2-Ebenen Compton Kamera bekannte physikalische Funktionszuordnung: der Detektor mit der Nr. 1 ist der Streu-Detektor und derjenige mit der Nr. 2 ist der Absorptionsdetektor.

Alle anderen ausgewählten Detektorpaare des erfindungsgemäßen bildgebenden Detektorsystems, welche nicht unidirektional sind, werden als *bidirektional* bezeichnet. In bidirektionalen Detektionsprozessen ist nicht feststellbar, in welcher Abfolge die Wechselwirkungen in einem Koinzidenzereignis stattgefunden haben. Sofern keine anderweitigen Informationen vorliegen, ist in solchen Detektorpaaren nicht identifizierbar, welcher Detektor in einem Koinzidenzereignis die Strahlung gestreut und welcher sie absorbiert hat. Die Reaktion kann in beiden Richtungen - von 1 nach 2 oder von 2 nach 1 abgelaufen sein, ihre Richtung bleibt unbekannt. In den bidirektionalen Detektorpaaren ist die Kennzeichnung der Detektoren als erster und zweiter Detektor lediglich von geometrischer Bedeutung. Eine physikalische Aussage bezüglich der Richtung der gestreuten Strahlung ist für bidirektionale Detektorpaare nicht möglich.

Weiter umfasst das erfindungsgemäße Detektorsystem eine Detektorelektronik, die Signale aus den Strahlungsdetektoren registriert und deren Information über die absorbierte Strahlungsenergie ermittelt. Jeder Detektor des erfindungsgemäßen Detektorsystems ist für eine Energiemessung kalibriert.

Das erfindungsgemäße Detektorsystem umfasst außerdem eine Systemelektronik zur Synchronisation der Signale aus den einzelnen Strahlungsdetektoren und zur Erkennung und Speicherung von Koinzidenzereignissen in Form einer chronologischen Liste. Die Systemelektronik identifiziert Koinzidenzereignisse aus zwei gleichzeitig auftretenden Signalen in den ausgewählten Detektorpaaren und leitet diese Informationen an die Datenauswertung weiter. Für jedes Koinzidenzereignis wird ein Vektor $\underline{y}$ definiert, der sämtliche Informationen über die Koinzidenz enthält, d.h. die Wechselwirkungspunkte ($\underline{d}_1, \underline{d}_2$) und die Energien ($E_1, E_2$) von beiden Wechselwirkungen in den Detektoren 1 und 2:

$$\underline{y} = \{\underline{d}_1, E_1, \underline{d}_2, E_2\} \qquad (1)$$

Eine geeignete Form der Datenspeicherung ist die Datenerfassung in Form einer chronologischen Liste im sog. List-Mode. Jedes Koinzidenzereignis wird mit den in Gl. (1) genannten Messgrößen $\underline{y}$ und der Detektionszeit t gespeichert.

Gemäß einer Ausführungsform des erfindungsgemäßen bildgebenden Detektorsystems werden die Wechselwirkungspunkte ($\underline{d}_1, \underline{d}_2$) mit einer geeigneten, im System enthaltenen Vorrichtung gemessen. Auf dem Gebiet der Detektorkonstruktion sind vielfältige Möglichkeiten zur ortsauflösenden Strahlungsdetektion bekannt. Hierzu gehören z.B. Module von pixelierten Strahlungsdetektoren. Andere Konstruktionsformen nutzen segmentierte Halbleiterdetektoren. Auch für Szintillationsdetektoren sind vielfältige Techniken zur ortsauflösenden Strahlungsdetektion bekannt. Ein als Temporal Imaging bezeichnetes Detektorsystem wird in der US 9,638,811 B2 beschrieben. Experten auf diesem Fachgebiet sind ohne weiteres in der Lage, ein ortsauflösendes Detektorsystem zu entwerfen, das die Wechselwirkungspunkte ($\underline{d}_1, \underline{d}_2$) bestimmen kann.

In einer anderen Ausführungsform des erfindungsgemäßen bildgebenden Detektorsystems werden die Wechselwirkungspunkte nicht individuell für jede Wechselwirkung gemessen, sondern lediglich anhand der räumlichen Lage des Strahlungsdetektors approximiert. Diese Ausführungsform gilt für Detektorsysteme, die aus Detektoren zusammengesetzt sind, welche nur die Energie, nicht aber den Wechselwirkungspunkt bestimmen können. In dieser Ausführungsform wird der Wechselwirkungspunkt eines im Detektor registrierten Ereignisses mit dem räumlichen Mittelpunkt des aktiven Detektormediums gleichgesetzt.

Unter den Projektionsdaten $\underline{p}(\underline{y})$ des bildgebenden Detektorsystems wird die Anzahl von Koinzidenzereignissen verstanden, die den gleichen Vektor $\underline{y}$ mit den gemessenen Attributen $\{\underline{d}_1, E_1, \underline{d}_2, E_2\}$ besitzen. Die Projektionsdaten $\underline{p}(\underline{y})$ entstehen aus der Wechselwirkung der Strahlung mit dem bildgebenden Detektorsystem.

Das erfindungsgemäße bildgebende Detektorsystem beinhaltet ferner eine Analyseeinheit, welche alle Koinzidenzereignisse, die in der chronologischen Liste vorhanden sind, verarbeitet und daraus die Projektionsdaten $\underline{p}(\underline{y})$ erstellt. Das erfindungsgemäße Detektorsystem nutzt prinzipiell *alle* Einträge in der chronologischen Liste der Koinzidenzereignisse für die Bildrekonstruktion. Ausgenommen sind lediglich solche Einträge, welche von Paaren stammen, in denen beide Detektoren von niedriger Ordnungszahl $Z_{eff} \leq 30$ sind und keiner der Detektoren über ausreichende Wahrscheinlichkeit für den photoelektrischen Effekt verfügt.

Die Analyseeinheit hat die Aufgabe, aus den Projektionsdaten $\underline{p}(\underline{y})$ ein Bild der Strahlungsverteilung zu rekonstruieren. Die Bildrekonstruktion wird dabei folgendermaßen durchgeführt:
Gemäß einer Ausführungsform des erfindungsgemäßen bildgebenden Detektorsystems befindet sich das Detektorsystem im Nahfeld eines Quellvolumens, von dem die Strahlung ausgeht. Wenn $\underline{x}$ ein Punkt im Quellvolumen ist, wird die Aktivitätsdichte $f(\underline{x})$ als Anzahl der am Punkt $\underline{x}$ emittierten Gamma-Photonen pro Zeiteinheit aufgefasst. Die Aufgabe der Bildrekonstruktion besteht darin, die Aktivitätsdichte $f(\underline{x})$ aus den Projektionsdaten $\underline{p}(\underline{y})$ zu rekonstruieren, welche vom bildgebenden Detektorsystem aufgezeichnet werden.

In einer anderen Ausführungsform befindet sich das erfindungsgemäße bildgebende Detektorsystem im Fernfeld eines Quellvolumens. In dieser Ausführungsform beschreibt $\underline{x}$ eine Einfallsrichtung, in der die vom Quellvolumen ausgehende Strahlung vom Detektorsystem empfangen wird. Die Funktion $f(\underline{x})$ beschreibt in der Ausführung für das Fernfeld eine Flussdichte, d.h. die Anzahl an Gamma-Photonen, die pro Raumwinkel und pro Zeiteinheit das Detektorsystem aus der Richtung $\underline{x}$ erreichen. In dieser Ausführungsvariante ist die Bildrekonstruktion mit der Aufgabe betraut, die Flussdichte $f(\underline{x})$ aus den Projektionsdaten $p(\underline{y})$ zu rekonstruieren. Im Kontext einer Nutzung des erfindungsgemäßen bildgebenden Detektorsystems für Aufgaben des ABC- und Strahlenschutzes kann die Flussdichte $f(\underline{x})$ als Dosisleistungsdichte kalibriert werden.

Im Sinne der Erfindung wird die Funktion $f(\underline{x})$ auch als Bild bezeichnet. Je nach Anwendungsfall kann das Bild $f(\underline{x})$ eine Aktivitätsdichte, eine Flussdichte bzw. eine Dosisleistungsdichte darstellen. Der Vorgang der Erstellung eines Bildes $f(\underline{x})$ aus den Projektionsdaten $p(\underline{y})$ wird auch Rückprojektion bzw. Entfaltung genannt. Der inverse Vorgang, mit dem eine Darstellung der Projektionsdaten $\underline{p}(\underline{y})$ aus einem Bild $f(\underline{x})$ erzeugt wird, heißt Vorwärtsprojektion.

Die zum erfindungsgemäßen bildgebenden Detektorsystem gehörige Analyseeinheit führt die Bildrekonstruktion mit einem statistischen bzw. iterativen Bildrekonstruktionsverfahren aus. Solche Bildrekonstruktionsverfahren wurden vornehmlich für klinische Anwendungen in der Emissionstomographie entwickelt. Zu den statistischen Bildrekonstruktionsverfahren zählt beispielsweise das Maximum Likelihood Expectation Maximization Verfahren (MLEM) von Shepp und Vardi [IEEE Transactions on Medical Imaging 1 (1982) 113]. Eine große Anzahl dieser Verfahren steht ohne Einschränkungen zur Nutzung frei und ihre Implementierung ist den Experten auf diesem Fachgebiet wohl vertraut.

Die statistischen Bildrekonstruktionsverfahren haben den Vorteil, dass sie mit geringfügigen Anpassungen für beliebige bildgebende Detektorsysteme einsetzbar sind, die es für Gammastrahlung gibt. Statistische Bildrekonstruktionsverfahren sind universell gültig. Sie gelten für PET und SPECT Scanner, für Compton Kameras und für das erfindungsgemäße bildgebende Detektorsystem. Ihr Geltungsbereich ist nicht auf ein bestimmtes Funktionsprinzip festgelegt, das dem Detektionsprozess zugrunde liegt. Die statistischen Verfahren unterstützen die Bildrekonstruktion für Gammastrahlung aus dem Nah- und Fernfeld und für alle Radioisotope.

Die statistischen Bildrekonstruktionsverfahren diskretisieren den Bild- und Detektorraum in Voxel bzw. Pixel.

In der Ausführung der Erfindung für das Nahfeld wird der 3-dimensionale Bildraum in Voxel unterteilt. In der Ausführung für das Fernfeld ist der Bildraum 2-dimensional und repräsentiert eine Kugeloberfläche. Die Kugeloberfläche wird in Pixel von gleicher Fläche unterteilt. Das statistische Bildrekonstruktionsverfahren berechnet die Pixelwerte der Flussdichte auf einer Kugeloberfläche. Eine häufig genutzte Projektion zur Darstellung einer Kugeloberfläche in einer Ebene ist z.B. die Mollweide-Projektion, die auch als Babinet-Projektion bekannt ist. In dieser Ausführungsform der Erfindung wird die Flussdichte als Ellipsenfläche abgebildet.

Wenn der Bild- und Detektorraum in Voxel bzw. Pixel unterteilt ist, gilt die folgende Vektorgleichung:

$$\underline{p} = \underline{H}\,\underline{f}^T \qquad\qquad (2)$$

Gl. (2) definiert einen funktionalen Zusammenhang zwischen den nunmehr diskreten Projektionsdaten $\underline{p}(\underline{y})$ und den diskreten Bilddaten $\underline{f}(\underline{x})$. $\underline{p}$ und $\underline{f}$ sind jetzt Vektoren, deren Elemente $p_i$ und $f_j$ die Werte an den $i$-ten und $j$-ten Stellen in den jeweiligen Vektorräumen repräsentieren. Die Matrix $\underline{H}$ wird üblicherweise als Projektions- bzw. als Systemmatrix bezeichnet. Die Elemente $H_{ij}$ der Systemmatrix $\underline{H}$ haben im Nah- und Fernfeld unterschiedliche Bedeutungen. Im Nahfeld repräsentieren die Elemente $H_{ij}$ die Wahrscheinlichkeiten, dass ein im Voxel $j$ emittiertes Gamma-Quant ein Koinzidenzereignis auslöst, welches symbolisch als $i$ gekennzeichnet wird. Im Fernfeld stellen die Elemente $H_{ij}$ die Wahrscheinlichkeiten dar, dass ein aus der Richtung $j$ eintreffendes Gamma-Quant ein Koinzidenzereignis $i$ auslöst. Beiden Ausprägungen gemeinsam ist, dass die Systemmatrix $\underline{H}$ alle physikalischen Zusammenhänge beschreibt, welche das Detektorsystem kennzeichnen, einschließlich der Charakteristika der Strahlungsdetektoren und der Einflüsse der Elektronik. Es gibt grundsätzlich drei Vorgehensweisen zur Erzeugung der Systemmatrix $\underline{H}$: sie kann auf direktem Wege durch Messungen mit dem Detektorsystem erzeugt, bzw. durch Monte Carlo Simulationen oder mittels eines theoretischen Modells erstellt werden.

Der diskrete Ansatz, Bild- und Detektorraum in Voxel bzw. Pixel zu unterteilen, der allen statistischen Bildrekonstruktionsverfahren zu Grunde liegt, vereinfacht die Bildrekonstruktion und ist besonders für solche Ausführungsformen der Erfindung geeignet, in denen die Messdaten ohnehin in diskreter Form vorliegen, wie z.B. bei pixelierten oder segmentierten Strahlungsdetektoren.

Es gibt eine Vielzahl von Ausführungsformen der Erfindung, wie der Detektorraum diskretisiert werden kann. In einer Ausführungsform der Erfindung ist das Detektorsystem aus Detektoren zusammengesetzt, die keine Ortsinformation bereitstellen. In dieser Ausführungsform kann jeder Strahlungsdetektor als diskrete Einheit aufgefasst werden. Jedes Element von $\underline{p}$ besteht aus einer Anzahl an Koinzidenzereignissen, die bei einer jeweiligen Kombination von einem bestimmten ersten Detektor mit einem bestimmten zweiten Detektor bei einem bestimmten Funktionswert $\sigma(E_1, E_2)$ registriert werden. Der Funktionswert $\sigma(E_1, E_2)$ repräsentiert die in den Energiewerten $E_1$ und $E_2$ enthaltene Richtungsinformation.

In einer klassischen 2-Ebenen Compton Kamera wird der Funktionswert typischerweise als Compton Streuwinkel $\vartheta$

$$\vartheta = \cos^{-1}\left(1 - \frac{mc^2}{E_2} + \frac{mc^2}{E_1 + E_2}\right) \qquad\qquad (3)$$

mit $mc^2$ = 511 keV, der Ruhenergie des Elektrons, definiert. Diese Definition ist jedoch nur sinnvoll, wenn vorausgesetzt werden kann, dass die Strahlung in Detektor 1 gestreut und in Detektor 2 absorbiert wurde. In dem erfindungsgemäßen bildgebenden Detektorsystem ist aber evt. gar nicht bekannt, wo die Strahlung gestreut und wo sie absorbiert worden ist.

Die Analyseeinheit des erfindungsgemäßen bildgebenden Detektorsystems macht Gebrauch von einer Funktion $\sigma(E_1, E_2)$, die für alle Energiewerte $0 \leq E_1 \leq C$ und $0 \leq E_2 \leq C$ eindeutig definiert sein muss. Hierbei repräsentiert $C$ die Strahlungsenergie $C = E_1 + E_2$.

Der Funktionswert $\sigma$ gemäß dieser Erfindung ist:

$$\sigma(E_1, E_2) = \frac{E_2 - E_1}{E_1 + E_2} \qquad (4)$$

In Gl. (4) verweist der Index 1 auf die in Detektor 1 gemessene Energie $E_1$ und der Index 2 auf die in Detektor 2 gemessene Energie $E_2$, gemäß der zuvor getroffenen Festlegung zur Nummerierung der Detektoren. Die in Gl. (4) gegebene Definition für den Funktionswert $\sigma$ stellt eine Ausführungsform der Erfindung dar; die Erfindung ist aber nicht auf diese beschränkt; alternative Definitionen für $\sigma$ sind in der Erfindung inbegriffen.

In einer Ausführungsform der Erfindung ist das Detektorsystem aus Modulen von pixelierten Strahlungsdetektoren mittlerer bis hoher Ordnungszahl zusammengesetzt. In dieser Ausführungsform besteht jedes Element von $\underline{p}$ aus einer Anzahl an Koinzidenzereignissen, die bei einer jeweiligen Kombination von einem bestimmten ersten Detektor-Bin mit einem bestimmten zweiten Detektor-Bin bei einem bestimmten Funktionswert $\sigma(E_1, E_2)$ gezählt werden. Der Vektorraum von $\underline{p}$ erstreckt sich über alle Kombinationen von je zwei Detektor-Bins innerhalb eines Moduls und über alle Kombinationen eines jeden Detektor-Bins in einem Modul mit jedem Detektor-Bin in jedem anderen Modul. Für jede Kombination von je zwei Detektor-Bins ist eindeutig festzulegen, welches Bin die Nummer 1 und welches Bin die Nummer 2 hat. Die Elemente von $\underline{p}$ sind des Weiteren auch nach Klassen im Funktionswert $\sigma(E_1, E_2)$ differenziert. Der Funktions-wert $\sigma$ kann gemäß Gl. (4) oder anderweitig berechnet werden.

In einer Ausführungsform wird das Detektorsystem aus Modulen von pixelierten Strahlungsdetektoren mittlerer bis hoher Ordnungszahl für eine Messung im Fernfeld eingesetzt. In dieser Ausführungsform kann die Dimensionalität des Vektors $\underline{p}$ reduziert werden, indem alle Elemente von $\underline{p}$, bei denen das erste und das zweite Detektor-Bin jeweils den gleichen Abstand und die gleiche Richtung haben und die Funktionswerte $\sigma(E_1, E_2)$ übereinstimmen, zusammengefasst werden.

In einer weiteren Ausführung der Erfindung ist das Detektorsystem aus Strahlungsdetektoren mittlerer bis hoher Ordnungszahl zusammengesetzt, die das Temporal Imaging Funktionsprinzip aus der US 9,638,811 B2 nutzen. Gemäß der US 9,638,811 B2 ist das Detektorsystem für schnelle Szintillationsmaterialien mit Pulsanstiegszeiten unter einer Nanosekunde geeignet. Es soll hier davon ausgegangen werden, dass alle technischen Voraussetzungen gemäß der US 9,638,811 B2 vorliegen. Erfindungsgemäß wird jeder im System enthaltene Strahlungsdetektor virtuell in diskrete Voxel unterteilt. Die Elemente von $\underline{p}$ sind die Anzahlen an Koinzidenzereignissen, die bei einer jeweiligen Kombination von einem bestimmten ersten Voxel mit einem bestimmten zweiten Voxel bei einem bestimmten Funktionswert $\sigma(E_1, E_2)$ gezählt werden. Der Vektorraum von $\underline{p}$ erstreckt sich über alle Kombinationen von einem Voxel in einem Strahlungsdetektor mit jedem Voxel in jedem anderen Detektor. Für jede Kombination von je zwei Voxeln ist eindeutig festzulegen, welches Voxel die Nummer 1 und welches Voxel die Nummer 2 hat. Die Elemente von $\underline{p}$ sind des Weiteren auch nach Klassen im Funktionswert $\sigma(E_1, E_2)$ differenziert. Der Funktionswert $\sigma$ kann gemäß Gl. (4) oder anderweitig berechnet werden.

Wenn die Technik aus der US 9,638,811 B2 es erlaubt, die Energien $E_1$ und $E_2$ von zwei gleichzeitig in einem Strahlungsdetektor auftretenden Wechselwirkungen separat zu messen, wird der Vektorraum von $\underline{p}$ entsprechend erweitert, so dass zusätzlich auch alle Kombinationen von je zwei Voxeln innerhalb eines jeden Strahlungsdetektors erfasst werden.

Im Folgenden wird davon ausgegangen, dass die Projektionsdaten $\underline{p}(\underline{y})$ des erfindungsgemäßen Detektor-systems als Vektor vorliegen. Der Bildraum für $f(\underline{x})$ ist ebenfalls diskretisiert, d.h. in Voxel oder Pixel unterteilt. Die statistischen Bildrekonstruktionsverfahren, welche erfindungsgemäß zur Analyseeinheit gehören, nutzen Gl. (2), um die Werte $f_j$ des Bildvektors $\underline{f}$ zu bestimmen.

In einer Ausführungsform der Erfindung werden Expectation Maximization Algorithmen (EM) als statistische Bildrekonstruktionsverfahren in der Analyseeinheit verwendet. EM Bildrekonstruktionsverfahren zählen zu den erfolgreichsten Verfahren in der Emissionstomographie. Diese Verfahren versuchen, eine akzeptable Lösung für $f$ durch eine Folge von Näherungen zu erreichen. Ausgehend von einer ersten Schätzung wird in einer Folge von immer besseren Schätzungen versucht, die beste Annäherung an das wahre Bild $\underline{f}$ zu rekonstruieren. In jedem Iterationsschritt wird das vorliegende Bild $\underline{f}$ benutzt, um eine neue Darstellung der Projektionsdaten durch Vorwärtsprojektion zu generieren. Die berechneten Projektionsdaten werden mit den gemessenen Daten $\underline{p}$ verglichen. Nach einigen Iterationen konvergiert das Verfahren zu immer besserer Übereinstimmung der aus $\underline{f}$ berechneten Projektionsdaten mit den Messdaten $\underline{p}$. Am Ende führt das Verfahren zu dem Bild $\underline{f}$, welches die höchste Wahrscheinlichkeit für die beobachtete Verteilung $\underline{p}$ besitzt.

In einer Ausführungsform der Erfindung wird in der Analyseeinheit das Maximum Likelihood Expectation Maximization Verfahren (MLEM) eingesetzt, um aus den Projektionsdaten $\underline{p}$ des Detektorsystems ein Bild $\underline{f}$ zu berechnen. Das Verfahren wendet die Iterationsvorschrift

$$f_j^{[n+1]} = \frac{f_j^{[n]}}{\sum_i^N H_{ij}} \sum_i^N \frac{p_i H_{ij}}{\sum_l H_{il} f_l^{[n]}} \qquad (5)$$

an und berechnet eine Bildfolge $\underline{f}^{[n]}$. Mit jedem Iterationsschritt $n$ wird aus einer vorliegenden Darstellung $\underline{f}^{[n]}$ eine neue Darstellung $\underline{f}^{[n+1]}$ berechnet. Das Verfahren beginnt mit einer Anfangsverteilung $\underline{f}^{[0]}$.

In einer Ausführungsform der Erfindung geht die Gammastrahlung von mehreren Radionukliden aus. Das erfindungsgemäße bildgebende Detektorsystem nutzt dann eine Analyseeinheit, die für jedes Radionuklid ein separates Bild rekonstruiert. Hierfür sortiert die Analyseeinheit die in der chronologischen Liste gespeicherten Koinzidenzereignisse nach ihrer Energiesumme $E_1 + E_2$. Es werden Selektionsbedingungen auf die Energiesumme $E_1 + E_2$ angewandt, die den charakteristischen Nuklidenergien entsprechen, deren Strahlung detektiert wird. Für jedes Radionuklid werden separate nuklidspezifische Projektionsdaten $\underline{p}(\underline{y})$ erstellt. Die Analyseeinheit registriert nur solche Koinzidenzereignisse in den nuklidspezifischen Projektionsdaten $\underline{p}(\underline{y})$, deren Energiesumme $E_1 + E_2$ innerhalb eines vordefinierten Bereichs um den Energiewert der jeweiligen Nuklidenergie liegt. Mit Hilfe solcher Selektionsbedingungen ist es möglich, separate Projektionsdaten $\underline{p}(\underline{y})$ für jedes detektierte Nuklid auszuwerten. Außerdem werden Ereignisse aussortiert, die nicht dem Detektionsprofil aus einer Koinzidenz einer Compton-Streuung und einer photoelektrischen Absorption entsprechen, weil es sich z.B. um zwei Compton-Streuungen handelt, ohne dass eine photoelektrische Absorption stattgefunden hat.

Wenn die Gammastrahlung von einem Radionuklid ausgeht, das auf mehreren Gammalinien emittiert, liegt es im Ermessen des Anwenders, ob die Koinzidenzereignisse in einem einheitlichen Projektionsdatensatz oder in mehreren Datensätzen erfasst werden sollen. Beispielsweise besitzt Co-60 zwei nah beieinander liegende Gammalinien bei 1173 keV und 1332 keV. Koinzidenzereignisse beider Emissionen können ohne Probleme im gleichen Projektionsdatensatz erfasst werden. Da beide Gammaenergien so nah beieinander liegen, sind auch die Projektionsdatensätze sehr ähnlich und können ohne Schwierigkeiten zusammen ausgewertet werden. Hat jedoch ein Nuklid mehrere, weit auseinanderliegende Gammalinien ist es empfehlenswert, diese getrennt zu erfassen. In diesem Fall werden mehrere Projektionsdatensätze angelegt, die später im Bildrekonstruktionsprogramm wieder zusammengeführt werden.

In einer Ausführungsform der Erfindung berechnet die Analyseeinheit mit dem Maximum Likelihood Expectation Maximization Verfahren (MLEM) gemäß Gl. (5) für jeden vorliegenden nuklidspezifischen Projektionsdatensatz $\underline{p}$ ein separates nuklidspezifisches Bild $\underline{f}$. Es wird vorausgesetzt, dass alle benötigten Systemmatrizen $\underline{H}$ für die betreffenden Radionuklide vorhanden sind.

Die Abbildungseigenschaften des erfindungsgemäßen bildgebenden Detektorsystems werden durch die Materialzusammensetzung, die Systemelektronik und den räumlichen Aufbau des Detektorsystems bestimmt. Diese Zusammenhänge gelten universell und sollen hier näher erläutert werden.

Gemäß einer Ausführungsform des erfindungsgemäßen bildgebenden Detektorsystems werden Detektorpaare ausgewählt, die kombinatorisch aus der Menge aller Detektoren gebildet werden können, wobei die ausgewählten Detektorpaare mindestens einen Detektor mittlerer bis hoher Ordnungszahl $Z_{eff} > 30$ enthalten. Es werden so viele Detektorpaare wie möglich ausgewählt, wie es für einen Anwendungsfall sinnvoll ist. Weiter kann als Material für die Strahlungsdetektoren mit $Z_{eff} > 30$ ein reines oder dotiertes Szintillationsmaterial wie NaI, CeBr$_3$, LaBr$_3$, LaCl$_3$, La(Br$_x$Cl$_{1-x}$)$_3$, CsI, SrI$_2$, BaF$_2$, CLYC, CLBC, CLCB, CLLB, BGO, LSO, LYSO, GAGG, YAP, YAG und/oder ein Halbleitermaterial wie Ge, GaAs, CdTe und/oder CdZnTe verwendet werden.

Gemäß einer Ausführungsform des erfindungsgemäßen bildgebenden Detektorsystems sind die Detektorpaare unidirektional ausgebildet, ein solches unidirektionales Detektorpaar enthält einen Detektor niedriger Ordnungszahl $Z_{eff} \leq 30$ und einen Detektor mittlerer bis hoher Ordnungszahl $Z_{eff} > 30$. Weiter kann als Material für die Strahlungsdetektoren mit $Z_{eff} \leq 30$ ein Szintillationsmaterial wie PVT, Anthracen, Stilben, p-Terphenyl, CaF$_2$, ein organischer Flüssigszintillator und/oder ein Halbleitermaterial wie Si verwendet werden.

Gemäß einer weiteren Ausführungsform des erfindungsgemäßen bildgebenden Detektorsystems sind die Detektorpaare bidirektional ausgebildet, ein solches bidirektionales Detektorpaar enthält zwei Detektoren mittlerer bis hoher Ordnungszahl $Z_{eff} > 30$.

Gemäß einer weiteren Ausführungsform des erfindungsgemäßen bildgebenden Detektorsystems sind die Detektorpaare sowohl unidirektional als auch bidirektional ausgebildet.

Gemäß einer Ausführungsform des erfindungsgemäßen bildgebenden Detektorsystems ist das Detektorsystem mit Detektor- und Systemelektronik ausgestattet, welche die Detektorsignale analysiert. Die Signale werden bezüglich ihrer Energien analysiert (diskriminiert) und auf ihre Zeitstruktur untersucht (Koinzidenzanalyse). Die Energiediskriminierung und die Koinzidenzanalyse können mit analoger und/oder digitaler Elektronik erfolgen. Weiter kann die analoge Elektronik eine Kombination verschiedener Arten von elektronischen Modulen umfassen, die zusammengeschaltet eine Energiediskriminierung und eine Koinzidenzanalyse der Signale ermöglichen. Weiter können als elektronische Module eine Hochspannungsversorgung, ein Vorverstärker, ein Verstärker, ein Pulsformer, ein Ladungsintegrator, ein Pulshöhenanalysator, ein Multikanalanalysator (MCA) und/oder eine Koinzidenzschaltung verwendet werden. Weiter besteht die digitale Elektronik aus Hardware- und Softwarekomponenten, welche die Signale aller Strahlungsdetektoren digitalisieren, prozessieren und speichern, um anschließend mit digitalen Mitteln eine Energiediskriminierung und Koinzidenzanalyse durchzuführen. Weiter gehören zur Hardware der digitalen Elektronik eine Hochspannungsversorgung, ein A/D-Wandler pro Strahlungsdetektor, ein Field Programmable Gate Array (FPGA), ein Speichermedium und/oder ein digitaler Signalprozessor. Weiter umfasst die digitale Elektronik eine Software für die digitale Signal- und Koinzidenzanalyse, welche in einem mehrstufigen Prozess den kompletten Datensatz voll automatisch auswertet. Weiter setzt sich der mehrstufige Auswerteprozess der digitalen Signal- und Koinzidenzanalyse zusammen aus dem Einlesen der Daten, der Ermittlung grundlegender Signaleigenschaften, einer digitalen Signalfilterung, der Integration von Signalverläufen, einer Energienormierung, einer Koinzidenzanalyse und der Ausgabe von Diskriminierungs- und Koinzidenzinformationen.

Gemäß einer Ausführungsform des erfindungsgemäßen bildgebenden Detektorsystems zeichnet die Systemelektronik die Daten von Koinzidenzereignissen als chronologische Liste auf. Für jedes in einem ausgewählten Detektorpaar auftretende Koinzidenzereignis werden die Wechselwirkungspunkte ($\underline{d}_1$, $\underline{d}_2$) und die Energien ($E_1$, $E_2$) der beiden Wechselwirkungen, einschließlich der Detektionszeit t gespeichert.

Gemäß einer Ausführungsform des erfindungsgemäßen bildgebenden Detektorsystems weisen alle Strahlungsdetektoren im Wesentlichen eine gleiche Bauart auf. Es ist jedoch auch vorgesehen, dass mindestens zwei der Strahlungsdetektoren eine voneinander verschiedene Bauart aufweisen können.

Gemäß einer Ausführungsform des erfindungsgemäßen bildgebenden Detektorsystems ist das Detektorsystem aus einer Gruppe gleichartiger Detektoren aufgebaut, alle Detektoren bestehen aus Materialien mittlerer bis hoher Ordnungszahl $Z_{eff}$ > 30 und besitzen signifikante Wahrscheinlichkeiten sowohl für photoelektrische Absorption als auch für Compton-Streuung im Energiebereich von 100 keV bis 3 MeV. Die Materialien können gleich oder verschiedenartig sein. Alle Detektoren sind in einer einlagigen, homogenen Fläche angeordnet, die Fläche kann eben oder gekrümmt sein. Weiter kann jeder Detektor mit jedem anderen zu einem Paar kombiniert werden. Die Detektorpaare sind bidirektional, d.h. die Strahlung wird in beide Richtungen mit ähnlicher Intensität gestreut. Ein solches Detektorsystem wird im Sinne der Erfindung als Typ B bezeichnet.

Gemäß einer weiteren Ausführungsform des erfindungsgemäßen bildgebenden Detektorsystems ist das Detektorsystem aus Detektoren aufgebaut, die in zwei Gruppen eingeteilt werden können. Eine Gruppe besteht aus Detektoren mit Materialien von mittlerer bis hoher Ordnungszahl $Z_{eff}$ > 30, sie besitzen hohe Wahrscheinlichkeiten für photoelektrische Absorption im Energiebereich von 100 keV bis 3 MeV. Eine zweite Gruppe besteht aus Detektoren mit Materialien niedriger Ordnungszahl $Z_{eff} \leq 30$, sie besitzen hohe Wahrscheinlichkeiten für Compton-Streuung im Energiebereich von 100 keV bis 3 MeV. Detektoren aus der einen Gruppe werden mit Detektoren aus der anderen Gruppe zu Paaren kombiniert, diese Detektorpaare sind unidirektional, d.h. die Strahlung wird hauptsächlich in eine Richtung gestreut, nämlich vom Detektor mit niedriger Ordnungszahl auf den Detektor mittlerer bis hoher Ordnungszahl. Zusätzlich bilden die Detektoren aus der Gruppe mittlerer bis hoher Ordnungszahl untereinander ein Detektorsystem vom Typ B. Detektoren mittlerer bis hoher Ordnungszahl zeigen neben dem photoelektrischen Effekt auch beträchtliches Compton-Streuverhalten. Die aus den Detektoren mit $Z_{eff}$ > 30 gebildeten Detektorpaare sind bidirektional. Die Detektoren niedriger Ordnungszahl $Z_{eff} \leq 30$ sind hingegen zur Paarbildung nicht geeignet, da sie nicht über ausreichend hohe Wahrscheinlichkeiten für den photoelektrischen Effekt verfügen. Ein solches Detektorsystem wird im Sinne der Erfindung als Typ A bezeichnet.

Detektorsysteme vom Typ A gibt es in zwei Ausführungsvarianten, als Typ A *getrennt* und als Typ A *gemischt*.

Gemäß einer weiteren Ausführungsform des erfindungsgemäßen bildgebenden Detektorsystems vom Typ A sind die Detektoren beider zum Typ A Detektorsystem gehörigen Gruppen mit $Z_{eff}$ > 30 bzw. $Z_{eff} \leq 30$ räumlich voneinander getrennt. Befinden sich die Strahlungsquellen außerhalb des Detektorsystems, bilden die Detektoren der Gruppe mit niedriger Ordnungszahl $Z_{eff} \leq 30$ die äußere Hülle, sie umschließen die Detektoren der anderen Gruppe aus Materialien mittlerer bis hoher Ordnungszahl $Z_{eff}$ > 30, welche im Inneren konzentriert sind. Befinden sich die Strahlungsquellen innerhalb des Detektorsystems, sind die Detektoren der Gruppe mit niedriger Ordnungszahl $Z_{eff} \leq 30$ in einem inneren Bereich konzentriert, dieser ist umgeben von einer äußeren Hülle aus den Detektoren der anderen Gruppe aus Materialien mittlerer bis hoher Ordnungszahl $Z_{eff}$ > 30. Ein solches Detektorsystem wird im Sinne der Erfindung als Typ A *getrennt* bezeichnet.

Gemäß einer weiteren Ausführungsform des erfindungsgemäßen bildgebenden Detektorsystems vom Typ A sind die Detektoren beider zum Typ A Detektorsystem gehörigen Gruppen mit $Z_{eff}$ > 30 bzw. $Z_{eff} \leq 30$ räumlich miteinander gemischt. Alle Detektoren sind in einer einlagigen, heterogenen Fläche angeordnet, die Fläche kann eben oder gekrümmt sein. Ein solches Detektorsystem wird im Sinne der Erfindung als Typ A *gemischt* bezeichnet.

Die erfindungsgemäßen bildgebenden Detektorsysteme vom Typ A *getrennt* sind eng verwandt mit dem Funktionsprinzip der klassischen 2-Ebenen Compton Kamera, haben aber ein wichtiges Herausstellungsmerkmal. In Detektorsystemen vom Typ A *getrennt* wird die Gruppe der Detektoren von mittlerer bis hoher Ordnungszahl zu einem Detektorsystem vom Typ B zusammengeschaltet. Diese neue Nutzungsform hat Auswirkungen auf die originale 2-Ebenen Compton Kamera, welche seit vielen Jahrzehnten den Stand der Technik repräsentiert. Die hintere Detektorebene in einer 2-Ebenen Compton Kamera ist ein Detektorsystem vom Typ B. Die Anzahl an nutzbaren Detektorpaaren in Typ A und B Detektorsystemen aus je n Detektoren ist in Tab. 1 angegeben. Zum Vergleich sind auch die Zahlen für eine klassische 2-Ebenen Compton Kamera aufgeführt. Das Typ A Detektorsystem enthält bei gleicher Anzahl n von Detektoren 50% mehr Paare als die 2-Ebenen Compton Kamera, das Typ B Detektorsystem sogar doppelt so viele Paare.

Tab. 1: Anzahl an Detektorpaaren für ein Detektorsystem aus n Detektoren bei Konfiguration als a) Klassische 2-Ebenen Compton Kamera, b) Typ A Detektorsystem und c) Typ B Detektorsystem. Für die 2-Ebenen Compton Kamera und für das Typ A Detektorsystem wurde angenommen, dass 50% der Detektoren von mittlerem bis hohen $Z_{eff}$, die anderen 50% von niedrigem $Z_{eff}$ sind.

| | Anzahl Detektorpaare | | Zugewinn in % für $n \to \infty$ |
|---|---|---|---|
| | | für $n \to \infty$ | |
| Klassische 2-Ebenen Compton Kamera (Stand der Technik) | $\dfrac{n^2}{4}$ | $\dfrac{n^2}{4}$ | |
| Typ A Detektorsystem | $\dfrac{n^2}{4} + \dfrac{n}{4}\left(\dfrac{n}{2} - 1\right)$ | $\dfrac{3n^2}{8}$ | 50 % |
| Typ B Detektorsystem | $\dfrac{n}{2}(n-1)$ | $\dfrac{n^2}{2}$ | 100 % |

Gemäß einer weiteren Ausführungsform des erfindungsgemäßen bildgebenden Detektorsystems ist ein Teil des Systems oder das ganze System als klassische 2-Ebenen Compton Kamera ausgebildet, welche nunmehr durch Verschalten der Detektoren als erfindungsgemäßes bildgebendes Detektorsystem vom Typ A *getrennt* aufgebaut wird. Hierfür sind die entsprechenden Erweiterungen in der Systemelektronik vorzunehmen, so dass die hintere Kameraebene der 2-Ebenen Compton Kamera als ein erfindungsgemäßes bildgebendes Detektorsystem vom Typ B eingerichtet wird. Die hintere Kameraebene, die erfindungsgemäß als Detektorsystem vom Typ B ausgebildet ist, wird in die vorhandene Datenauswertung integriert. Die Analyseeinheit verarbeitet die Daten aller Detektorpaare, die erfindungsgemäß zum Detektorsystem gehören, einschließlich der Daten von den bidirektionalen Detektorpaaren in der hinteren Typ B Kameraebene.

Detektorsysteme vom Typ A *gemischt* und vom Typ B stellen eine Weiterentwicklung der Single Plane Compton Kamera dar, die erstmalig in der US2012/0043467 beschrieben wurde. Es soll an dieser Stelle dargelegt werden, wie das erfindungsgemäße bildgebende Detektorsystem den Stand der Technik gegenüber der US2012/0043467 verbessert. Die Erfindung macht mehrere Verbesserungen am Datenakquisitionssystem und an der Analyseeinheit des Detektorsystems.

Die in der US2012/0043467 beschriebenen Datenakquisitionssysteme sortieren die in Koinzidenzereignissen registrierten Energiewerte $E_1$ und $E_2$ - ähnlich wie bei einem Gammaspektrometer - in zwei Histogramme ein. Anhand dieser Histogramme werden später Energiemittelwerte berechnet. Mit einem solchen Datenakquisitionssystem ist es aber sehr schwierig, mehrere Strahlungsquellen eines Nuklids separat zu erkennen. Die für die Entfaltung der Daten für mehrere Quellen notwendigen Informationen werden von einem solchen Datenakquisitionssystem nur unzureichend gespeichert. Die Energiewerte $E_1$ und $E_2$ in einem Koinzidenzereignis sind aufgrund der Energieerhaltung stark miteinander korreliert. Die in der Korrelation enthaltene Information geht jedoch verloren, wenn die Energiewerte in separaten eindimensionalen Histogrammen gespeichert werden.

Ein wesentliches Merkmal der Messwerterfassung des erfindungsgemäßen bildgebenden Detektorsystems ist, dass eine Funktion $\sigma(E_1, E_2)$ verwendet wird, die eine von $E_1$ und $E_2$ abhängige Funktion ist. Nur eine von beiden Werten $E_1$ und $E_2$ abhängige Funktion wie z.B. die Asymmetrie in Gl. (4) kann die Richtungsinformation der Koinzidenzereignisse adäquat repräsentieren. Wenn aber $E_1$ und $E_2$ - wie in der US2012/0043467 - in getrennten Histogrammen gespeichert werden, geht ein wesentlicher Teil der Richtungsinformation unwiederbringlich verloren.

Zusätzlich zur qualitativen Verbesserung in Bezug auf die Messgröße $\sigma(E_1, E_2)$ und deren richtungsabhängigen

EP 3 825 736 B1

Informationswert, gibt es eine quantitative Informationserweiterung in Bezug auf die Anzahl an Detektorpaaren, deren Messwerte während einer Messung gespeichert und dem Bildrekonstruktionsverfahren zugänglich gemacht werden. Die in der US2012/0043467 beschriebenen Verfahren berücksichtigen lediglich Koinzidenzen von Detektorpaaren entlang zweier ausgewählter Richtungen, alle anderen werden verworfen. Die Festlegung auf zwei Richtungen ist aber lediglich die minimal notwendige Auswahl, um den Azimut- und Höhenwinkel einer Strahlungsquelle zu berechnen. Dieser erhebliche Informationsverlust durch die willkürliche Einschränkung auf Detektorpaare entlang zweier durch den Nutzer ausgewählter Richtungen wird durch die Erfindung aufgehoben. Das erfindungsgemäße bildgebende Detektorsystem berücksichtigt alle Paarkombinationen von Strahlungsdetektoren, in denen mindestens ein Detektor eine Ordnungszahl $Z_{eff}$ > 30 besitzt. Ohne Einschränkung bezüglich der Richtung der Detektorpaare werden alle Detektorpaare, die im erfindungsgemäßen bildgebenden Detektorsystem vorhanden sind, in der Bildrekonstruktion genutzt.

Ferner beinhaltet das erfindungsgemäße bildgebende Detektorsystem eine Analyseeinheit mit einem Bildrekonstruktionsverfahren, mit dem ein Bild von einer Richtungs- bzw. einer Aktivitätsverteilung berechnet wird. Damit wird der Funktionsbereich der Single Plane Compton Kamera erweitert, der bisher auf die Richtungsmessung einer Strahlungsquelle pro Nuklid beschränkt war.

Das erfindungsgemäße bildgebende Detektorsystem kann flexibel an sehr unterschiedliche Einsatzbedingungen angepasst werden. U.a. unterstützt das erfindungsgemäße Detektorsystem den Einsatz verschiedenartiger Materialkombinationen für unterschiedliche Detektorausführungen, die ein breites Spektrum an Rekonstruktionsaufgaben abdecken.

Detektorsysteme vom Typ A *gemischt* und vom Typ B besitzen mehrere charakteristische Herausstellungsmerkmale, die bei der klassischen 2-Ebenen Compton Kamera so nicht vorhanden sind. Hervorzuheben ist, dass Detektorsysteme vom Typ A *gemischt* und vom Typ B als einlagige Flächenanordnungen von Strahlungsdetektoren realisiert werden können, seien dies nun ebene oder gekrümmte Flächen im Raum.

Bei den Detektorsystemen vom Typ B können die Detektoren in den einlagigen Flächenanordnungen aus einem einheitlichen Material bestehen. Es besteht keine Notwendigkeit mehr, zwei unterschiedliche Materialien auszuwählen. Die bisherige Anforderung, welche von 2-Ebenen Compton Kameras bekannt ist, dass ein Material eine hohe, das andere eine niedrige Ordnungszahl haben muss, gibt es bei Typ B Detektorsystemen nicht mehr. In Fällen, wenn nur wenige Materialien die konstruktiven Anforderungen an die Strahlungsdetektoren erfüllen, kann diese Eigenschaft von Typ B Detektorsystemen sehr nützlich sein. Ein Beispiel sind Detektorsysteme, die das Temporal Imaging Funktionsprinzip aus der US 9,638,811 B2 nutzen. Temporal Imaging Systeme vom Typ B benötigen nur ein Detektormaterial mittlerer bis hoher Ordnungszahl, welches die Anforderungen an die Pulsanstiegszeit von unter einer Nanosekunde erfüllt.

Die Detektortopologien der erfindungsgemäßen Typ A und Typ B Detektorsysteme sind sehr flexibel gestaltbar und können ein-, zwei- oder drei-dimensional sein. Die Dimensionalität des Detektoraufbaus bestimmt den Sichtbereich.

Das einfachste erfindungsgemäße bildgebende Detektorsystem ergibt sich, wenn mehrere Strahlungsdetektoren hintereinander in einer Reihe aufgestellt werden. Im Minimalfall genügen zwei Strahlungsdetektoren. Das 1-dimensionale Detektorsystem hat den Sichtbereich eines Halbkreises.

Gemäß einer Ausführungsform des erfindungsgemäßen bildgebenden Detektorsystems weist dieses mindestens zwei Strahlungsdetektoren auf, die in einer 1-dimensionalen Detektorreihe zueinander ausgebildet sind. Weiter weist ein solches System einen Sichtbereich von -90° bis +90° senkrecht zur Detektorreihe auf. Ein solches Detektorsystem wird im Sinne der Erfindung als 1-dimensional bezeichnet.

Ein erfindungsgemäßes Detektorsystem mit einer 2-dimensionalen Anordnung von Strahlungsdetektoren ist für Abbildungen in zwei Perspektiven geeignet. Hierbei wird im Sinne der Erfindung zwischen einem halbsphärischen Detektorsystem und einem 360° Panoramasystem unterschieden. Das halbsphärische Detektorsystem dient zur Abbildung von Strahlungsquellen in einem $2\pi$ Halbraum vor der Detektorebene. Das 360° Panoramasystem ist für die 2-dimensionale Ortung von Strahlungsquellen in der Detektorebene ausgelegt.

Gemäß einer Ausführungsform des erfindungsgemäßen bildgebenden Detektorsystems weist dieses mindestens drei Strahlungsdetektoren auf, die in einer im Wesentlichen 2-dimensionalen Anordnung zueinander ausgebildet sind. Weiter weist ein solches System einen Sichtbereich von einem $2\pi$ Raumwinkel für Strahlungsquellen in einem Halbraum vor der 2-dimensionalen Detektorebene auf. Ein solches Detektorsystem wird im Sinne der Erfindung als halbsphärisch bezeichnet.

Bei der Benutzung des halbsphärischen Detektorsystems ist darauf zu achten, dass sich alle Strahlungsquellen auf der gleichen Seite der Detektorebene befinden. Das halbsphärische Detektorsystem erfasst Strahlungsquellen aus dem gesamten $4\pi$ Raumwinkel, ohne dass der Nutzer feststellen kann, ob eine bestimmte Quelle vor oder hinter der Detektorebene liegt. Es wird implizit vorausgesetzt, dass der Nutzer weiß, auf welcher Seite der Detektorebene Strahlungsquellen vermutet werden. Das Detektorsystem ist so aufzustellen, dass alle Strahlungsquellen - vom Standpunkt des Betrachters ausgesehen - in der gleichen Hemisphäre vor der Detektorebene liegen.

Gemäß einer Ausführungsform des erfindungsgemäßen bildgebenden Detektorsystems weist dieses mindestens drei Strahlungsdetektoren auf, die in einer im Wesentlichen 2-dimensionalen Anordnung zueinander ausgebildet sind. Weiter weist ein solches System einen 360° Sichtbereich für Strahlungsquellen in der 2-dimensionalen Detektorebene auf. Ein solches Detektorsystem wird im Sinne der Erfindung als 360° Panoramasystem bezeichnet.

Das 360° Panoramasystem stellt eine neue Nutzungsform für die Single Plane Compton Kamera dar. Die im Stand der Technik beschriebenen Verfahren für Single Plane Compton Kameras sind für die Quellenortung im Sichtbereich einer Hemisphäre konzipiert; sie sind aber nicht anwendbar, wenn die Strahlungsquellen in der Detektorebene liegen. Die Verfahren nach dem Stand der Technik berechnen die Einfallsrichtung der Gammastrahlung als Schnittlinie zweier sogenannter Peilungsebenen. Im Grenzfall, wenn die Quelle in der Detektorebene liegt, sind jedoch die Peilungsebenen identisch mit der Detektorebene; eine Schnittlinie existiert nicht und die Richtung der Strahlungsquelle ist nicht bestimmbar. Das erfindungsgemäße 360° Panoramasystem löst dieses Problem und erlaubt eine 2-dimensionale Richtungsmessung für Strahlungsquellen in der Detektorebene.

Gemäß einer weiteren Ausführungsform des erfindungsgemäßen bildgebenden Detektorsystems ist ein Teil des Systems oder das ganze System als Single Plane Compton Kamera ausgebildet, die zu einem erfindungsgemäßen bildgebenden Detektorsystem vom Typ A *gemischt* und/oder zu einem erfindungsgemäßen bildgebenden Detektorsystem vom Typ B erweitert wird. Hierfür wird die Datenerfassung der Single Plane Compton Kamera, welche gemäß der US2012/0043467 lediglich Energiespektren von Koinzidenzereignissen speichert, auf List-Mode umgestellt. Die Analyseeinheit wird so eingerichtet, dass für jedes Koinzidenzereignis eine von beiden Energiewerten $E_1$ und $E_2$ abhängige Funktion $\sigma(E_1, E_2)$ berechnet wird. Die Menge der Detektorpaare wird so erweitert, dass alle Paarkombinationen von Detektoren, welche erfindungsgemäß zum Detektorsystem gehören, in die Bildrekonstruktion einfließen. Insbesondere sollen Paarkombinationen in allen verfügbaren Richtungen berücksichtigt werden. Die Systemelektronik ist so auszulegen, dass die Daten aller ausgewählten Detektorpaare erfasst werden. Ferner erstellt die Analyseeinheit aus den List-Mode Daten der Single Plane Compton Kamera die für die Bildrekonstruktion benötigten Projektionsdaten $p(\underline{y})$ gemäß der Vorgehensweise dieser Erfindung. Nach der Umstellung liegt die Single Plane Compton Kamera in einer von zwei Ausführungsformen vor: als erfindungsgemäßes bildgebendes Detektorsystem vom Typ A *gemischt* und/oder als erfindungsgemäßes bildgebendes Detektorsystem vom Typ B. Die auf ein erfindungsgemäßes bildgebendes Detektorsystem erweiterte Single Plane Compton Kamera kann als halbsphärisches und/oder als 360° Panoramasystem genutzt werden.

Gemäß einer Ausführungsform des erfindungsgemäßen bildgebenden Detektorsystems weist dieses mindestens vier Strahlungsdetektoren auf, die in einer im Wesentlichen 3-dimensionalen Anordnung zueinander ausgebildet sind. Weiter besitzt das System hinreichend viele Detektorpaare, deren Achsen gut über den $4\pi$ Raumwinkel verteilt sind. Ein solches System weist einen Sichtbereich von einem $4\pi$ Raumwinkel auf und wird im Sinne der Erfindung als vollsphärisch bezeichnet.

Ausführungsformen für 3-dimensionale erfindungsgemäße bildgebende Detektorsysteme sind besonders vorteilhaft für Anwendungen im medizinischen Gerätebau. Ausführungsbeispiele 3-dimensionaler Detektorsysteme vom Typ B aus Modulen baugleicher Detektoren in einlagigen Flächenanordnungen bieten vielfältige konstruktive Vorteile. Beispielsweise kann ein SPECT Scanner für hochenergetische Radionuklide wie z.B. I-131 als Detektorsystem vom Typ B realisiert werden. Für SPECT Scanner nach dem Stand der Technik, die das Anger Gammakamera Prinzip nutzen, ist der hochenergetische Energiebereich bisher nur schwer zugänglich. Insbesondere können kombinierte SPECT und PET Scanner gemäß den erfindungsgemäßen bildgebenden Detektorsystemen vom Typ B konzipiert werden. Die im klinischen Umfeld weit verbreiteten Vollring-PET-Systeme besitzen viele gerätetechnische Gemeinsamkeiten mit den hier vorgeschlagenen erfindungsgemäßen bildgebenden Detektorsystemen vom Typ B. Für eine kombinierte Nutzung als SPECT und PET Scanner sind evt. neue Szintillationsmaterialien in Betracht zu ziehen, deren Ordnungszahl in einem mittleren Bereich liegt und welche die Compton-Streuung stärker begünstigen als bisherige PET Szintillationskristalle. Ein PET/SPECT Hybrid-Scanner gemäß einer der Ausführungsformen der erfindungsgemäßen bildgebenden Detektorsysteme ist insbesondere für Radionuklide wie z.B. I-124 geeignet, die zugleich Positronen- und Single Photon-Emitter sind. Für solche Radionuklide ist die PET/SPECT Hybridisierung besonders sinnvoll, da beide Strahlungsarten in der Bildrekonstruktion kombiniert werden können. Auch können die PET Scanner durch die Berücksichtigung von Compton Streuereignissen profitieren, die bisher nur selten in der Bildrekonstruktion genutzt werden.

Detektorsysteme vom Typ A *gemischt* sind denen vom Typ B insofern ähnlich, als diese auch einlagige Flächenanordnungen bilden. Die heterogenen Detektorflächen von Typ A *gemischt* Systemen bieten Vorteile im niederenergetischen Bereich unter 300 keV, wenn die Wahrscheinlichkeit für Compton-Streuung in vielen Materialien nachlässt. Es ist dann vorteilhaft, Detektoren niedriger Ordnungszahl in Typ A *gemischt* Systeme zu integrieren, um eine gute Detektionseffizienz zu gewährleisten. Detektorsysteme vom Typ A *gemischt* stellen eine geeignete Bauform für einen SPECT Scanner für Radiopharmaka auf Basis von Tc-99m dar.

Gemäß einer weiteren Ausführungsform des erfindungsgemäßen bildgebenden Detektorsystems ist ein Teil des Systems oder das ganze System als PET Scanner ausgebildet, der zu einem erfindungsgemäßen bildgebenden Detektorsystem vom Typ B und/oder zu einem PET/SPECT Hybrid-Scanner erweitert wird. Hierfür werden ein Teil oder alle der im PET Scanner vorhandenen Szintillationsdetektoren samt Elektronik, der Systemelektronik und der Analyseeinheit als erfindungsgemäßes bildgebendes Detektorsystem genutzt. Für eine Nutzung als erfindungsgemäßes bildgebendes Detektorsystem ist der PET Scanner in Verbindung mit einem Radiopharmakon einzusetzen, welches Single Photon Gammastrahlung emittiert. In einer Ausführungsform der Erfindung emittiert das Radiopharmakon sowohl Single Photon Gammastrahlung als auch Positronen.

Für die Erweiterung des PET Scanners auf ein erfindungsgemäßes bildgebendes Detektorsystem ist eine neue Systemmatrix $\underline{H}$ zu erstellen, die den PET Scanner als Single Photon Compton Scanner vom Typ B beschreibt. Die Analyseeinheit speichert die Daten im List-Mode Format, wie es für PET Scanner üblich ist. Die Datenerfassung ist auf den Energiebereich der Single Photon Gammastrahlung anzupassen. Es sollen Koinzidenzereignisse registriert werden, deren Energiesumme $E_1 + E_2$ auf die charakteristische Radionuklid-Energie des Radiopharmakons abgestimmt ist. Die Projektionsdaten $\underline{p}(\underline{y})$ sind gemäß der Vorgehensweise dieser Erfindung aus den List-Mode Daten zu erstellen. Die Analyseeinheit verarbeitet die Projektionsdaten $\underline{p}(\underline{y})$ unter Verwendung der Systemmatrix $\underline{H}$. Optional können auch Teile der zum PET Scanner gehörigen Bildrekonstruktionssoftware durch das erfindungsgemäße bildgebende Detektorsystem genutzt werden, wenn diese für eine solche Nutzung geeignet ist. Nach der Erweiterung liegt der PET Scanner in einer von zwei Ausführungsformen vor: als erfindungsgemäßes bildgebendes Detektorsystem vom Typ B und/oder als erfindungsgemäßer PET/SPECT Hybrid-Scanner. Beide Ausführungsformen sind erfindungsgemäße bildgebende Detektorsysteme für das Nahfeld. Die Ausführungsform des PET/SPECT Hybrid-Scanners kann zur Bildgebung mit Radionukliden genutzt werden, die (a) Positronen emittieren, (b) Single Photon Gammastrahlung emittieren bzw. (c) gleichzeitig Positronen und Single Photon Gammastrahlung emittieren. In der Ausführungsform (a) wird der PET/SPECT Hybrid-Scanner mit der PET Bildrekonstruktions-Software betrieben, in der Ausführungsform (b) mit der erfindungsgemäßen Bildrekonstruktions-Software und in der Ausführungsform (c) werden beide Bildrekonstruktionsverfahren zu einem PET/SPECT Hybrid-Verfahren kombiniert. Das Bildrekonstruktionsverfahren in der Ausführungsform (c) nutzt sowohl die Bildrekonstruktions-Software des PET-Scanners als auch die zum erfindungsgemäßen bildgebenden Detektorsystem gehörige Bildrekonstruktions-Software, die miteinander fusioniert werden.

Nachfolgend wird das erfindungsgemäße bildgebende Detektorsystem an Hand von Ausführungsbeispielen und in Zeichnungen näher beschrieben. Die Erläuterungen sind lediglich beispielhaft und schränken den allgemeinen Erfindungsgedanken nicht ein. Auch können Ausführungsbeispiele beliebig kombiniert werden. Beispiele für das erfindungsgemäße Verfahren sind ebenfalls in Ausführungsbeispielen angegeben.

Gemäß einem vorteilhaften Ausführungsbeispiel des erfindungsgemäßen bildgebenden Detektorsystems detektiert dieses Strahlungsfelder, die eine diskrete und/oder eine kontinuierliche Verteilung von Strahlung aufweisen, wobei sich das Detektorsystem im geometrischen Nah- und/oder Fernfeld des Strahlungsfeldes befindet.

Gemäß einem weiteren vorteilhaften Ausführungsbeispiel des erfindungsgemäßen bildgebenden Detektorsystems detektiert dieses Strahlungsquellen, die Gamma-, Elektronen- , Positronen-, Protonen-, Ionen- und/oder Neutronenstrahlung emittieren.

Gemäß einem weiteren vorteilhaften Ausführungsbeispiel des erfindungsgemäßen bildgebenden Detektorsystems stammt die Strahlung aus dem radioaktiven Zerfall eines oder mehrerer Radionuklide und/oder ist die Strahlung die prompte Gammastrahlung, welche bei der Absorption von Protonen- oder Ionenstrahlung in Targetmaterialien entsteht.

Gemäß einem weiteren vorteilhaften Ausführungsbeispiel des erfindungsgemäßen bildgebenden Detektorsystems weist die Strahlung eine niedrige Intensität auf, wie dies z.B. in der Astronomie der Fall ist.

Gemäß einem weiteren vorteilhaften Ausführungsbeispiel des erfindungsgemäßen bildgebenden Detektorsystems weisen die Detektoren einen Szintillator mit einem Photodetektor und/oder ein Halbleitermaterial auf. Der Szintillator kann ein reines oder dotiertes Material der Gruppe aus PVT, Anthracen, Stilben, p-Terphenyl, $CaF_2$, $BaF_2$, NaI, $CeBr_3$, $LaBr_3$, $LaCl_3$, $La(Br_xCl_{1-x})_3$, CsI, $SrI_2$, CLYC, CLBC, CLCB, CLLB, BGO, LSO, LYSO, GAGG, YAP und/oder YAG umfassen. Weiter kann der Szintillator als monolithischer Block oder als pixeliertes Szintillatormodul vorliegen. Darüber hinaus kann der Photodetektor ein Photomultiplier (PMT), ein ortsauflösender Photomultiplier (PSPMT), ein Silizium Photomultiplier (SiPM), ein ortsauflösender Silizium Photomultiplier (PS-SiPM) und/oder eine Silizium Photodiode sein. Erfindungsgemäß kann das Halbleitermaterial ein Material aus der Gruppe von Si, Ge, GaAs, CdTe und/oder CdZnTe umfassen und/oder eine planare oder koaxiale Geometrie aufweisen. Auch kann das Halbleitermaterial mit segmentierten oder unsegmentierten Kontakten vorliegen.

Gemäß einem weiteren vorteilhaften Ausführungsbeispiel des erfindungsgemäßen bildgebenden Detektorsystems sind die Detektormaterialien virtuell oder physisch in eine beliebige ganzzahlige Anzahl von $\geq 1$ Voxeln unterteilt.

Gemäß einem weiteren vorteilhaften Ausführungsbeispiel des erfindungsgemäßen bildgebenden Detektorsystems verwendet die Systemelektronik analoge und/oder digitale Elektronikkomponenten.

Gemäß einem weiteren vorteilhaften Ausführungsbeispiel des erfindungsgemäßen bildgebenden Detektorsystems umfassen die analogen Elektronikkomponenten eine Kombination verschiedener Module, zu denen eine Hochspannungsversorgung, ein Vorverstärker, ein Verstärker, ein Pulsformer, ein Ladungsintegrator, ein Pulshöhenanalysator, ein Multikanalanalysator (MCA) und/oder eine Koinzidenzschaltung zählen.

Gemäß einem weiteren vorteilhaften Ausführungsbeispiel des erfindungsgemäßen bildgebenden Detektorsystems umfassen die digitalen Elektronikkomponenten eine Kombination verschiedener Hard- und Softwarekomponenten, zu denen eine Hochspannungsversorgung, ein A/D-Wandler pro Detektorvoxel, ein Field Programmable Gate Array (FPGA), ein Speichermedium, ein digitaler Signalprozessor und/oder eine Auswertesoftware zählen.

Gemäß einem weiteren vorteilhaften Ausführungsbeispiel des erfindungsgemäßen bildgebenden Detektorsystems werden die Wechselwirkungspunkte ($\underline{d}_1$,$\underline{d}_2$) anhand der ortsauflösenden Eigenschaften der Detektoren und/oder anhand der vorliegenden Segmentierung der Detektoren bestimmt.

Gemäß einem weiteren vorteilhaften Ausführungsbeispiel des erfindungsgemäßen bildgebenden Detektorsystems sind die Wechselwirkungspunkte ($\underline{d}_1$, $\underline{d}_2$) als die räumlichen Mittelpunkte der an einem Koinzidenzereignis beteiligten Detektorvoxel eines ersten und eines zweiten Detektorvoxels definiert.

Gemäß einem weiteren vorteilhaften Ausführungsbeispiel des erfindungsgemäßen bildgebenden Detektorsystems ist der Funktionswert $\sigma(E_1, E_2)$ gemäß

$$\sigma(E_1, E_2) = (E_2 - E_1)/(E_1 + E_2)$$

definiert.

Gemäß einem weiteren vorteilhaften Ausführungsbeispiel des erfindungsgemäßen bildgebenden Detektorsystems verwendet die Analyseeinheit zur Rekonstruktion des Strahlungsfeldes Projektionsdaten $\underline{p}(\underline{y})$, die als Funktion der Attribute $\underline{y} = \{\underline{d}_1, E_1, \underline{d}_2, E_2\}$ definiert sind.

Gemäß einem weiteren vorteilhaften Ausführungsbeispiel des erfindungsgemäßen bildgebenden Detektorsystems wird für jedes Element von $\underline{p}$ eine Anzahl von Koinzidenzereignissen ermittelt, die bei einer jeweiligen Kombination eines bestimmten ersten Detektorvoxels mit einem bestimmten zweiten Detektorvoxel bei einem bestimmten Funktionswert $\sigma(E_1, E_2)$ auftritt.

Gemäß einem weiteren vorteilhaften Ausführungsbeispiel des erfindungsgemäßen bildgebenden Detektorsystems wird für jedes Element von $\underline{p}$ eine Anzahl von **Koinzidenzereignissen** ermittelt, die bei einer jeweiligen Kombination eines bestimmten ersten Detektionsorts $\underline{d}_1$ mit einem bestimmten zweiten Detektionsort $\underline{d}_2$ bei einem bestimmten Funktionswert $\sigma(E_1, E_2)$ auftritt.

Gemäß einem weiteren vorteilhaften Ausführungsbeispiel des erfindungsgemäßen bildgebenden Detektorsystems wird eine Selektionsbedingung für Koinzidenzereignisse bezüglich der Energiesumme $E_1 + E_2$ der in beiden Detektorvoxeln eines Paares detektierten Energien angewendet.

Gemäß einem weiteren vorteilhaften Ausführungsbeispiel des erfindungsgemäßen bildgebenden Detektorsystems werden separate Projektionsdaten $\underline{p}(\underline{y})$ für jedes detektierte Radionuklid angelegt und/oder die Analyseeinheit berechnet für jedes Radionuklid ein separates Bild $f(\underline{x})$.

Gemäß einem weiteren vorteilhaften Ausführungsbeispiel des erfindungsgemäßen bildgebenden Detektorsystems stellen die Bilder $f(\underline{x})$ eine Aktivitätsdichte, eine Flussdichte und/oder eine Dosisleistungsdichte dar und/oder die Bilder sind $f(\underline{x})$ tomographische Schnittbilder einer Aktivitätsverteilung von Radionukliden (z.B. auch Radiopharmaka).

Gemäß einem weiteren vorteilhaften Ausführungsbeispiel des erfindungsgemäßen bildgebenden Detektorsystems weisen alle Detektoren im Wesentlichen eine gleiche Bauart auf oder mindestens zwei der Detektoren weisen eine voneinander verschiedene Bauart auf.

Gemäß einem weiteren vorteilhaften Ausführungsbeispiel des erfindungsgemäßen bildgebenden Detektorsystems weist dieses mindestens vier Detektorvoxel in einer im Wesentlichen 3-dimensionalen Anordnung auf und verfügt über einen Sichtbereich von einem $4\pi$ Raumwinkel.

Gemäß einem weiteren vorteilhaften Ausführungsbeispiel des erfindungsgemäßen bildgebenden Detektorsystems weist dieses mindestens drei Detektorvoxel in einer im Wesentlichen 2-dimensionalen Anordnung auf und verfügt über einen halbsphärischen Sichtbereich mit einem $2\pi$ Raumwinkel.

Gemäß einem weiteren vorteilhaften Ausführungsbeispiel des erfindungsgemäßen bildgebenden Detektorsystems weist dieses mindestens drei Detektorvoxel in einer im Wesentlichen 2-dimensionalen Anordnung auf und verfügt über einen 360° Sichtbereich in der Ebene der Strahlungsdetektoren.

Gemäß einem weiteren vorteilhaften Ausführungsbeispiel des erfindungsgemäßen bildgebenden Detektorsystems weist dieses mindestens zwei Detektorvoxel in einer 1-dimensionalen Detektorreihe auf und verfügt über einen 180° Sichtbereich von -90° bis +90° senkrecht zur Detektorreihe.

Gemäß einem weiteren vorteilhaften Ausführungsbeispiel des erfindungsgemäßen bildgebenden Detektorsystems weist dieses eine erste Gruppe von Detektoren/Voxeln mit einer Ordnungszahl $Z_{eff} > 30$ auf, die hohe Wahrscheinlichkeiten für photoelektrische Absorption im Energiebereich von 100 keV bis 3 MeV besitzen. Weiter kann das Detektorsystem optional eine zweite Gruppe von Detektoren/Voxeln mit einer Ordnungszahl $Z_{eff} \leq 30$ umfassen, die hohe Wahrscheinlichkeiten für Compton-Streuung im Energiebereich von 100 keV bis 3 MeV besitzen.

Gemäß einem weiteren vorteilhaften Ausführungsbeispiel des erfindungsgemäßen bildgebenden Detektorsystems werden Daten von allen Detektorpaaren/Voxelpaaren erfasst, die kombinatorisch aus der Menge aller Detektoren/Voxel gebildet werden können, wobei solche Detektorpaare/Voxelpaare ausgenommen sind in denen beide Detektoren/Voxel zur zweiten Gruppe mit Ordnungszahl $Z_{eff} \leq 30$ gehören.

Gemäß einem weiteren vorteilhaften Ausführungsbeispiel des erfindungsgemäßen bildgebenden Detektorsystems sind die gemischten Detektorpaare/Voxelpaare, die je einen Detektor/Voxel aus der ersten Gruppe und einen Detektor/Voxel aus der zweiten Gruppe aufweisen, unidirektional, wobei die Strahlung vornehmlich in eine Richtung gestreut wird.

Gemäß einem weiteren vorteilhaften Ausführungsbeispiel des erfindungsgemäßen bildgebenden Detektorsystems sind die Detektorpaare/Voxelpaare der ersten Gruppe mit Ordnungszahl $Z_{eff} > 30$, bidirektional, wobei die Strahlung in beide Richtungen mit ähnlicher Intensität gestreut wird.

Gemäß einem weiteren vorteilhaften Ausführungsbeispiel des erfindungsgemäßen bildgebenden Detektorsystems weist die Gesamtheit der Detektoren/Voxel die das Detektorsystem umfasst eine erste homogene oder heterogene ringförmige, röhrenförmige, zylindrische, kugelförmige, polyedrische und/oder sonstige geometrische Form auf.

Gemäß einem weiteren vorteilhaften Ausführungsbeispiel des erfindungsgemäßen bildgebenden Detektorsystems weist die Gesamtheit der Detektoren/Voxel die das Detektorsystem umfasst zusätzlich zur ersten Form eine zweite homogene oder heterogene ringförmige, röhrenförmige, zylindrische, kugelförmige, polyedrische und/oder sonstige geometrische Form auf. Wobei die zweite Form in die erste Form eingeschrieben ist oder wobei die zweite Form in einem inneren Bereich angeordnet ist, den die erste Form als äußere Hülle umschließt.

Gemäß einem weiteren vorteilhaften Ausführungsbeispiel des erfindungsgemäßen bildgebenden Detektorsystems ist die erste und/oder die zweite Form um einen oder mehrere Zentraldetektoren angeordnet.

Gemäß einem weiteren vorteilhaften Ausführungsbeispiel des erfindungsgemäßen bildgebenden Detektorsystems ist ein Teil des Systems oder das ganze System als Ortungsgerät für Strahlungsquellen, als Compton Kamera, als Compton Teleskop, als Detektorsystem gemäß beispielsweise der US 9,638,811 B2, als SPECT Scanner, als PET/-SPECT Hybrid-Scanner, als SPECT Sonde und/oder als PET/SPECT Hybrid-Sonde, ausgebildet.

Gemäß einem vorteilhaften Ausführungsbeispiel des erfindungsgemäßen Verfahrens umfasst dieses zusätzlich in Teilen oder in Gänze die folgenden Schritte:

- Kalibrieren der Signale aller Detektorvoxel als absorbierte Strahlungsenergie E;
- Festlegen eines geeigneten Koordinatensystems;
- Anlegen eines oder mehrerer Projektionsdatensätze $\underline{p}(\underline{y})$, welche die Anzahlen von Koinzidenzereignissen erfassen, die bei einer jeweiligen Kombination von einem bestimmten ersten Detektorvoxel $\underline{d}_1$ mit einem bestimmten zweiten Detektorvoxel $\underline{d}_2$ bei einem bestimmten Funktionswert $\sigma(E_1, E_2)$ gezählt werden, wobei für jedes Radionuklid ein separater Projektionsdatensatz $\underline{p}(\underline{y})$ angelegt wird;
- Anlegen einer oder mehrerer Systemmatrizen $\underline{H}$ für jedes zu detektierende Radionuklid;
- Erstellen und Validieren der Systemmatrizen $\underline{H}$ durch Messungen mit dem Detektorsystem oder durch Monte Carlo Simulationen oder mittels eines theoretischen Modells;
- Übergeben sämtlicher Systemmatrizen $\underline{H}$ an einen Algorithmus der Bildrekonstruktion, welcher die Projektionsdaten $\underline{p}(\underline{y})$ verarbeitet und die Bilder $f(x)$ berechnet.

Es zeigen:

Abb. 1 ein Klassifizierungsschema für bildgebende Detektorsysteme in Typ A *getrennt,* Typ A *gemischt* und Typ B, gemäß dreier Ausführungsformen des erfindungsgemäßen bildgebenden Detektorsystems;

Abb. 2 eine nicht-maßstabsgetreue, schematische Darstellung von ringförmigen Detektorsystemen vom Typ A *getrennt,* Typ A *gemischt* und Typ B, gemäß dreier Ausführungsformen des erfindungsgemäßen bildgebenden Detektorsystems;

Abb. 3 eine nicht-maßstabsgetreue, schematische Darstellung eines Detektorsystems aus zwei Detektoren mit Komponenten der Systemelektronik, gemäß einer Ausführungsform des erfindungsgemäßen bildgebenden Detektorsystems;

Abb. 4a eine nicht-maßstabsgetreue, schematische Darstellung eines erfindungsgemäßen Detektorsystems aus zwei Detektoren;

Abb. 4b eine nicht-maßstabsgetreue, schematische Darstellung der Datenerfassung eines Detektorsystems aus zwei Detektoren, gemäß einer Ausführungsform des erfindungsgemäßen bildgebenden Detektorsystems;

Abb. 5 eine nicht-maßstabsgetreue, schematische Darstellung zur Auswahl und Kennzeichnung von Detektorpaaren am Beispiel eines Detektorsystems aus drei Detektoren, gemäß einer Ausführungsform des erfindungsgemäßen bildgebenden Detektorsystems;

Abb. 5a eine nicht-maßstabsgetreue, schematische Darstellung zur Auswahl und Kennzeichnung eines ersten Detektorpaares P1 im Detektorsystem von Abb. 5, gemäß einer Ausführungsform des erfindungsgemäßen bildgebenden Detektorsystems;

Abb. 5b eine nicht-maßstabsgetreue, schematische Darstellung zur Auswahl und Kennzeichnung eines zweiten Detektorpaares P2 im Detektorsystem von Abb. 5, gemäß einer Ausführungsform des erfindungsgemäßen bildgebenden Detektorsystems;

Abb. 5c eine nicht-maßstabsgetreue, schematische Darstellung zur Auswahl und Kennzeichnung eines dritten Detektorpaares P3 im Detektorsystem von Abb. 5, gemäß einer Ausführungsform des erfindungsgemäßen bildgebenden Detektorsystems;

Abb. 6a eine nicht-maßstabsgetreue, schematische Darstellung eines erfindungsgemäßen Detektorsystems aus drei Detektoren;

Abb. 6b eine nicht-maßstabsgetreue, schematische Darstellung der Datenerfassung eines Detektorsystems aus drei Detektoren, gemäß einer Ausführungsform des erfindungsgemäßen bildgebenden Detektorsystems;

Abb. 7 eine nicht-maßstabsgetreue, schematische Darstellung zur Auswahl und Kennzeichnung von Detektorpaaren am Beispiel eines Detektorsystems aus vier Detektoren, gemäß einer Ausführungsform des erfindungsgemäßen bildgebenden Detektorsystems;

Abb. 7a eine nicht-maßstabsgetreue, schematische Darstellung zur Auswahl und Kennzeichnung von Detektorpaaren einer ersten Richtung P1 im Detektorsystem von Abb. 7, gemäß einer Ausführungsform des erfindungsgemäßen bildgebenden Detektorsystems;

Abb. 7b eine nicht-maßstabsgetreue, schematische Darstellung zur Auswahl und Kennzeichnung von Detektorpaaren einer zweiten Richtung P2 im Detektorsystem von Abb. 7, gemäß einer Ausführungsform des erfindungsgemäßen bildgebenden Detektorsystems;

Abb. 7c eine nicht-maßstabsgetreue, schematische Darstellung zur Auswahl und Kennzeichnung von Detektorpaaren einer dritten Richtung P3 im Detektorsystem von Abb. 7, gemäß einer Ausführungsform des erfindungsgemäßen bildgebenden Detektorsystems;

Abb. 7d eine nicht-maßstabsgetreue, schematische Darstellung zur Auswahl und Kennzeichnung von Detektorpaaren einer vierten Richtung P4 im Detektorsystem von Abb. 7, gemäß einer Ausführungsform des erfindungsgemäßen bildgebenden Detektorsystems;

Abb. 8a eine nicht-maßstabsgetreue, schematische Darstellung eines erfindungsgemäßen Detektorsystems aus vier Detektoren;

Abb. 8b eine nicht-maßstabsgetreue, schematische Darstellung der Datenerfassung eines Detektorsystems aus vier Detektoren, gemäß einer Ausführungsform des erfindungsgemäßen bildgebenden Detektorsystems;

Abb. 9a eine nicht-maßstabsgetreue, schematische Darstellung einer 2-Ebenen Compton Kamera, gemäß einer Ausführungsform des Stands der Technik;

Abb. 9b eine nicht-maßstabsgetreue, schematische Darstellung einer 2-Ebenen Compton Kamera, die als erfindungsgemäßes bildgebendes Detektorsystem vom Typ A *getrennt* ausgeführt ist, gemäß einer Ausführungsform des erfindungsgemäßen bildgebenden Detektorsystems;

Abb. 10a eine nicht-maßstabsgetreue, schematische Darstellung eines 2-dimensionalen Detektorsystems mit halbsphärischem Sichtbereich, gemäß einer Ausführungsform des erfindungsgemäßen bildgebenden Detektorsystems;

Abb. 10b eine nicht-maßstabsgetreue, schematische Darstellung eines 2-dimensionalen Detektorsystems mit 360° Sichtbereich in der Detektorebene, gemäß einer Ausführungsform des erfindungsgemäßen bildgebenden Detektorsystems;

Abb. 11a eine nicht-maßstabsgetreue, schematische Darstellung eines vollsphärischen Detektorsystems vom Typ A, gemäß einer Ausführungsform des erfindungsgemäßen bildgebenden Detektorsystems;

Abb. 11b eine nicht-maßstabsgetreue, schematische Darstellung eines vollsphärischen Detektorsystems vom Typ B, gemäß einer Ausführungsform des erfindungsgemäßen bildgebenden Detektorsystems;

Abb. 12a eine nicht-maßstabsgetreue, schematische Darstellung eines 2-dimensionalen Detektorsystems vom Typ A, gemäß einer Ausführungsform des erfindungsgemäßen bildgebenden Detektorsystems;

Abb. 12b eine nicht-maßstabsgetreue, schematische Darstellung eines würfelförmigen Detektorsystems vom Typ A, gemäß einer Ausführungsform des erfindungsgemäßen bildgebenden Detektorsystems;

Abb. 12c eine nicht-maßstabsgetreue, schematische Darstellung eines tetraederförmigen Detektorsystems vom Typ A, gemäß einer Ausführungsform des erfindungsgemäßen bildgebenden Detektorsystems;

Abb. 13a eine nicht-maßstabsgetreue, schematische Darstellung eines tetraederförmigen Detektorsystems vom Typ B, gemäß einer Ausführungsform des erfindungsgemäßen bildgebenden Detektorsystems;

Abb. 13b eine nicht-maßstabsgetreue, schematische Darstellung eines würfelförmigen Detektorsystems vom Typ B, gemäß einer Ausführungsform des erfindungsgemäßen bildgebenden Detektorsystems

Abb. 14 ein Diagramm mit Simulationsergebnissen für Detektorpaare aus einem Cerbromid- und einem Plastik-Detektor, gemäß einer Ausführungsform des erfindungsgemäßen bildgebenden Detektorsystems;

Abb. 15 ein Diagramm mit Simulationsergebnissen für Detektorpaare aus zwei Cerbromid-Detektoren, gemäß einer Ausführungsform des erfindungsgemäßen bildgebenden Detektorsystems;

Abb. 16 drei Diagramme mit Simulationsergebnissen für Cerbromid-Plastik und Cerbromid-Cerbromid Detektorpaare, gemäß einer Ausführungsform des erfindungsgemäßen bildgebenden Detektorsystems;

Abb. 17 eine nicht-maßstabsgetreue, schematische Darstellung eines Messaufbaus für ein 1-dimensionales Detektorsystem aus zwei Strahlungsdetektoren, gemäß einer Ausführungsform des erfindungsgemäßen bildgebenden Detektorsystems;

Abb. 18 ein Diagramm mit Messergebnissen für die in Abb. 17 gezeigte Zwei-Detektor-Anordnung zur Richtungsmessung einer Co-60 Quelle, gemäß einer Ausführungsform des erfindungsgemäßen bildgebenden Detektorsystems;

Abb. 19 ein Diagramm mit Messergebnissen für die in Abb. 17 gezeigte Zwei-Detektor-Anordnung zur Richtungsmessung einer Cs-137 Quelle, gemäß einer Ausführungsform des erfindungsgemäßen bildgebenden Detektorsystems;

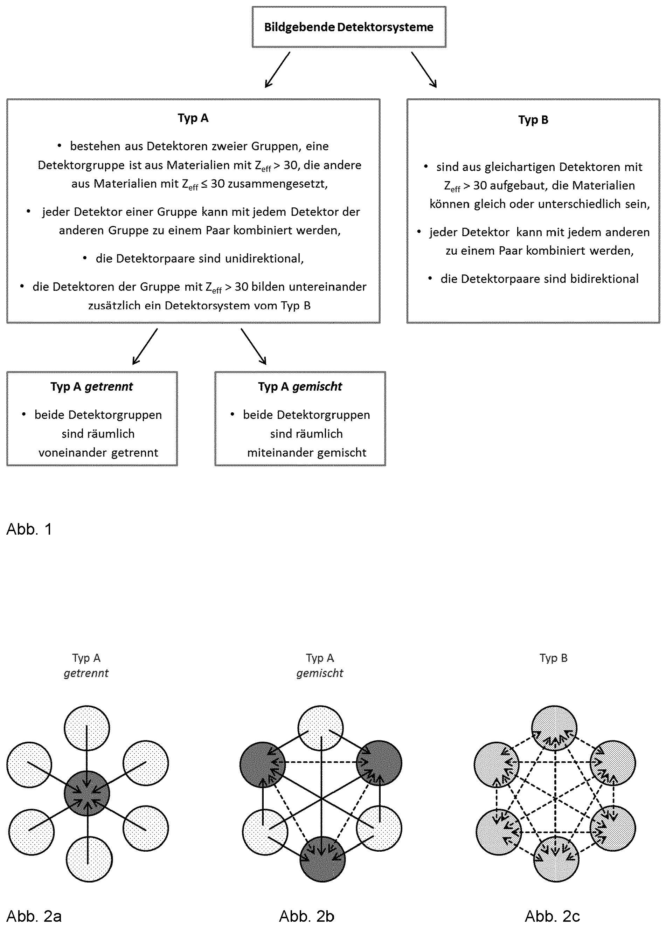

Abb. 1 zeigt ein Klassifizierungsschema für Detektorsysteme vom Typ A und Typ B. Typ A Detektorsysteme können entsprechend der räumlichen Anordnung der Detektoren weiter differenziert werden nach Typ A *getrennt* und Typ A *gemischt.*

Abb. 2 verdeutlicht am Beispiel von ringförmigen Detektorsystemen das Klassifizierungsschema von Abb. 1. In der Anordnung Typ A *getrennt* bilden die Detektoren niedriger Ordnungszahl einen äußeren Ring um einen inneren Zentraldetektor mittlerer bis hoher Ordnungszahl. In der Anordnung Typ A *gemischt* gibt es keinen Zentraldetektor, jeder Detektor im Ring ist von zwei Detektoren der jeweils anderen Gruppe umgeben. Die Anordnung vom Typ B ist ein Ring gleichartiger Detektoren, in dem alle Detektoren aus einem Material mittlerer bis hoher Ordnungszahl bestehen.

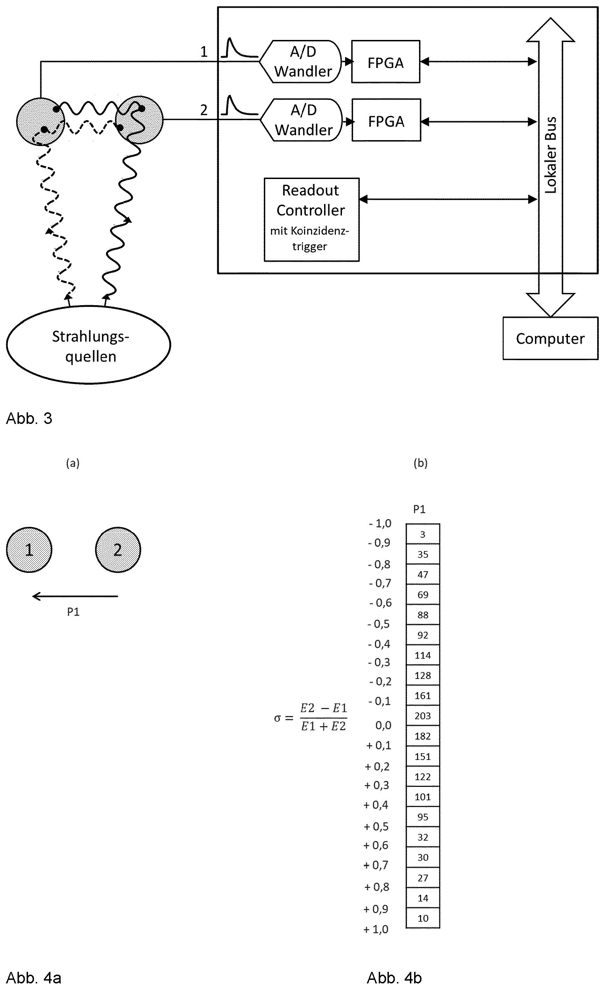

Abb. 3 zeigt ein Blockdiagramm mit einer Ausführung der Systemelektronik für ein Detektorsystem aus zwei Detektoren. Das gezeigte Detektorpaar besteht aus zwei Detektoren mittlerer bis hoher Ordnungszahl. Der Strahlungsfluss zwischen den Detektoren ist bidirektional.

Abb. 4 illustriert schematisch die Datenerfassung am Beispiel eines Detektorsystems aus zwei Detektoren mittlerer bis hoher Ordnungszahl. Abb. 4a zeigt das Detektorsystem mit dem Detektorpaar P1. Abb. 4b zeigt die Datenerfassung für das Detektorsystem in Abb. 4a. Für das Detektorpaar P1 werden die Anzahlen an Koinzidenzereignissen ermittelt, die bei einem bestimmten Wert einer von beiden Energiewerten $E_1$ und $E_2$ abhängigen Funktion $\sigma(E_1, E_2)$ registriert werden. Die in Abb. 4b gezeigte Tabelle ist eine tabellarische Darstellung der Projektionsdaten $\underline{p}(\underline{y})$ gemäß dieser Erfindung.

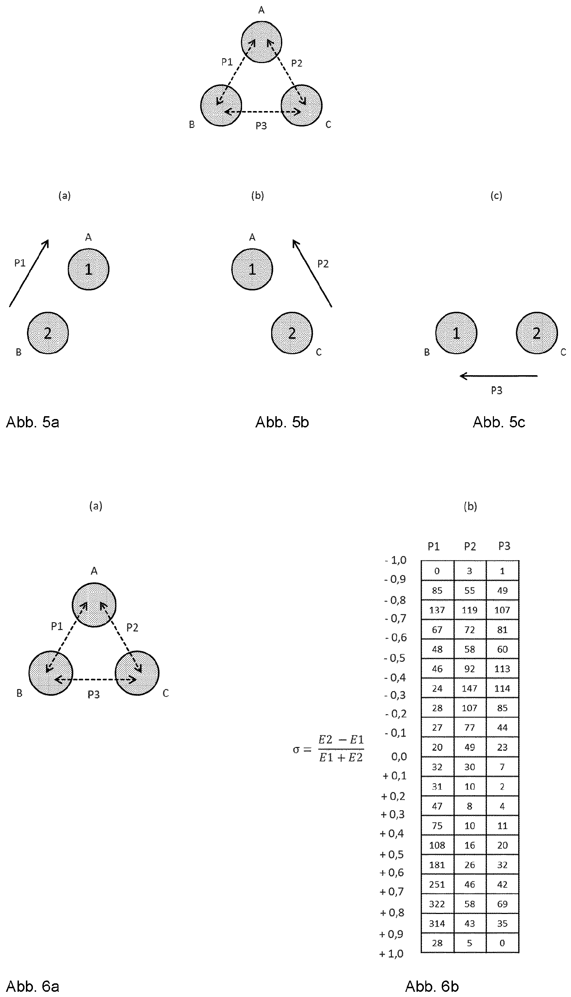

Abb. 5 illustriert schematisch die Auswahl und Kennzeichnung von Detektorpaaren am Beispiel eines Detektorsystems aus drei Detektoren mittlerer bis hoher Ordnungszahl. Das in Abb. 5 dargestellte Detektorsystem besitzt drei bidirektionale Detektorpaare P1, P2 und P3. Abb. 5a, 5b und 5c zeigen Beispiele für die Kennzeichnung der Detektoren eines ersten Paares P1 (Abb. 5a), eines zweiten Paares P2 (Abb. 5b) und eines dritten Paares P3 (Abb. 5c) mit den Nummer 1 bzw. 2.

Abb. 6 illustriert schematisch die Datenerfassung am Beispiel des Detektorsystems aus Abb. 5. Abb. 6a zeigt das Detektorsystem mit drei Detektorpaaren P1, P2 und P3. Abb. 6b zeigt die Datenerfassung für das Detektorsystem in Abb. 6a. Für jedes Detektorpaar P1, P2 und P3 werden die Anzahlen an Koinzidenzereignissen ermittelt, die bei einem bestimmten Wert einer von beiden Energiewerten $E_1$ und $E_2$ abhängigen Funktion $\sigma(E_1, E_2)$ registriert werden. Die in Abb. 6b gezeigte Tabelle ist eine tabellarische Darstellung der Projektionsdaten $\underline{p}(\underline{y})$ gemäß dieser Erfindung.

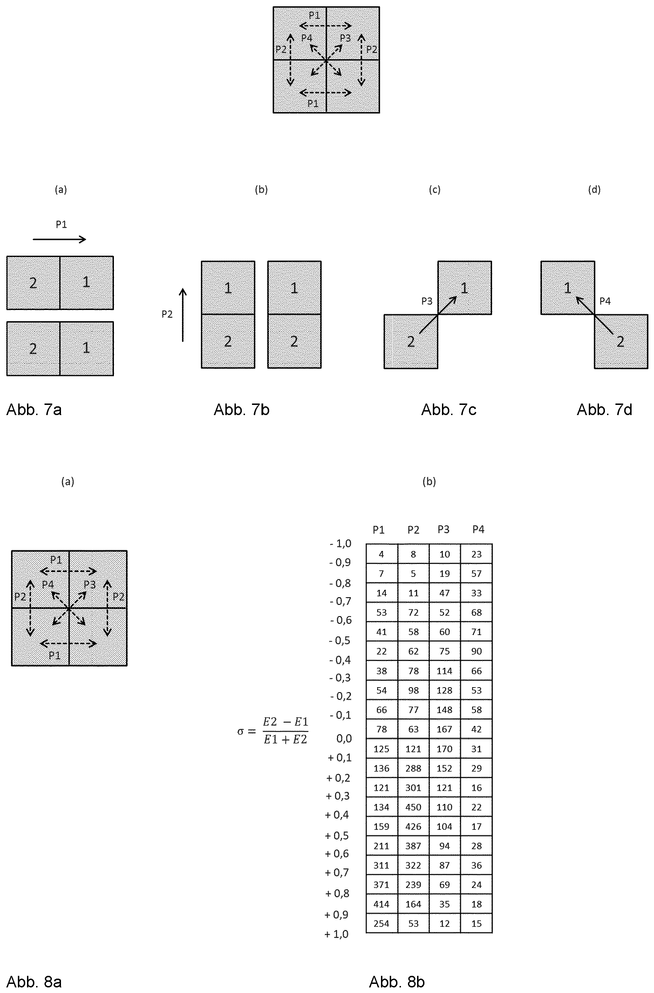

Abb. 7 illustriert schematisch die Auswahl und Kennzeichnung von Detektorpaaren am Beispiel eines Detektorsystems aus vier Detektoren mittlerer bis hoher Ordnungszahl. Das in Abb. 7 dargestellte Detektorsystem besitzt sechs bidirektionale Detektorpaare. Für Ausführungen der Erfindung im Fernfeld genügt es, vier Richtungen P1, P2, P3 und P4 auszuwählen. In der horizontalen und in der vertikalen Richtung gibt es jeweils zwei Detektorpaare, die die gleiche Richtung haben. Im Fernfeld können beide horizontale Detektorpaare gemeinsam unter der Richtung P1 erfasst werden, wie auch beide vertikale Detektorpaare gemeinsam unter der Richtung P2 erfasst werden können. Abb. 7a, 7b, 7c und 7d zeigen Beispiele für die Kennzeichnung der Detektoren einer ersten Richtung P1 (Abb. 7a), einer zweiten Richtung P2 (Abb. 7b), einer dritten Richtung P3 (Abb. 7c) und einer vierten Richtung P4 (Abb. 7d) mit den Nummer 1 bzw. 2.

Abb. 8 illustriert schematisch die Datenerfassung am Beispiel des Detektorsystems aus Abb. 7. Abb. 8a zeigt das Detektorsystem mit vier Detektorpaaren P1, P2, P3 und P4. Abb. 8b zeigt die Datenerfassung für das Detektorsystem in Abb. 8a. Für jede der vier Richtungen P1, P2, P3 und P4 werden die Anzahlen an Koinzidenzereignissen ermittelt, die bei einem bestimmten Wert einer von beiden Energiewerten $E_1$ und $E_2$ abhängigen Funktion $\sigma(E_1,E_2)$ registriert werden. Die in Abb. 8b gezeigte Tabelle ist eine tabellarische Darstellung der Projektionsdaten $\underline{p}(\underline{y})$ gemäß dieser Erfindung.

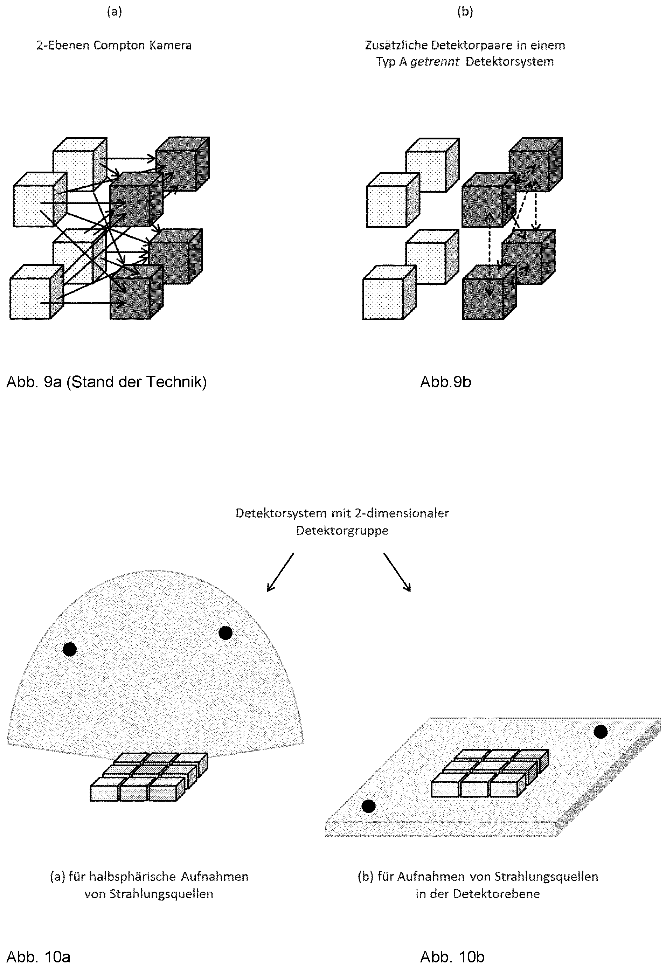

Abb. 9 illustriert schematisch die Erweiterung einer 2-Ebenen Compton Kamera auf ein erfindungsgemäßes bildgebendes Detektorsystem vom Typ A *getrennt*. Die in Abb. 9 (a) gezeigte 2-Ebenen Compton Kamera mit 8 Detektoren besitzt 16 unidirektionale Detektorpaare. Gemäß der Erfindung können die vier Detektoren der hinteren Detektorebene zu 6 bidirektionalen Detektorpaaren kombiniert werden, wie Abb. 9 (b) verdeutlicht. In einem Detektorsystem vom Typ A *getrennt* werden die Daten von 16 unidirektionalen und 6 bidirektionalen Detektorpaaren gemeinsam erfasst und verarbeitet. Die Bildrekonstruktionsverfahren der 2-Ebenen Compton Kamera können entsprechend erweitert werden, um die zusätzlichen Datensätze der hinteren Typ B Detektorebene in die Bildrekonstruktion zu integrieren. Durch die Umstellung erhöht sich die Anzahl der Detektorpaare von 16 auf 22, wodurch die Effizienz und die Bildqualität der 2-Ebenen Compton Kamera verbessert werden.

Abb. 10 zeigt schematisch zwei Nutzungsvarianten für Detektorsysteme mit einer 2-dimensionalen Anordnung der Detektoren. In Abb. 10 (a) ist das 2-dimensionale Detektorsystem als halbsphärisches Detektorsystem dargestellt, in Abb. 10 (b) als 360° Panoramasystem. Das Panoramasystem in Abb. 10 (b) dient zur Messung von 2-dimensionalen Richtungsverteilungen in der Detektorebene. Der Perspektivwechsel kann durch Umstellung des Bildrekonstruktionsverfahrens bewirkt werden.

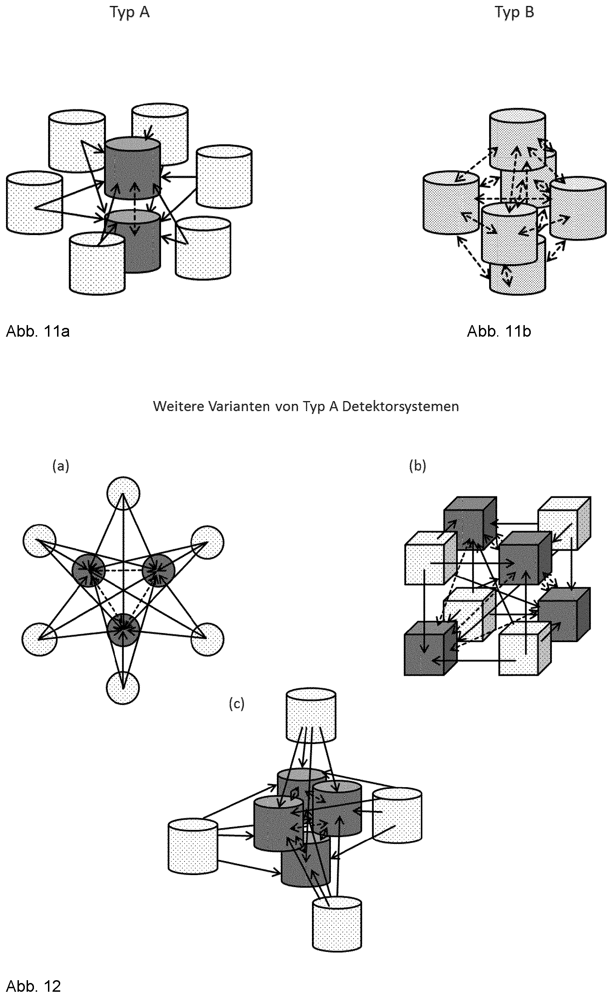

Abb. 11 zeigt eine schematische Darstellung von zwei vollsphärischen Detektorsystemen.

Das in Abb. 11 (a) gezeigte vollsphärische Detektorsystem vom Typ A hat die Gestalt eines Hexagons aus sechs äußeren Detektoren, die um zwei innere Detektoren gruppiert sind. Das Detektorsystem besitzt zwölf unidirektionale Detektorpaare aus je einem inneren und einem äußeren Detektor. Die zwei inneren Detektoren bilden zusätzlich ein bidirektionales Detektorpaar. Dieses dreizehnte Detektorpaar aus den beiden inneren Detektoren liefert zusätzliche Informationen über den Höhenwinkel der Strahlungsquellen.

Abb. 11 (b) zeigt eine schematische Darstellung eines vollsphärischen Detektorsystems vom Typ B. Die als Oktaeder angeordneten sechs Detektoren können zu 15 bidirektionalen Detektorpaaren kombiniert werden.

Abb. 12 zeigt weitere Detektorvarianten vom Typ A.

Das in Abb. 12 (a) dargestellte 2-dimensionale Detektorsystem besteht aus drei inneren Detektoren hoher Ordnungszahl und sechs äußeren Detektoren niedriger Ordnungszahl. Sie bilden ein Dreieck in einem Hexagon. Es ergeben sich 18 unidirektionale und 3 bidirektionale Detektorpaare (insgesamt 21 Detektorpaare).

Abb. 12 (b) zeigt ein würfelförmiges Detektorsystem. Vier Würfelecken sind mit Detektoren hoher Ordnungszahl besetzt, sie bilden untereinander einen Tetraeder. Der Tetraeder aus den vier Detektoren hoher Ordnungszahl liefert 6 bidirektionale Detektorpaare. Zusätzlich wird einfallende Strahlung auch von den vier Detektoren niedriger Ordnungszahl auf je vier Detektoren hoher Ordnungszahl gestreut, wodurch 16 weitere unidirektionale Detektorpaare hinzukommen. Insgesamt stehen 22 Detektorpaare zur Verfügung.

Das Detektorsystem in Abb. 12 (c) besteht aus einem kleinen Tetraeder, der von einem größeren Tetraeder umgeben ist. Der innere Tetraeder enthält vier Detektoren hoher Ordnungszahl, der äußere Tetraeder vier Detektoren niedriger Ordnungszahl. Die vier Detektoren hoher Ordnungszahl sitzen jeweils an den Flächenmittelpunkten der vier Seitenflächen des äußeren Tetraeders. Der kleine Tetraeder aus den Detektoren hoher Ordnungszahl hat ein Drittel der Seitenlänge des einhüllenden äußeren Tetraeders. Der "Tetraeder im Tetraeder" verfügt über 16 unidirektionale und 6 bidirektionale Detektorpaare (insgesamt 22 Detektorpaare).

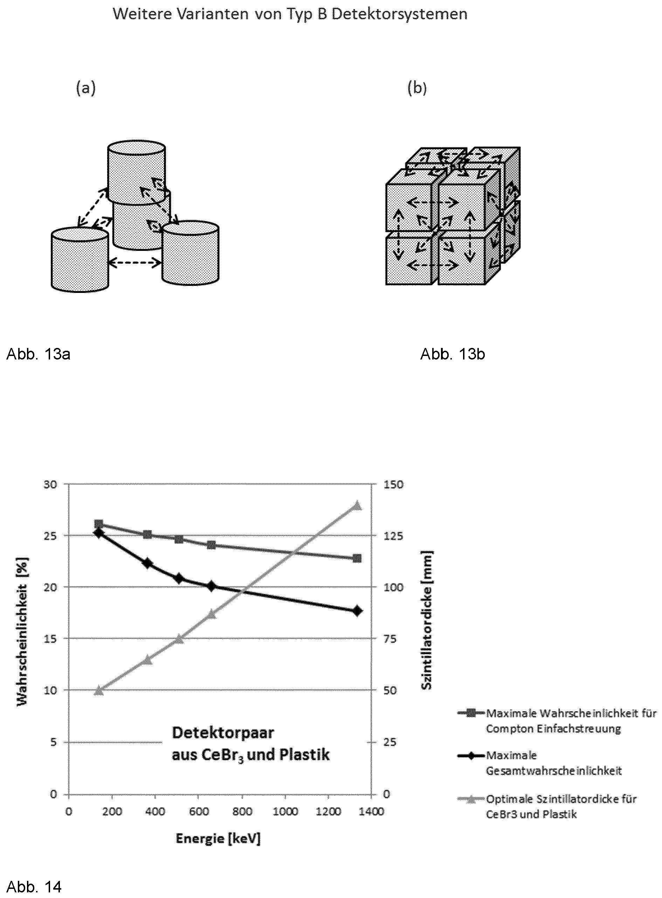

Abb. 13 zeigt weitere Varianten für Detektorsysteme vom Typ B. In Abb. 13 (a) ist eine Anordnung aus vier Detektoren in Form eines Tetraeders mit sechs bidirektionalen Detektorpaaren dargestellt. Der Detektor-Würfel in Abb. 13 (b) besteht aus acht Detektoren mit insgesamt 28 bidirektionalen Detektorpaaren.

Abb. 14 zeigt Simulationsergebnisse für unidirektionale Detektorpaare mit einem Cerbromid- und einem Plastik-Detektor. Die Grafik veranschaulicht den Einfluss der Szintillatordicke auf die Wechselwirkungswahrscheinlichkeiten für Compton Einfachstreuung $P_{CSS}$ in Plastikszintillatoren und den photoelektrischen Effekt $P_{PE}$ in Cerbromidkristallen. Die Kurven zeigen die maximale Wahrscheinlichkeit $P_{CSS}^{max} = \mathrm{Max}(P_{CSS})$ und die maximale Gesamt-wahrscheinlichkeit $P_{ges}^{max} = \mathrm{Max}(P_{CSS} \cdot P_{PE})$ als Funktion der Gammaenergie unter der Annahme, dass beide Szintillatoren die gleichen Abmessungen besitzen. Im Energiebereich von 140 keV bis 1400 keV liegt $P_{CSS}^{max}$ immer oberhalb von 20%, vorausgesetzt, dass die Szintillatordicke optimal gewählt wird. Außerdem ist dargestellt, bei welcher Szintillatordicke die maximale Gesamtwahrscheinlichkeit $P_{ges}^{max}$ erzielt wird.

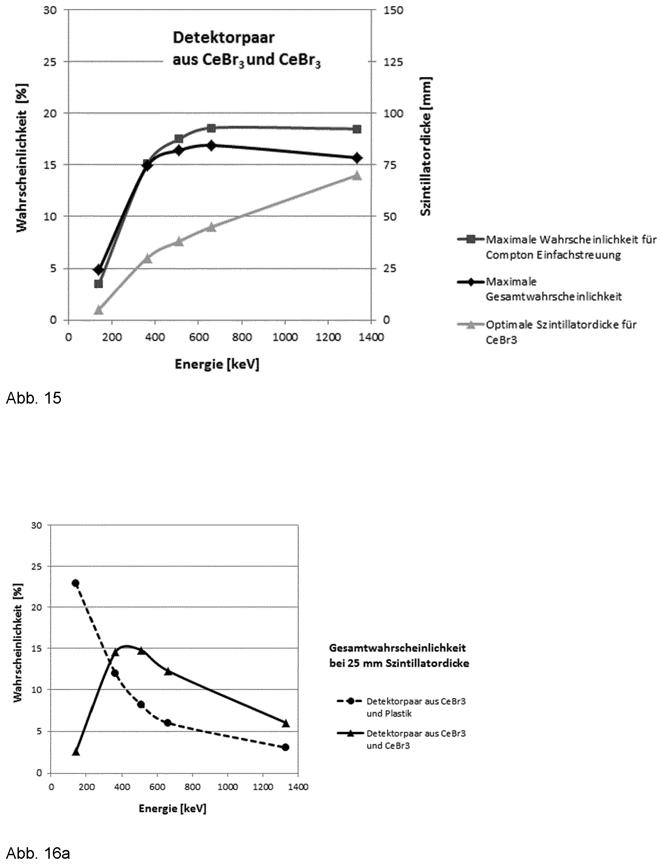

Abb. 15 zeigt Simulationsergebnisse für bidirektionale Detektorpaare aus zwei Cerbromid-Detektoren. Es ist dargestellt, welche Wechselwirkungswahrscheinlichkeiten für Compton Einfachstreuung $P_{CSS}^{max} = \mathrm{Max}(P_{CSS})$ und Gesamtwahrscheinlichkeiten $P_{ges}^{max} = \mathrm{Max}(2 \cdot P_{CSS} \cdot P_{PE})$ unter optimalen Voraussetzungen erzielt werden können. Unterhalb von 300 keV fällt $P_{CSS}^{max}$ schnell ab. Im Energiebereich von 662 bis 1332 keV ist $P_{CSS}^{max}$ hingegen nahezu konstant bei 18,5%. Die Kurve zur optimalen Szintillatordicke zeigt die Dicke, bei der - in Abhängigkeit von der Energie der einfallenden Strahlung - die maximale Gesamtwahrscheinlichkeit $P_{ges}^{max}$ erzielt wird.

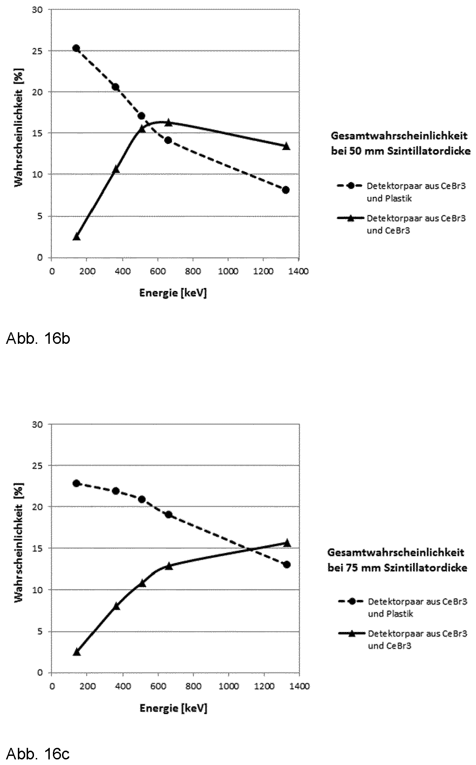

Abb. 16 vergleicht die Simulationsergebnisse für Cerbromid-Plastik und Cerbromid-Cerbromid Detektorpaare. Es sind jeweils die Gesamtwahrscheinlichkeiten bei Szintillatordicken von (a) 25 mm, (b) 50 mm und (c) 75 mm dargestellt. Für Cerbromid-Plastik Detektorpaare wird die Gesamtwahrscheinlichkeit mit $P_{ges} = P_{CSS} \cdot P_{PE}$ berechnet, für Cerbromid-Cerbromid Detektorpaare mit $P_{ges} = 2 \cdot P_{CSS} \cdot P_{PE}$.

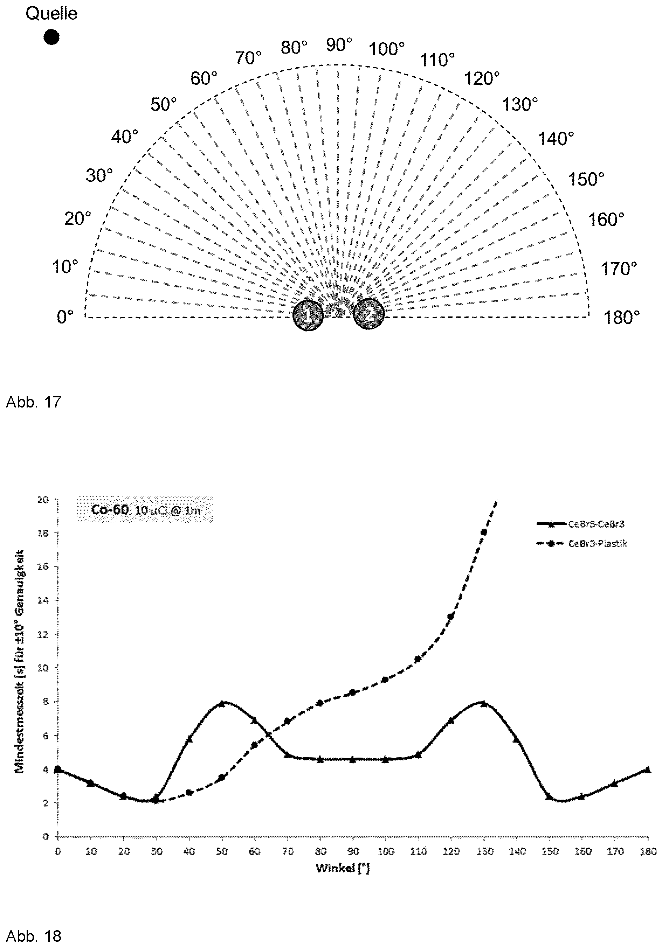

Abb. 17 zeigt eine schematische Darstellung für einen Messaufbau mit einem 1-dimensionalen Detektorsystem aus zwei Strahlungsdetektoren. Der Messaufbau kann als unidirektionales oder als bidirektionales Detektorsystem realisiert werden. Der Sichtbereich des Detektorsystems ist auf einen Halbkreis beschränkt.

Abb. 18 zeigt Messergebnisse für die in Abb. 17 gezeigte Zwei-Detektor-Anordnung zur Richtungsmessung einer Co-60 Quelle. Dargestellt ist die Mindestmesszeit für eine Genauigkeit des Winkels von ± 10° als Funktion des Einfallswinkels, gemessen in zwei Detektorvarianten mit einem Cerbromid-Cerbromid und einem Cerbromid-Plastik Detektorpaar. Die Ortsdosisleistung der Co-60 Quelle (10 μCi) am Messpunkt betrug 0,11 μSv/h.

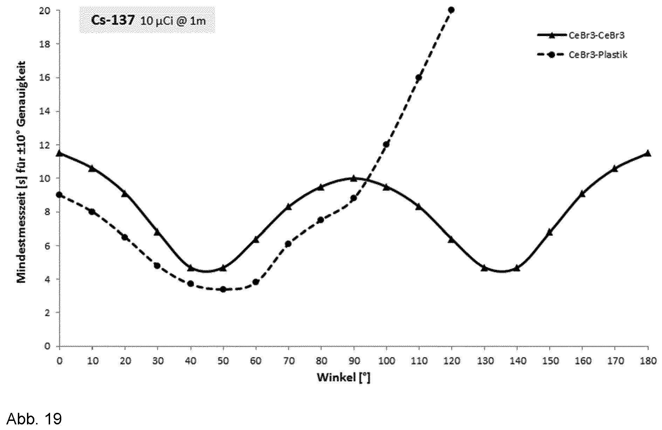

Abb. 19 zeigt Messergebnisse für die in Abb. 17 gezeigte Zwei-Detektor-Anordnung zur Richtungsmessung einer Cs-137 Quelle. Dargestellt ist die Mindestmesszeit für eine Genauigkeit des Winkels von ± 10° als Funktion des Einfallswinkels, gemessen in zwei Detektorvarianten mit einem Cerbromid-Cerbromid und einem Cerbromid-Plastik Detektorpaar. Die Ortsdosisleistung der Cs-137 Quelle (10 μCi) am Messpunkt betrug 0,03 μSv/h.

Im Folgenden sollen verschiedene bildgebende Detektorsysteme in konkreten Ausführungsformen im Detail behandelt werden. Beispiele von diesen werden in den beiliegenden Zeichnungen erläutert. Die hier vorgestellten Detektorsysteme wurden für die Richtungsmessung von Strahlungsquellen im Fernfeld entwickelt. Die Detektorsysteme verwenden Strahlungsdetektoren ohne Ortsinformation über den Wechselwirkungspunkt. Die Bildrekonstruktionsverfahren sind für die 2-dimensionale und die 3-dimensionale Richtungsmessung bei stationären bzw. quasistationären Messbedingungen geeignet.

Es sollen zunächst verschiedene Ausführungen für 3-dimensionale Detektortopologien vorgestellt werden. Die Detektoren eines Detektorsystems vom Typ B können z.B. in den Eckpunkten eines Polyeders angeordnet sein (Abb. 11b und 13). In den meisten Fällen wird der Polyeder vorzugsweise konvex sein.

Bei einem Detektorsystem vom Typ A *gemischt* kann die Detektorhülle ebenfalls ein Polyeder bilden. Detektoren mit niedriger und hoher Ordnungszahl sind heterogen über den Polyeder verteilt.

Ausgewählte Ecken eines Typ A *gemischt* Systems in Gestalt eines Polyeders können derart mit Detektoren hoher Ordnungszahl besetzt werden, dass eingeschriebene Polyeder entstehen. Beispielsweise zeigt Abb. 12 (b) eine würfelförmige Detektoranordnung vom Typ A *gemischt.* Vier Würfelecken sind mit Detektoren hoher Ordnungszahl besetzt. Sie bilden untereinander ein Typ B Detektorsystem in Gestalt eines Tetraeders.

Detektorsysteme vom Typ A *getrennt* können als Polygone oder Polyeder von Detektoren niedriger Ordnungszahl konstruiert werden. In ihrem Inneren befindet sich eine Gruppe von Detektoren hoher Ordnungszahl. Das in Abb. 11 (a) gezeigte Detektorsystem enthält zwei übereinander angeordnete innere Detektoren hoher Ordnungszahl um die herum sechs Ringdetektoren gruppiert sind. Es ist auch möglich, dass der innere Kern von Detektoren hoher Ordnungszahl einen kleinen Polyeder innerhalb eines äußeren Polyeders aus Detektoren niedriger Ordnungszahl bildet (Abb. 12 a und c).

Ein für erfindungsgemäße bildgebende Detektorsysteme geeignetes Detektormaterial ist das Szintillationsmaterial Cerbromid. Mit einer effektiven Ordnungszahl $Z_{eff}$ von 45,9 ist es sowohl als absorbierendes Detektormaterial in unidirektionalen Paaren als auch als streuendes und absorbierendes Material in bidirektionalen Paaren gut geeignet. Cerbromid-Kristalle haben eine Dichte von 5,1 g/cm$^3$. Typische Werte für die Energieauflösung liegen im Bereich von 3,8% bis 4,2% bei 662 keV. Mit einer Zerfallszeit (1/e) von ca. 20 ns lassen sich Koinzidenzen mit einer Zeitauflösung von wenigen Nanosekunden feststellen.

Im Folgenden sollen zwei Ausführungsformen für Detektorpaare anhand spezifischer Materialkombinationen vorgestellt werden. Jedes hier betrachtete Detektorpaar enthält jeweils einen Cerbromidkristall. Wird der Cerbromidkristall mit einem Plastikszintillator kombiniert, entsteht ein unidirektionales Detektorpaar. Durch Paarbildung von je zwei Cerbromidkristallen entstehen bidirektionale Detektorpaare.

Bei der Dimensionierung der Detektoren ist auf die Wechselwirkungswahrscheinlichkeiten der Detektormaterialien in dem Energiebereich zu achten, in dem das Gerät genutzt werden soll. Für die Gammaenergien von Cs-137 und Co-60 sind die entsprechenden Wahrscheinlichkeiten für Cerbromidkristalle und Plastikszintillatoren in Tab. 2 und 3 zusammengestellt. Es wurden Detektorebenen mit Dicken von 1", 2" oder 3" und unendlicher lateraler Ausdehnung betrachtet. Die Angaben zur Einfachstreuung beziehen sich auf Ereignisse, in denen die Gammastrahlung einmal gestreut wird, bevor sie den Szintillator durch die Vorder- oder Rückseite verlässt.

| Cerbromid-Plastik Detektorpaar (je eine Ebene) | | | |
|---|---|---|---|
| Nuklid | Wahrscheinlichkeit $P_{CSS}$ für Einfachstreuung (unter Ausschluss von Mehrfachstreuungen) | Wahrscheinlichkeit $P_{PE}$ für photoelektrische Absorption | Gesamt-wahrscheinlichkeit $P_{ges} = P_{CSS} \cdot P_{PE}$ |
| | 1" Plastik | 1" CeBr$_3$ | |
| Cs-137 | 17% | 34% | 6% |
| Co-60 | 14% | 18% | 3% |
| | 2" Plastik | 2" CeBr$_3$ | |
| Cs-137 | 23% | 62% | 14% |
| Co-60 | 20% | 42% | 8% |
| | 3" Plastik | 3" CeBr$_3$ | |
| Cs-137 | 24% | 79% | 19% |
| Co-60 | 22% | 60% | 13% |

Tab. 2 Wechselwirkungswahrscheinlichkeiten für Einfachstreuung in einer Detektorebene aus einem Plastikszintillator und für photoelektrische Absorption in einer Detektorebene aus kristallinem CeBr$_3$ in Abhängigkeit von der Nuklidenergie und der Szintillatordicke.

| Cerbromid-Cerbromid Detektorpaar (je eine Ebene) | | | |
|---|---|---|---|
| Nuklid | Wahrscheinlichkeit $P_{CSS}$ für Einfachstreuung (unter Ausschluss von Mehrfachstreuungen) | Wahrscheinlichkeit $P_{PE}$ für photoelektrische Absorption | Gesamt-wahrscheinlichkeit $P_{ges} = 2 \cdot P_{CSS} \cdot P_{PE}$ |
| | 1" CeBr$_3$ | 1" CeBr$_3$ | |
| Cs-137 | 18% | 34% | 12% |
| Co-60 | 18% | 18% | 6% |
| | 2" CeBr$_3$ | 2" CeBr$_3$ | |
| Cs-137 | 13% | 62% | 16% |
| Co-60 | 16% | 42% | 13% |
| | 3" CeBr$_3$ | 3" CeBr$_3$ | |
| Cs-137 | 8% | 79% | 13% |
| Co-60 | 13% | 60% | 16% |

Tab. 3 Wechselwirkungswahrscheinlichkeiten für Einfachstreuung und photoelektrische Absorption in je einer Detektorebene aus kristallinem CeBr$_3$ in Abhängigkeit von der Nuklidenergie und der Szintillatordicke.

Für Cerbromid-Cerbromid Detektorpaare bei einer Gammaenergie von 662 keV liegt die optimale Szintillatordicke für Einfachstreuung im Bereich von 20 bis 30 mm, bei 1332 keV wird das Optimum zwischen 30 bis 40 mm erreicht. Bei größeren Kristallen nehmen die Mehrfachstreuungen zu, die keine verwertbaren Richtungsinformationen beisteuern. Die besten Werte für die Gesamtwahrscheinlichkeit $P_{ges}^{max}$ im Energiebereich von Cs-137 und Co-60 werden bei 2" bis 3" Szintillatordicke erzielt.

Für Energien unterhalb von 300 keV ist die Cerbromid-Plastik Kombination - unabhängig von der Dicke - der Cerbromid-Cerbromid Kombination immer überlegen.

Oberhalb von 300 keV ist die Situation etwas komplexer. Abb. 16 (a) verdeutlicht, dass bei einer Szintillatordicke von 1" die Gesamtwahrscheinlichkeit $P_{ges}$ für Cerbromid-Cerbromid Paare ab 350 keV über der von Cerbromid-Plastik Paaren liegt. Ein ähnlicher Trend ist auch bei 2" Szintillatordicke zu beobachten (Abb. 16 b). Bei einer Szintillatordicke von 1" bis 2" lassen sich in einem weiten Energiebereich mit Cerbromid-Cerbromid Paaren höhere Gesamtwahrscheinlichkeiten erzielen, als mit Paaren aus Cerbromid und Plastik. Detektorsysteme, welche ausschließlich aus Cerbromid-Detektoren bestehen, sind daher für kleine kompakte Handgeräte prädestiniert.

Für eine gute Gesamtwahrscheinlichkeit im Energiebereich von 100 keV bis 1500 keV ist es von Vorteil, die jeweiligen Stärken der uni- und bidirektionalen Detektion miteinander zu kombinieren. Dies lässt sich am besten mit einem Typ A System aus Cerbromid- und Plastikdetektoren bewerkstelligen, das einen hohen Anteil an bidirektionalen Detektorpaaren besitzt. Für eine optimale Auslegung auf den Energiebereich von 100 keV bis 1500 keV sind Cerbromid- und Plastikdetektoren der Größe von 2" bis 3" geeignet.

Nachdem verschiedene Varianten für den räumlichen Detektoraufbau und die Detektormaterialien behandelt wurden, werden nachfolgend verschiedene Messungen vorgestellt, die mit bestimmten Ausführungen des erfindungsgemäßen bildgebenden Detektorsystems durchgeführt wurden.

In seiner einfachsten Ausführung besteht das erfindungsgemäße bildgebende Detektorsystem aus zwei Strahlungsdetektoren. Ein solches Detektorsystem wurde mit zwei 3"x3" Cerbromid-Szintillationsdetektoren im Abstand von 18 cm (gemessen von Mittelpunkt zu Mittelpunkt) aufgebaut (Abb. 17). Des Weiteren wurde ein zweites Detektorsystem verwendet, das einen 3"x3" Cerbromid-Detektor und einen 3"x3" Plastik (EJ-200)-Detektor enthält. Das zweite Detektorsystem diente als Vergleichsgrundlage, um das bidirektionale Cerbromid-Cerbromid Detektorpaar mit einem unidirektionalen Cerbromid-Plastik Detektorpaar vergleichen zu können.

Alle Szintillatoren waren zylinderförmig und hatten eine Größe von je 3"x3". Jeder Szintillationsdetektor war mit einem Photomultiplier und einer Hochspannungsversorgung ausgestattet. Cerbromid- und Plastikszintillationsdetektoren liefern kurze schnelle Pulse mit großen Amplituden, die ohne Vorverstärker direkt an einen Digitizer angeschlossen werden können. Das Digitizer-Modul enthielt je einen A/D-Wandler pro Detektor mit nachfolgendem FPGA zur Digitalisierung der Signale, sowie einen Readout-Controller FPGA zur schnellen Auswertung. Der Digitizer verfügte über eine ausreichend hohe Sampling Rate von 500 MS/s, so dass der Signalverlauf aus den Cerbromid- und Plastikdetektoren digital integriert werden konnte. Die aufbereiteten Daten wurden per USB von einem PC ausgelesen, mit einer Messtechnik-Software weiterverarbeitet und für den Nutzer dargestellt.

Der Sichtbereich der Zwei-Detektor-Anordnung ist auf einen Halbkreis beschränkt. Man beachte, dass mit einem solchen System nicht feststellbar ist, auf welcher Seite der Verbindungsachse eine Strahlungsquelle liegt.

Das Datenakquisitionssystem wurde entsprechend dem Schema von Abb. 3b eingerichtet. Die Projektionsdaten $\underline{p}(\underline{y})$ wurden als Histogramm mit dem Funktionswert $\sigma(E_1, E_2)$ von Gl. (4) erfasst. Die Systemmatrizen $\underline{H}$ wurden für beide Detektorsysteme auf experimentelle Weise bestimmt, indem eine Strahlungsquelle unter verschiedenen Einfallswinkeln relativ zur Detektorpaarachse aufgestellt wurde. Es wurden Systemmatrizen für je zwei Radionuklide angelegt (Co-60 und Cs-137). Die Bildrekonstruktion erfolgte mit dem Maximum Likelihood Expectation Maximization Verfahren (MLEM). Das Ergebnis der Bildrekonstruktion war eine Richtungsverteilung $\underline{f}(\underline{x})$ über den Azimutwinkel $\underline{x}$ im Winkelbereich von 0° bis 180°.

Die Messungen wurden mit einer Co-60 (10 µCi) und einer Cs-137 Quelle (10 µCi) in je einem Meter Abstand durchgeführt. Die Messungen liefen jeweils über eine Minute. Zu jedem Zeitpunkt $t$ wurde ein Schätzwert für die Richtung der Quelle am Maximum der MLEM-Funktion $f(\underline{x})$ berechnet. Zur Erhebung der Daten wurden bei jedem Einfallswinkel der Strahlungsquelle insgesamt 20 Messungen durchgeführt. Für jeden Einfallswinkel von 0° bis 180° wurde die Mindestmesszeit $t_{90}$ ermittelt, um den Einfallswinkel mit einer Genauigkeit von $\pm 10°$ bei 90% Konfidenzlevel zu bestimmen. $t_{90}$ ist als diejenige Zeit definiert, ab der mindestens 18 der insgesamt 20 Messungen richtig sind, d.h. in einem $\pm 10°$ Intervall um den wahren Wert liegen. Abb. 18 und 19 zeigen die $t_{90}$ Messzeiten für das Cerbromid-Cerbromid Detektorpaar im Vergleich mit dem Cerbromid-Plastik Detektorpaar über den Winkelbereich von 0° bis 180°.

Die bidirektionale Messung mit dem Cerbromid-Cerbromid Detektorpaar zeichnet sich durch ihren großen Sichtbereich, der den gesamten Winkelbereich von 0° bis 180° abdeckt, aus. Für Co-60 lagen alle $t_{90}$ Messzeiten des Cerbromid-Cerbromid Detektorpaars unter 8 s und für Cs-137 unter 12 s.

Hingegen ist der Sichtbereich des unidirektionalen Cerbromid-Plastik Detektorpaars effektiv auf ca. 100° beschränkt. Auf der Seite des Plastik-Detektors gelingt die Richtungsmessung schnell und zuverlässig, auf der Seite des Cerbromid-Detektors hingegen ist die Richtungsmessung nur sehr spät bzw. gar nicht möglich.

Innerhalb des Sichtbereichs, der bei dem Cerbromid-Plastik Detektorpaar auf ca. 100° beschränkt ist, sind die $t_{90}$ Messzeiten beider Detektorpaare von ähnlicher Größenordnung. Es wurden nur geringfügige Unterschiede zwischen dem bidirektionalen Cerbromid-Cerbromid und dem unidirektionalen Cerbromid-Plastik Detektorpaar festgestellt.

Die Messungen zeigen, dass bidirektionale Cerbromid-Cerbromid Detektorpaare sehr gut zur Richtungsmessung geeignet sind. In der quantitativen Bewertung liefern Cerbromid-Cerbromid Detektorpaare ähnlich gute Ergebnisse wie Cerbromid-Plastik Detektorpaare, ihr Messbereich ist sogar größer, da der gesamte Winkelbereich von 0° bis 180° gemessen werden kann.

Anhand der Messergebnisse können Aussagen für 1-dimensionale Detektorsysteme abgeleitet werden. Es soll hier eine Ausführung für ein Typ A *gemischt* System betrachtet werden, bei dem jeweils ein Cerbromid- und ein Plastik-Detektor hintereinander in einer Reihe angeordnet sind. Die Detektorreihe kann den gesamten Winkelbereich von 0° bis 180° messen, da es in der Reihe unidirektionale Detektorpaare aus je einem Cerbromid- und einem Plastik-Detektor gibt, die den Plastik-Detektor sowohl auf der linken als auch auf der rechten Seite haben. In einer anderen Ausführungsform für ein Typ B System besteht die gesamte Reihe aus Cerbromid-Detektoren. Jedes Paar in der Cerbromid-Detektorreihe erfasst den gesamten Winkelbereich von 0° bis 180°.

Neben den Detektormaterialien Cerbromid und Plastik aus den hier vorgestellten Systemvarianten sind eine Vielzahl weiterer Detektormaterialien bekannt, welche für die Erfindung genutzt werden können. Insbesondere können Halbleiter- und Szintillationsmaterialien auch miteinander kombiniert werden. Ein häufig genutztes Bauprinzip in Detektorsystemen vom Typ A ist die Kombination eines Silizium Pad- oder Streifen-Detektors mit einem Szintillationsdetektor wie NaI, CsI, $CeBr_3$ oder $LaBr_3$.

Auf dem Gebiet der Detektorkonstruktion sind vielfältige Bauweisen für den Bau von Strahlungsdetektoren bekannt. Zu jeder Detektorbauweise gibt es wiederum auf die jeweilige Betriebsweise abgestimmte Elektronikkomponenten. Experten auf diesem Fachgebiet sind ohne Schwierigkeiten in der Lage, die experimentellen Aufbauten auf bestimmte Detektionsanforderungen hin zu optimieren.

Für die Bildrekonstruktion stehen eine Vielzahl von Verfahren zur Verfügung. Im Sinne der Erfindung sind grundsätzlich alle statistischen Bildrekonstruktionsverfahren der Emissionstomographie für das erfindungsgemäße bildgebende Detektorsystem geeignet. Die zuvor beschriebenen Ausführungsformen verwenden das Maximum Likelihood Expectation Maximization Verfahren (MLEM), welches zur Gruppe der EM Bildrekonstruktionsverfahren gehört. Andere bekannte Verfahren aus der Gruppe der EM Verfahren sind z.B.

- Ordered Subset Expectation Maximization (OSEM)
- List Mode - Maximum Likelihood Expectation Maximization (LM-MLEM)
- List Mode - Ordered Subset Expectation Maximization (LM-OSEM) Algorithmus
- EM Verfahren mit Penalty-Funktionen, z.B. für dünnbesetzte (sparse) Lösungen
- Generalized Expectation Maximization (GEM)
- Space-Alternating Generalized EM (SAGE)

Auf dem Gebiet der Emissionstomographie sind darüber hinaus weitere statistische Verfahren bekannt, die für das erfindungsgemäße bildgebende Detektorsystem geeignete Verfahrensausführungen darstellen. Zu ihnen zählen:

- Algebraische Rekonstruktionstechnik (ART)
- Maximum A Posterior Algorithmus (MAP)
- Maximum Entropy Algorithmus (ME)
- Origin Ensemble Algorithmus (OE)

Das erfindungsgemäße bildgebende Detektorsystem mit dem hier beschriebenen Verfahren erfüllt die Anforderungen, die im ABC- und Strahlenschutz an die Richtungsmessung, Ortung und Kartierung von Strahlungsquellen gestellt werden. Die Erfindung bereichert das Gebiet der richtungsauflösenden Strahlungsdetektion um vielfältige Aspekte, u.a. durch folgende Beiträge:

• Sichtbereich über den gesamten $4\pi$ Raumwinkel

• schnelle Richtungsbestimmung

• typische Winkelauflösung bei Dosisleistung der Quelle von 0,5 $\mu$Sv/h

| nach 2 s: | besser als 10° |
|---|---|
| nach 3 s: | besser als 5° |

• Erkennung multipler Quellen gleicher und unterschiedlicher Nuklide mit separater Richtungs- und Intensitätsmessung

• kompakte Geräteformen mit wenigen Detektoren:

> $\geq 2$ Detektoren für 2-dimensionale Ortung in der Ebene
> $\geq 3$ Detektoren für 3-dimensionale Ortung im Halbraum
> $\geq 4$ Detektoren für 3-dimensionale Ortung mit $4\pi$ Raumwinkel

• Aufbauten, die nur ein Detektormaterial oder mehrere Materialien in flexibel gestaltbaren geometrischen Anordnungen verwenden
• einfache und schnelle Algorithmen, die Ergebnisse in Echtzeit liefern
• Gerät unterstützt Ortung und Kartierung von Strahlungsquellen
• vielfältige Verwendungsmöglichkeiten: stationär und mobil, mit einem oder mehreren Geräten
• Bewegungserkennung und Tracking von bewegten Strahlungsquellen möglich

Darüber hinaus ist das erfindungsgemäße bildgebende Detektorsystem auch für Anwendungen in der Nuklearmedizin geeignet. Die Erfindung eröffnet vielfältige neue Möglichkeiten in der medizinischen Bildgebung mit Gammastrahlung, u.a. durch folgende Beiträge:

• hocheffiziente SPECT Scanner ohne Kollimatoren auf Basis des Compton-Effekts
• SPECT Scanner für Gammastrahlung im Energiebereich > 200 keV
• Reduktion der Strahlenbelastung für die Patienten
• Aufbauten, die nur ein Detektormaterial oder mehrere Materialien in flexibel gestaltbaren geometrischen Anordnungen verwenden
• Nutzung von PET Scannern als SPECT und PET/SPECT Hybrid-Scanner
• Algorithmen aus der Emissionstomographie verwendbar
• neue Bildgebungstechniken für Radiopharmaka, die Single Photon und Positronen Emitter sind
• kompakte Bauformen für SPECT und PET/SPECT Hybrid-Sonden in der dezentralen Diagnostik

Eine weitere Verwendungsmöglichkeit für das erfindungsgemäße bildgebende Detektorsystem liegt in der Astronomie mit Gammastrahlung. Die Erfindung unterstützt die Konstruktion neuartiger Compton Teleskope als erfindungsgemäße bildgebende Detektorsysteme mit verbesserter Empfindlichkeit bei gleichem Materialeinsatz.

## Patentansprüche

1. Vorrichtung zur Erzeugung eines oder mehrerer Bilder von einer Quellenverteilung eines Gammastrahlungsfeldes im Nah- und Fernfeld umfassend:

   • ein Detektorsystem, das eine Gruppe von mehreren synchronisierten Detektoren zur Erfassung von Strahlung enthält, wobei mindestens ein Detektormaterial eine Ordnungszahl von $Z_{eff} > 30$ aufweist und alle Detektoren die Energien $E$ und die Wechselwirkungspunkte $\underline{d}$ messen, welche in Wechselwirkungen der Strahlung mit den Detektormaterialien auftreten, wobei die Detektormaterialien virtuell oder physisch in eine beliebige Anzahl von $\geq$ 1 Voxel segmentiert sind;
   • eine Systemelektronik, welche Koinzidenzereignisse registriert, wenn in je zwei Detektorvoxeln aus einer Liste von definierten Voxelpaaren gleichzeitig Wechselwirkungen stattfinden; wobei

   a) die Liste von definierten Voxelpaaren alle Paare umfasst, die kombinatorisch aus der Menge aller Detektorvoxel gebildet werden, und die definierten Voxelpaare mindestens ein Detektorvoxel aus einem Material mit einer Ordnungszahl von $Z_{err} > 30$ enthalten;

   • ein Datenakquisitionssystem, das die Messdaten der Koinzidenzereignisse speichert, und

   b) für beide an einem Koinzidenzereignis beteiligte Detektorvoxel eine Rangordnung festgelegt, die einen ersten und einen zweiten Detektorvoxel definiert; wobei in jedem definierten Voxelpaar der Voxel mit der niedrigeren Ordnungszahl die Nummer 1 und derjenige mit der höheren Ordnungszahl die Nummer 2 erhält; sollten beide Detektorvoxel eines Paares die gleiche Ordnungszahl haben, wird die Kennzeichnung in 1 bzw. 2 willkürlich getroffen; wobei die Vorrichtung **dadurch gekennzeichnet ist, dass** das Datenakquisitionssystem
   c) die in Koinzidenzereignissen gemessenen Energien ($E_1$, $E_2$) und die Wechselwirkungspunkte ($\underline{d}_1$, $\underline{d}_2$)

entsprechend ihrer Kennzeichnung 1,2 ordnet und in einer chronologischen Liste mit den Attributen $y = \{\underline{d}_1, E_1, \underline{d}_2, E_2\}$ und der Detektionszeit $t$ speichert; und

• eine Analyseeinheit, die an das Datenakquisitionssystem angeschlossen ist, welche

d) aus den in Schritt c) gespeicherten Messdaten Projektionsdaten p(y) erstellt, die als Funktion der Attribute $\underline{d} = \{\underline{d}_1, E_1, \underline{d}_2, E_2\}$ definiert sind, wobei eine Funktion $\sigma(E_1, E_2) = (E_2 - E_1)/(E_1 + E_2)$ zur Anwendung kommt, die von zwei Energiewerten ($E_1$, $E_2$) abhängig ist; und

e) die Analyseeinheit eine Bildrekonstruktion ausführt, die aus den Projektionsdaten $\underline{p(\underline{y})}$ ein oder mehrere Bilder $\underline{f(\underline{x})}$ der Quellenverteilung des Strahlungsfeldes rekonstruiert.

2. Vorrichtung nach Anspruch 1, **dadurch gekennzeichnet, dass** das Detektorsystem Strahlungsfelder detektiert, die eine diskrete und/oder eine kontinuierliche Verteilung von Strahlung aufweisen, wobei sich das Detektorsystem im geometrischen Nah- und/oder Fernfeld des Strahlungsfeldes befindet;

und/oder dass die Strahlungsquellen neben Gammastrahlung auch Partikelstrahlung aus einer Partikelgruppe von Elektronen, Positronen, Protonen, Ionen und/oder Neutronen emittieren;
und/oder dass die Strahlung aus dem radioaktiven Zerfall eines oder mehrerer Radionuklide stammt;
und/oder dass die Strahlung die prompte Gammastrahlung ist, welche bei der Absorption von Protonen- oder Ionenstrahlung in Targetmaterialien entsteht;
und/oder dass die Strahlung von niedriger Intensität ist.

3. Vorrichtung nach einem der vorhergehenden Ansprüche, **dadurch gekennzeichnet, dass** die Detektoren einen Szintillator mit einem Photodetektor und/oder ein Halbleitermaterial aufweisen;

und/oder dass der Szintillator ein reines oder dotiertes Material der Gruppe aus PVT, Anthracen, Stilben, p-Terphenyl, $CaF_2$, $BaF_2$, NaI, $CeBr_3$, $LaBr_3$, $LaCl_3$, $La(Br_xCl_{1-x})_3$, CsI, $SrI_2$, CLYC, CLBC, CLCB, CLLB, BGO, LSO, LYSO, GAGG, YAP und/oder YAG umfasst;
und/oder dass der Szintillator als monolithischer Block oder als pixeliertes Szintillatormodul vorliegt;
und/oder dass der Photodetektor ein Photomultiplier (PMT), ein ortsauflösender Photomultiplier (PSPMT), ein Silizium Photomultiplier (SiPM), ein ortsauflösender Silizium Photomultiplier (PS-SiPM) und/oder eine Silizium Photodiode ist;
und/oder dass das Halbleitermaterial ein Material aus der Gruppe von Si, Ge, GaAs, CdTe und/oder CdZnTe umfasst;
und/oder dass das Halbleitermaterial eine planare oder koaxiale Geometrie aufweist und/oder mit segmentierten oder unsegmentierten Kontakten vorliegt;
und/oder dass der Szintillator bzw. das Halbleitermaterial virtuell oder physisch in eine beliebige ganzzahlige Anzahl von $\geq 1$ Voxeln unterteilt sind.

4. Vorrichtung nach einem der vorhergehenden Ansprüche, **dadurch gekennzeichnet, dass** die Systemelektronik analoge und/oder digitale Elektronikkomponenten verwendet;

und/oder dass die analogen Elektronikkomponenten eine Kombination verschiedener Module umfassen, zu denen eine Hochspannungsversorgung, ein Vorverstärker, ein Verstärker, ein Pulsformer, ein Ladungsintegrator, ein Pulshöhenanalysator, ein Multikanalanalysator (MCA) und/oder eine Koinzidenzschaltung zählen;
und/oder dass die digitalen Elektronikkomponenten eine Kombination verschiedener Hard- und Softwarekomponenten umfassen, zu denen eine Hochspannungsversorgung, ein A/D-Wandler pro Detektorvoxel, ein Field Programmable Gate Array (FPGA), ein Speichermedium, ein digitaler Signalprozessor und/oder eine Auswertesoftware zählen.

5. Vorrichtung nach einem der vorhergehenden Ansprüche, **dadurch gekennzeichnet, dass** die Wechselwirkungspunkte ($\underline{d}_1$, $\underline{d}_2$) anhand der ortsauflösenden Eigenschaften der Detektoren und/oder anhand der vorliegenden Segmentierung der Detektoren bestimmt werden;
und/oder dass die Wechselwirkungspunkte ($\underline{d}_1$, $\underline{d}_2$) als die räumlichen Mittelpunkte der an einem Koinzidenzereignis beteiligten Detektorvoxel eines ersten und eines zweiten Detektorvoxels definiert sind.

6. Vorrichtung nach einem der vorhergehenden Ansprüche, **dadurch gekennzeichnet, dass** die Analyseeinheit zur Rekonstruktion des Strahlungsfeldes Projektionsdaten $p(y)$ verwendet, die als Funktion der Attribute $\underline{y} = \{\underline{d}_1, E_1, \underline{d}_2,$

$E_2$} definiert sind;

und/oder dass für jedes Element von p eine Anzahl von Koinzidenzereignissen ermittelt wird, die bei einer jeweiligen Kombination eines bestimmten ersten Detektorvoxels mit einem bestimmten zweiten Detektorvoxel bei einem bestimmten Funktionswert $\sigma(E_1,E_2)$ auftritt;

und/oder dass für jedes Element von p eine Anzahl von Koinzidenzereignissen ermittelt wird, die bei einer jeweiligen Kombination eines bestimmten ersten Detektionsorts $\underline{d}_1$ mit einem bestimmten zweiten Detektionsort $d_2$ bei einem bestimmten Funktionswert $\sigma(E_1,E_2)$ auftritt.

7. Vorrichtung nach einem der vorhergehenden Ansprüche, **dadurch gekennzeichnet, dass** eine Selektionsbedingung für Koinzidenzereignisse bezüglich der Energiesumme $E_1 + E_2$ der in beiden Detektorvoxeln eines Paares detektierten Energien angewendet wird;

und/oder dass separate Projektionsdaten $\underline{p}(\underline{y})$ für jedes detektierte Radionuklid angelegt werden;

und/oder die Analyseeinheit für jedes Radionuklid ein separates Bild $\underline{f}(\underline{x})$ berechnet.

8. Vorrichtung nach einem der vorhergehenden Ansprüche, **dadurch gekennzeichnet, dass** die Bilder $\underline{f}(\underline{x})$ eine Aktivitätsdichte, eine Flussdichte bzw. eine Dosisleistungsdichte darstellen;

und/oder die Bilder $\underline{f}(\underline{x})$ tomographische Schnittbilder einer Aktivitätsverteilung von Radionukliden (z.B. auch Radiopharmaka) sind.

9. Vorrichtung nach einem der vorhergehenden Ansprüche, **dadurch gekennzeichnet, dass** alle Detektoren im Wesentlichen eine gleiche Bauart aufweisen oder dass mindestens zwei der Detektoren eine voneinander verschiedene Bauart aufweisen.

10. Vorrichtung nach einem der vorhergehenden Ansprüche, **dadurch gekennzeichnet, dass** das Detektorsystem mindestens vier Detektorvoxel in einer im Wesentlichen 3-dimensionalen Anordnung aufweist und über einen Sichtbereich von einem $4\pi$ Raumwinkel verfügt;

oder dass das Detektorsystem mindestens drei Detektorvoxel in einer im Wesentlichen 2-dimensionalen Anordnung aufweist und über einen halbsphärischen Sichtbereich mit einem $2\pi$ Raumwinkel verfügt;

oder dass das Detektorsystem mindestens drei Detektorvoxel in einer im Wesentlichen 2-dimensionalen Anordnung aufweist und einen 360° Sichtbereich in der Ebene der Strahlungsdetektoren besitzt;

oder dass das Detektorsystem mindestens zwei Detektorvoxel in einer 1-dimensionalen Detektorreihe aufweist und einen 180° Sichtbereich von -90° bis +90° senkrecht zur Detektorreihe besitzt.

11. Vorrichtung nach einem der vorhergehenden Ansprüche, **dadurch gekennzeichnet, dass** das Detektorsystem eine erste Gruppe von Detektoren/Voxeln mit einer Ordnungszahl $Z_{eff} > 30$, die hohe Wahrscheinlichkeiten für photoelektrische Absorption im Energiebereich von 100 keV bis 3 MeV aufweisen, und optional eine zweite Gruppe von Detektoren/Voxeln mit einer Ordnungszahl $Z_{eff} \leq 30$ umfasst, die hohe Wahrscheinlichkeiten für Compton-Streuung im Energiebereich von 100 keV bis 3 MeV aufweisen;

und/oder Daten von allen Detektorpaaren/Voxelpaaren erfasst werden, die kombinatorisch aus der Menge aller Detektoren/Voxel gebildet werden können, wobei solche Detektorpaare/Voxelpaare ausgenommen sind in denen beide Detektoren/Voxel zur zweiten Gruppe mit Ordnungszahl $Z_{eff} \leq 30$ gehören;

und/oder die gemischten Detektorpaare/Voxelpaare, die je einen Detektor/Voxel aus der ersten Gruppe und einen Detektor/Voxel aus der zweiten Gruppe aufweisen, unidirektional sind, wobei die Strahlung vornehmlich in eine Richtung gestreut wird;

und/oder die Detektorpaare/Voxelpaare der ersten Gruppe mit Ordnungszahl $Z_{eff} > 30$, bidirektional sind, wobei die Strahlung in beide Richtungen mit ähnlicher Intensität gestreut wird.

12. Vorrichtung nach einem der vorhergehenden Ansprüche, **dadurch gekennzeichnet, dass** die Gesamtheit der Detektoren/Voxel die das Detektorsystem umfasst eine erste homogene oder heterogene ringförmige, röhrenförmige, zylindrische, kugelförmige und/oder polyedrische Form aufweist;

und/oder dass die Gesamtheit der Detektoren/Voxel die das Detektorsystem umfasst zusätzlich zur ersten Form eine zweite homogene oder heterogene ringförmige, röhrenförmige, zylindrische, kugelförmige und/oder polyedrische Form aufweist, wobei die zweite Form in die erste Form eingeschrieben ist oder wobei die zweite Form in einem inneren Bereich angeordnet ist, den die erste Form als äußere Hülle umschließt;
und/oder wobei die erste und/oder die zweite Form um einen oder mehrere Zentraldetektoren angeordnet ist.

13. Vorrichtung nach einem der vorhergehenden Ansprüche, **dadurch gekennzeichnet, dass** ein Teil des Systems oder das ganze System als Ortungsgerät für Strahlungsquellen, als Compton Kamera, als Compton Teleskop, als SPECT Scanner, als PET/SPECT Hybrid-Scanner, als SPECT Sonde und/oder als PET/SPECT Hybrid-Sonde ausgebildet ist.

14. Verfahren für die Verwendung einer Vorrichtung zur Erzeugung eines oder mehrerer Bilder einer Quellenverteilung eines Gammastrahlungsfeldes nach einem der Ansprüche 1 bis 13, wobei die Detektormaterialien virtuell oder physisch in eine beliebige Anzahl von $\geq 1$ Voxel segmentiert sind, das Verfahren uni- und bidirektionale Compton Streuprozesse nutzt und eine Systemmatrix $\underline{H}$, einen definierten Funktionswert $\sigma(E_1, E_2)$ und eine Liste von definierten Voxelpaaren aufweist und für die Erfassung von Projektionsdaten $\underline{p}(\underline{y})$ der Messwerte und für die Berechnung von Bilddaten $\underline{f}(\underline{x})$ eingerichtet ist, und die folgenden Schritte umfasst:

   • Erstellen einer Liste mit definierten Voxelpaaren, wobei die definierten Voxelpaare alle Paare umfassen, welche kombinatorisch aus der Menge der Detektorvoxel gebildet werden und jedes Paar mindestens einen Detektorvoxel aus einem Material mit einer Ordnungszahl von $Z_{eff} > 30$ enthält;
   • Verschalten aller Detektoren/Voxel in einer Koinzidenzschaltung derart, dass Koinzidenzereignisse in allen definierten Voxelpaaren erfasst werden;
   • Kennzeichnen der beiden Detektorvoxel eines jeden Voxelpaares mit den Nummern 1 bzw. 2, wobei der Detektorvoxel mit der niedrigeren Ordnungszahl die Nummer 1 erhält, und derjenige mit der höheren Ordnungszahl die Nummer 2, wenn beide Detektorvoxel aus dem gleichen Material bestehen wird die Kennzeichnung willkürlich getroffen;
   • Definieren einer Funktion $\sigma(E_1, E_2) = (E_2 - E_1)/(E_1 + E_2)$ welche aus zwei Energiewerten ($E_1, E_2$) berechnet wird;
   • Akquirieren von Messwerten $\underline{y} = \{\underline{d}_1, E_1, \underline{d}_2, E_2\}$ von Koinzidenzereignissen, wenn in je zwei Detektorvoxeln von allen definierten Voxelpaaren gleichzeitig Wechselwirkungen stattfinden, wobei die Messwerte einem Strahlungsnah- oder Fernfeld entstammen und die Messwerte die in den Detektorvoxeln gemessenen Energien ($E_1, E_2$) und die Wechselwirkungspunkte ($\underline{d}_1, \underline{d}_2$) sind,
   • Assoziieren von Koinzidenzereignissen $\underline{y} = \{\underline{d}_1, E_1, \underline{d}_2, E_2\}$ mit einem ersten Detektorvoxel/Detektionsort $\underline{d}_1$ und einem zweiten Detektorvoxel/ Detektionsort $\underline{d}_2$;
   • Berechnen des Funktionswertes $\sigma(E_1, E_2)$ aus zwei Energiewerten ($E_1, E_2$) pro Koinzidenzereignis;
   • Erfassen der Koinzidenzereignisse entsprechend ihres ersten Detektorvoxels $\underline{d}_1$, ihres zweiten Detektorvoxels $\underline{d}_2$ und ihres $\sigma(E_1, E_2)$ Wertes in einem Element der Projektionsdaten $\underline{p}(\underline{y})$, wobei für jedes Radionuklid separate Projektionsdaten $\underline{p}(\underline{y})$ erfasst werden;
   • Berechnen eines oder mehrerer Bilder $\underline{f}(\underline{x})$ aus den Projektionsdaten $p(\underline{y})$ mit einem statistischen Bildrekonstruktionsverfahren der Emissionstomographie unter Nutzung der Systemmatrix $\underline{H}$, wobei für jedes Radionuklid ein separates Bild $\underline{f}(\underline{x})$ berechnet wird; die Bilder $f(\underline{x})$ repräsentieren eine Aktivitätsverteilung in einem Quellvolumen oder eine Flussdichteverteilung über die Einfallsrichtungen.

15. Verfahren gemäß Anspruch 14, wobei das Verfahren zusätzlich in Teilen oder in Gänze die folgenden Schritte umfasst:

   • Kalibrieren der Signale aller Detektorvoxel als absorbierte Strahlungsenergie $E$;
   • Festlegen eines geeigneten Koordinatensystems;
   • Einteilen des Messbereiches für den Funktionswert $\sigma(E_1, E_2)$ in äquidistante Messwertkanäle;
   • Anlegen eines oder mehrerer Projektionsdatensätze $\underline{p}(\underline{y})$, welche die Anzahlen von Koinzidenzereignissen erfassen, die bei einer jeweiligen Kombination von einem bestimmten ersten Detektorvoxel $\underline{d}_1$ mit einem bestimmten zweiten Detektorvoxel $\underline{d}_2$ bei einem bestimmten Funktionswert $\sigma(E_1, E_2)$ gezählt werden, wobei für jedes Radionuklid ein separater Projektionsdatensatz $\underline{p}(\underline{y})$ angelegt wird;
   • Anlegen einer oder mehrerer Systemmatrizen $\underline{H}$ für jedes zu detektierende Radionuklid;
   • Erstellen und Validieren der Systemmatrizen $\underline{H}$ durch Messungen mit dem Detektorsystem oder durch Monte Carlo Simulationen oder mittels eines theoretischen Modells;
   • Übergeben sämtlicher Systemmatrizen $\underline{H}$ an einen Algorithmus der Bildrekonstruktion, welcher die Projektions-daten $\underline{p}(\underline{y})$ verarbeitet und die Bilder $\underline{f}(\underline{x})$ berechnet.

**Claims**

1. A device for generating one or more images of a source distribution of a gamma radiation field in the near and far field, comprising:

   • a detector system containing a group of several synchronized detectors for detecting radiation, wherein at least one detector material has an atomic number of $Z_{eff} > 30$ and all detectors measure the energies $E$ and the interaction points $\underline{d}$ which occur in interactions of the radiation with the detector materials, wherein the detector materials are virtually or physically segmented into an arbitrary number of $\geq 1$ voxel;
   • a system electronics which registers coincidence events when interactions occur simultaneously in two detector voxels from a list of defined voxel pairs; wherein

   a) the list of defined voxel pairs comprises all pairs that are combinatorially formed from the set of all detector voxels, and the defined voxel pairs contain at least one detector voxel made of a material with an atomic number of $Z_{eff} > 30$;

   • a data acquisition system that stores the measurement data of the coincidence events, and

   b) a ranking is determined for both detector voxels involved in a coincidence event, which ranking defines a first and a second detector voxel; wherein in each defined voxel pair the voxel with the lower atomic number is given the number 1 and the voxel with the higher atomic number is given the number 2; if both detector voxels of a pair have the same atomic number, the identification as 1 and 2 is arbitrarily taken; wherein the device is **characterized in that** the data acquisition system
   c) sorts the energies $(E_1,E_2)$ measured in coincidence events and the interaction points $(\underline{d}_1,\underline{d}_2)$ according to their identification 1,2 and stores them in a chronological list with the attributes $\underline{y} = \{\underline{d}_1,E_1,\underline{d}_2,E_2\}$ and the detection time t; and

   • an analysis unit connected to the data acquisition system, which

   d) from the measurement data stored in step c), creates projection data $\underline{p}(\underline{y})$, which are defined as a function of the attributes $\underline{y} = \{\underline{d}_1,E_1,\underline{d}_2,E_2\}$, using a function $\sigma(E_1, E_2) = (E_2\text{-}E_1)/(E_1 + E_2)$ which depends on two energy values $(E_1,E_2)$; and
   e) the analysis unit performs an image reconstruction which reconstructs one or more images $f(\boldsymbol{x})$ of the source distribution of the radiation field from the projection data $\underline{p}(\underline{y})$.

2. The device according to claim 1, **characterized in that** the detector system detects radiation fields having a discrete and/or a continuous distribution of radiation, wherein the detector system is located in the geometric near and/or far field of the radiation field;

   and/or **in that** the radiation sources emit, in addition to gamma radiation, particle radiation from a particle group of electrons, positrons, protons, ions and/or neutrons;
   and/or **in that** the radiation originates from the radioactive decay of one or more radionuclides;
   and/or **in that** the radiation is the prompt gamma radiation generated during the absorption of proton or ion radiation in target materials;
   and/or **in that** the radiation is of low intensity.

3. The device according to any one of the preceding claims, **characterized in that** the detectors comprise a scintillator with a photodetector and/or a semiconductor material;

   and/or **in that** the scintillator comprises a pure or doped material from the group consisting of PVT, anthracene, stilbene, p-terphenyl, $CaF_2$, $BaF_2$, NaI, $CeBr_3$, $LaBr_3$, $LaCl_3$, $La(Br_xCl_{1-x})_3$, CsI, $SrI_2$, CLYC, CLBC, CLCB, CLLB, BGO, LSO, LYSO, GAGG, YAP and/or YAG;
   and/or **in that** the scintillator is in the form of a monolithic block or a pixelated scintillator module;
   and/or **in that** the photodetector is a photomultiplier (PMT), a spatially resolved photomultiplier (PSPMT), a silicon photomultiplier (SiPM), a spatially resolved silicon photomultiplier (PS-SiPM) and/or a silicon photodiode;
   and/or **in that** the semiconductor material comprises a material from the group of Si, Ge, GaAs, CdTe and/or CdZnTe;
   and/or **in that** the semiconductor material has a planar or coaxial geometry and/or is present with segmented or unsegmented contacts;
and/or **in that** the scintillator or semiconductor material is virtually or physically divided into any integer number of $\geq 1$ voxels.

4.  The device according to any one of the preceding claims, **characterized in that** the system electronics use analog and/or digital electronic components;

    and/or **in that** the analog electronic components comprise a combination of different modules including a high voltage supply, a preamplifier, an amplifier, a pulse shaper, a charge integrator, a pulse height analyzer, a multichannel analyzer (MCA) and/or a coincidence circuit;
    and/or **in that** the digital electronic components comprise a combination of different hardware and software components, which comprise a high-voltage supply, an A/D converter per detector voxel, a field programmable gate array (FPGA), a storage medium, a digital signal processor and/or an evaluation software.

5.  The device according to any one of the preceding claims, **characterized in that** the interaction points ($\underline{d}_1$, $\underline{d}_2$) are determined based on the spatially resolving properties of the detectors and/or based on the existing segmentation of the detectors;
    and/or **in that** the interaction points ($\underline{d}_1$, $\underline{d}_2$) are defined as the spatial centers of the detector voxels of a first and a second detector voxel involved in a coincidence event.

6.  The device according to any one of the preceding claims, **characterized in that** the analysis unit uses projection data $p(\underline{y})$ defined as a function of the attributes $\underline{y} = \{\underline{d}_1, E_1, \underline{d}_2, E_2\}$ to reconstruct the radiation field;

    and/or **in that** for each element of p a number of coincidence events is determined which occur in a respective combination of a specific first detector voxel with a specific second detector voxel at a specific function value $\sigma(E_1, E_2)$;
    and/or **in that** for each element of p a number of coincidence events is determined which occur in a respective combination of a specific first detector location $\underline{d}_1$ with a specific second detector location $\underline{d}_2$ at a specific function value $\sigma(E_1, E_2)$.

7.  The device according to any one of the preceding claims, **characterized in that** a selection condition for coincidence events is applied in relation to the energy sum $E_1 + E_2$ of the energies detected in both detector voxels of a pair;

    and/or **in that** separate projection data $\underline{p}(\underline{y})$ are created for each detected radionuclide;
    and/or the analysis unit calculates a separate image $\underline{f}(\underline{x})$ for each radionuclide.

8.  The device according to any one of the preceding claims, **characterized in that** the images $\underline{f}(\underline{x})$ represent an activity density, a flux density and a dose rate density, respectively;
    and/or the images $\underline{f}(\underline{x})$ are tomographic sectional images of an activity distribution of radionuclides (e.g. also radiopharmaceuticals).

9.  The device according to any one of the preceding claims, **characterized in that** all detectors have substantially the same design or that at least two of the detectors have a different design from one another.

10. The device according to any one of the preceding claims, **characterized in that** the detector system has at least four detector voxels in a substantially 3-dimensional arrangement and has a field of view of a $4\pi$ solid angle;

    or **in that** the detector system comprises at least three detector voxels in a substantially 2-dimensional arrangement and has a hemispherical field of view with a $2\pi$ solid angle;
    or **in that** the detector system comprises at least three detector voxels in a substantially 2-dimensional arrangement and has a 360° field of view in the plane of the radiation detectors;
    or **in that** the detector system has at least two detector voxels in a 1-dimensional detector array and has a 180° field of view from -90° to +90° perpendicular to the detector array.

11. The device according to any one of the preceding claims, **characterized in that** the detector system comprises a first group of detectors/voxels with an atomic number $Z_{eff} > 30$, which have high probabilities for photoelectric absorption in the energy range from 100 keV to 3 MeV, and optionally a second group of detectors/voxels with an atomic number $Z_{eff} \leq 30$, which have high probabilities for Compton scattering in the energy range from 100 keV to 3 MeV;

and/or data are acquired from all detector pairs/voxel pairs that can be formed combinatorially from the set of all detectors/voxels, excluding those detector pairs/voxel pairs in which both detectors/voxels belong to the second group with atomic number $Z_{eff} \leq 30$;

and/or the mixed detector pairs/voxel pairs, each comprising one detector/voxel from the first group and one detector/voxel from the second group, are unidirectional, with the radiation being scattered predominantly in one direction;

and/or the detector pairs/voxel pairs of the first group with atomic number $Z_{eff} > 30$ are bidirectional, with the radiation being scattered in both directions with similar intensity.

12. The device according to any one of the preceding claims, **characterized in that** the entirety of the detectors/voxels comprising the detector system has a first homogeneous or heterogeneous annular, tubular, cylindrical, spherical and/or polyhedral shape;

and/or **in that** the entirety of the detectors/voxels comprising the detector system has, in addition to the first shape, a second homogeneous or heterogeneous annular, tubular, cylindrical, spherical and/or polyhedral shape, wherein the second shape is inscribed in the first shape or wherein the second shape is arranged in an inner region which the first shape encloses as an outer shell;

and/or wherein the first and/or second shape are arranged around one or more central detectors.

13. The device according to any one of the preceding claims, **characterized in that** part of the system or the entire system is designed as a locating device for radiation sources, as a Compton camera, as a Compton telescope, as a SPECT scanner, as a PET/SPECT hybrid scanner, as a SPECT probe and/or as a PET/SPECT hybrid probe.

14. A method for using a device for generating one or more images of a source distribution of a gamma radiation field according to any one of claims 1 to 13, wherein the detector materials are virtually or physically segmented into any number of $\geq 1$ voxels, the method uses unidirectional and bidirectional Compton scattering processes and has a system matrix $\underline{H}$, a defined function value $\sigma(E_1,E_2)$ and a list of defined voxel pairs and is configured for acquiring projection data $\underline{p}(\underline{y})$ of the measured values and for calculating image data $\underline{f}(\underline{x})$, and comprises the following steps:

• creating a list of defined voxel pairs, wherein the defined voxel pairs comprise all pairs formed combinatorially from the set of detector voxels, and each pair contains at least one detector voxel made of a material having an atomic number of $Z_{eff} > 30$;

• interconnecting all detectors/voxels in a coincidence circuit such that coincidence events are detected in all defined voxel pairs;

• identifying both detector voxels of each voxel pair with the numbers 1 and 2, respectively, wherein the detector voxel with the lower atomic number receives the number 1 and the detector voxel with the higher atomic number receives the number 2, if both detector voxels are made of the same material, the identification is made arbitrarily;

• defining a function $\sigma(E_1, E_2) = (E_2-E_1) / (E_1+E_2)$ which is calculated from two energy values $(E_1,E_2)$;

• acquiring measured values $\underline{y} = \{\underline{d}_1,E_1,\underline{d}_2,E_2\}$ of coincidence events if interactions take place simultaneously in two respective detector voxels of all defined voxel pairs, wherein the measured values originate from a radiation near field or far field and the measured values are the energies $(E_1,E_2)$ measured in the detector voxels and the interaction points $(\underline{d}_1, \underline{d}_2)$;

• associating coincidence events $\underline{y} = \{\underline{d}_1,E_1,\underline{d}_2,E_2\}$ with a first detector voxel/detection location $\underline{d}_1$ and a second detector voxel/detection location $\underline{d}_2$;

• calculating the functional value $\sigma(E_1, E_2)$ from two energy values $(E_1, E_2)$ per coincidence event;

• detecting the coincidence events corresponding to their first detector voxel $\underline{d}_1$, their second detector voxel $\underline{d}_2$ and their $\sigma(E_1,E_2)$ value in an element of the projection data $\underline{p}(\underline{y})$, wherein separate projection data $\underline{p}(\underline{y})$ are detected for each radionuclide;

• calculating one or more images $\underline{f}(\underline{x})$ from the projection data $\underline{p}(\underline{y})$ using a statistical image reconstruction method of emission tomography using the system matrix $\underline{H}$, wherein a separate image $\underline{f}(\underline{x})$ is calculated for each radionuclide; the images $\underline{f}(\underline{x})$ represent an activity distribution in a source volume or a flux density distribution over the incidence directions.

15. The method according to claim 14, wherein the method additionally comprises, in part or in whole, the following steps:

• calibrating the signals from all detector voxels as absorbed radiation energy E;

• determining a suitable coordinate system;

• dividing the measurement range for the functional value $\sigma(E_1, E_2)$ into equidistant measured value channels;

• creating one or more projection data sets $p(\underline{y})$ which record the numbers of coincidence events counted for a respective combination of a specific first detector voxel $\underline{d}_1$ with a specific second detector voxel $\underline{d}_2$ at a specific function value $\sigma(E_1,E_2)$, wherein a separate projection data set $p(\underline{y})$ is created for each radionuclide;
• creating one or more system matrices $\underline{H}$ for each radionuclide to be detected;
• creating and validating the system matrices $\underline{H}$ by measurements with the detector system or by Monte Carlo simulations or by means of a theoretical model;
• passing all system matrices $\underline{H}$ to an image reconstruction algorithm, which processes the projection data $p(\underline{y})$ and calculates the images $f(\underline{x})$.

**Revendications**

1. Dispositif permettant de générer une ou plusieurs images d'une distribution de source d'un champ de rayonnement gamma en champ proche et lointain, comprenant :

   • un système de détecteur contenant un groupe de plusieurs détecteurs synchronisés pour détecter un rayonnement, dans lequel au moins un matériau détecteur présente un numéro atomique de $Z_{eff} > 30$ et tous les détecteurs mesurent les énergies E et les points d'interaction $\underline{d}$ qui se produisent dans les interactions du rayonnement avec les matériaux détecteurs, dans lequel les matériaux détecteurs sont segmentés virtuellement ou physiquement en un nombre quelconque de $\geq 1$ voxels ;
   • une électronique système qui enregistre les événements de coïncidence lorsque des interactions se produisent simultanément dans deux voxels de détecteur à partir d'une liste de paires de voxels définies ; dans lequel

   a) la liste des paires de voxels définies comprend toutes les paires qui sont formées combinatoirement à partir de l'ensemble de tous les voxels de détecteur, et les paires de voxels définies contiennent au moins un voxel de détecteur constitué d'un matériau avec un numéro atomique de $Z_{eff} > 30$ ;

   • un système d'acquisition de données qui stocke les données de mesure des événements de coïncidence, et

   b) un classement est établi pour les deux voxels de détecteur impliqués dans un événement de coïncidence, définissant un premier et un second voxel de détecteur ; dans lequel, dans chaque paire de voxels définie, le voxel avec le numéro atomique le plus bas reçoit le numéro 1 et celui avec le numéro atomique le plus élevé reçoit le numéro 2 ; si les deux voxels de détecteur d'une paire ont le même numéro atomique, l'étiquetage en 1 ou 2 est choisi arbitrairement ; dans lequel le dispositif est **caractérisé en ce que** le système d'acquisition de données
   c) trie les énergies $(E_1,E_2)$ mesurées dans les événements de coïncidence et les points d'interaction $(\underline{d}_1,\underline{d}_2)$ selon leur étiquetage 1,2 et les stocke dans une liste chronologique avec les attributs $\underline{y} = \{\underline{d}_1,E_1,\underline{d}_2,E_2\}$ et le temps de détection $t$ ; et

   • une unité d'analyse connectée au système d'acquisition de données, qui

   d) crée des données de projection $p(\underline{y})$ à partir des données de mesure stockées à l'étape c), qui sont définies en fonction des attributs $\underline{y} = \{\underline{d}_1,E_1,\underline{d}_2,E_2\}$, dans lequel une fonction $\sigma(E_1,E_2) = (E_2-E_1)/(E_1 + E_2)$ est utilisée, laquelle dépend de deux valeurs d'énergie $(E_1,E_2)$ ; et
   e) l'unité d'analyse effectue une reconstruction d'image qui reconstruit une ou plusieurs images $f(\underline{x})$ de la distribution source du champ de rayonnement à partir des données de projection $p(\underline{y})$.

2. Dispositif selon la revendication 1, **caractérisé en ce que** le système de détecteur détecte des champs de rayonnement présentant une distribution discrète et/ou continue de rayonnement, dans lequel le système de détecteur est situé dans le champ géométrique proche et/ou lointain du champ de rayonnement ;

   et/ou que les sources de rayonnement émettent, en plus du rayonnement gamma, des rayonnements particulaires provenant d'un groupe de particules d'électrons, de positrons, de protons, d'ions et/ou de neutrons ;
   et/ou que le rayonnement provient de la décomposition radioactive d'un ou de plusieurs radionucléides ;
   et/ou que le rayonnement est le rayonnement gamma instantané résultant de l'absorption du rayonnement protonique ou ionique dans les matériaux cibles ;
   et/ou que le rayonnement est de faible intensité.

**3.** Dispositif selon l'une des revendications précédentes, **caractérisé en ce que** les détecteurs présentent un scintillateur avec un photodétecteur et/ou un matériau semi-conducteur ;

et/ou que le scintillateur comprend un matériau pur ou dopé du groupe constitué du PVT, de l'anthracène, du stilbène, du p-terphényle, du $CaF_2$, du $BaF_2$, du NaI, du $CeBr_3$, du $LaBr_3$, du $LaCl_3$, du $La(Br_xCl_{1-x})_3$, du CsI, du $SrI_2$, du CLYC, du CLBC, du CLCB, du CLLB, du BGO, du LSO, du LYSO, du GAGG, du YAP et/ou du YAG ;
et/ou que le scintillateur est présent sous la forme d'un bloc monolithique ou d'un module scintillateur pixellisé ;
et/ou que le photodétecteur est un photomultiplicateur (PMT), un photomultiplicateur à résolution spatiale (PSPMT), un photomultiplicateur au silicium (SiPM), un photomultiplicateur au silicium à résolution spatiale (PS-SiPM) et/ou une photodiode au silicium ;
et/ou que le matériau semi-conducteur comprend un matériau du groupe constitué de Si, de Ge, de GaAs, de CdTe et/ou de CdZnTe ; et/ou que le matériau semi-conducteur présente une géométrie plane ou coaxiale et/ou est présent avec des contacts segmentés ou non segmentés ;
et/ou que le scintillateur ou le matériau semi-conducteur est virtuellement ou physiquement divisé en un nombre entier quelconque de $\geq 1$ voxels.

**4.** Dispositif selon l'une des revendications précédentes, **caractérisé en ce que** l'électronique du système utilise des composants électroniques analogiques et/ou numériques ;

et/ou **en ce que** les composants électroniques analogiques comprennent une combinaison de différents modules, y compris une alimentation haute tension, un préamplificateur, un amplificateur, un formateur d'impulsions, un intégrateur de charge, un analyseur de hauteur d'impulsion, un analyseur multicanal (MCA) et/ou un circuit de coïncidence ;
et/ou **en ce que** les composants électroniques numériques comprennent une combinaison de différents composants matériels et logiciels, y compris une alimentation haute tension, un convertisseur A/N par voxel de détecteur, un réseau de portes programmables en champ (FPGA), un support de stockage, un processeur de signaux numériques et/ou un logiciel d'évaluation.

**5.** Dispositif selon l'une des revendications précédentes, **caractérisé en ce que** les points d'interaction ($\underline{d}_1$,$\underline{d}_2$) sont déterminés sur la base des propriétés de résolution spatiale des détecteurs et/ou sur la base de la segmentation existante des détecteurs ;
et/ou que les points d'interaction ($\underline{d}_1$,$\underline{d}_2$) sont définis comme les centres spatiaux des voxels de détecteur d'un premier et d'un second voxel de détecteur impliqué dans un événement de coïncidence.

**6.** Dispositif selon l'une des revendications précédentes, **caractérisé en ce que** l'unité d'analyse utilise des données de projection $p(y)$ pour reconstruire le champ de rayonnement, lesquelles données sont définies en fonction des attributs $\underline{y} = \{\underline{d}_1,E_1,\underline{d}_2,E_2\}$ ;

et/ou que, pour chaque élément de $\underline{p}$, un certain nombre d'événements de coïncidence est déterminé, qui se produisent dans une combinaison respective d'un premier voxel de détecteur spécifique avec un deuxième voxel de détecteur spécifique à une valeur de fonction spécifique $\sigma(E_1,E_2)$ ;
et/ou que, pour chaque élément de $\underline{p}$, un certain nombre d'événements de coïncidence est déterminé, qui se produisent dans une combinaison respective d'un premier lieu de détection spécifique $\underline{d}_1$ avec un second voxel de détecteur $\underline{d}_2$ spécifique à une valeur de fonction spécifique $\sigma(E_1,E_2)$.

**7.** Dispositif selon l'une des revendications précédentes, **caractérisé en ce qu'**une condition de sélection des événements de coïncidence est appliquée par rapport à la somme d'énergie $E_1 + E_2$ des énergies détectées dans les deux voxels de détecteur d'une paire ;

et/ou que des données de projection distinctes $\underline{p}(\underline{y})$ sont créées pour chaque radionucléide détecté ;
et/ou l'unité d'analyse calcule une image distincte $\underline{f}(\underline{x})$ pour chaque radionucléide.

**8.** Dispositif selon l'une des revendications précédentes, **caractérisé en ce que** les images $\underline{f}(\underline{x})$ représentent respectivement une densité d'activité, une densité de flux et une densité de débit de dose ;
et/ou les images $\underline{f}(\underline{x})$ sont des images tomographiques en coupe d'une distribution d'activité de radionucléides (par exemple des produits radiopharmaceutiques).

**9.** Dispositif selon l'une des revendications précédentes, **caractérisé en ce que** tous les détecteurs présentent sensiblement la même conception ou qu'au moins deux des détecteurs présentent une conception différente l'une de l'autre.

**10.** Dispositif selon l'une des revendications précédentes, **caractérisé en ce que** le système de détecteur présente au moins quatre voxels de détecteur disposés sensiblement en 3 dimensions et présente un champ de vision d'un angle solide de $4\pi$ ;

ou que le système de détecteur présente au moins trois voxels de détecteur dans un agencement sensiblement bidimensionnel et possède un champ de vision hémisphérique avec un angle solide de $2\pi$ ;

ou que le système de détecteur présente au moins trois voxels de détecteur dans un agencement sensiblement bidimensionnel et présente un champ de vision de 360° dans le plan des détecteurs de rayonnement ;

ou que le système de détecteur présente au moins deux voxels de détecteur dans un réseau de détection unidimensionnel et présente un champ de vision de 180° de -90° à +90° perpendiculairement au réseau de détection.

**11.** Dispositif selon l'une des revendications précédentes, **caractérisé en ce que** le système de détecteur comprend un premier groupe de détecteurs/voxels de numéro atomique $Z_{eff} > 30$, qui présentent de fortes probabilités d'absorption photoélectrique dans la gamme d'énergie de 100 keV à 3 MeV, et éventuellement un second groupe de détecteurs/voxels de numéro atomique $Z_{eff} \leq 30$, qui présentent de fortes probabilités de diffusion Compton dans la gamme d'énergie de 100 keV à 3 MeV ;

et/ou les données sont acquises à partir de toutes les paires de détecteurs/paires de voxels qui peuvent être formées de manière combinatoire à partir de l'ensemble de tous les détecteurs/voxels, à l'exclusion des paires de détecteurs/paires de voxels dans lesquelles les deux détecteurs/voxels appartiennent au second groupe avec un nombre ordinal $Z_{eff} \leq 30$ ; et/ou les paires de détecteurs mixtes/paires de voxels, comprenant chacune un détecteur/voxel du premier groupe et un détecteur/voxel du second groupe, sont unidirectionnelles, dans lequel le rayonnement est diffusé principalement dans une direction ;

et/ou les paires de détecteurs/paires de voxels du premier groupe avec un numéro atomique $Z_{eff} > 30$ sont bidirectionnelles, de sorte que le rayonnement est diffusé dans les deux directions avec une intensité similaire.

**12.** Dispositif selon l'une des revendications précédentes, **caractérisé en ce que** la totalité des détecteurs/voxels comprenant le système de détecteur présente une première forme annulaire, tubulaire, cylindrique, sphérique et/ou polyédrique homogène ou hétérogène ;

et/ou que l'ensemble des détecteurs/voxels comprenant le système de détecteur présente, en plus de la première forme, une seconde forme annulaire, tubulaire, cylindrique, sphérique et/ou polyédrique homogène ou hétérogène, dans lequel la seconde forme est inscrite dans la première forme ou dans laquelle la seconde forme est disposée dans une région intérieure que la première forme entoure comme une coque extérieure ;

et/ou dans lequel la première et/ou la seconde forme est disposée autour d'un ou de plusieurs détecteurs centraux.

**13.** Dispositif selon l'une des revendications précédentes, **caractérisé en ce qu'**une partie du système ou l'ensemble du système est conçu comme un dispositif de localisation de sources de rayonnement, comme une caméra Compton, comme un télescope Compton, comme un scanner SPECT, comme un scanner hybride PET/SPECT, comme une sonde SPECT et/ou comme une sonde hybride PET/SPECT.

**14.** Procédé d'utilisation d'un dispositif de génération d'une ou de plusieurs images d'une distribution de source d'un champ de rayonnement gamma selon l'une quelconque des revendications 1 à 13, dans lequel les matériaux détecteurs sont segmentés virtuellement ou physiquement en un nombre quelconque de $\geq 1$ voxel, le procédé utilise des processus de diffusion Compton unidirectionnels et bidirectionnels et présente une matrice système $\underline{H}$, une valeur de fonction définie $\sigma(E_1,E_2)$ et une liste de paires de voxels définies, et est configuré pour l'acquisition de données de projection $\underline{p}(\underline{y})$ des valeurs mesurées et pour le calcul de données d'image $\underline{f}(\underline{x})$, et comprend les étapes suivantes :

• l'établissement d'une liste de paires de voxels définies, dans lequel les paires de voxels définies comprennent toutes les paires formées de manière combinatoire à partir de l'ensemble des voxels de détecteur et chaque paire contient au moins un voxel de détecteur constitué d'un matériau avec un numéro atomique de $Z_{eff} > 30$ ;

• la commutation de tous les détecteurs/voxels dans un circuit de coïncidence de sorte que les événements de coïncidence sont enregistrés dans toutes les paires de voxels définies ;

• l'identification des deux voxels de détecteur de chaque paire de voxels par les numéros 1 et 2, dans lequel le voxel de détecteur avec le numéro atomique le plus bas reçoit le numéro 1, et celui avec le numéro atomique le plus élevé reçoit le numéro 2, si les deux voxels de détecteur sont constitués du même matériau, l'identification est aléatoire ;

• la définition d'une fonction $\sigma(E_1,E_2) = (E_2 - E_1)/(E_1 + E_2)$ étant calculée à partir de deux valeurs d'énergie $(E_1,E_2)$ ;

• l'acquisition des valeurs mesurées $\underline{y} = \{\underline{d}_1,E_1,\underline{d}_2,E_2\}$ des événements de coïncidence lorsque des interactions se produisent simultanément dans deux voxels de détecteur de toutes les paires de voxels définies, dans lequel les valeurs mesurées proviennent d'un champ proche ou lointain de rayonnement et les valeurs mesurées sont les énergies $(E_1,E_2)$ mesurées dans les voxels de détecteur et les points d'interaction $(\underline{d}_1,\underline{d}_2)$,

• l'association d'événements de coïncidence $\underline{y} = \{\underline{d}_1,E_1,\underline{d}_2,E_2\}$ à un premier voxel de détecteur/emplacement de détection $\underline{d}_1$ et à un second voxel de détecteur/emplacement de détection $\underline{d}_2$ ;

• le calcul de la valeur de fonction $\sigma(E_1,E_2)$ à partir de deux valeurs d'énergie $(E_1,E_2)$ par événement de coïncidence ;

• la détection des événements de coïncidence en fonction de leur premier voxel de détecteur $\underline{d}_1$, de leur deuxième voxel de détecteur $\underline{d}_2$ et de leur valeur $\sigma(E_1,E_2)$ dans un élément des données de projection $\underline{p}(\underline{y})$, dans lequel des données de projection distinctes $\underline{p}(\underline{y})$ sont détectées pour chaque radionucléide ;

• le calcul d'une ou de plusieurs images $\underline{f}(\underline{x})$ à partir des données de projection $\underline{p}(\underline{y})$ à l'aide d'un procédé de reconstruction d'image statistique de tomographie par émission à l'aide de la matrice système $\underline{H}$, dans lequel une image distincte $\underline{f}(\underline{x})$ est calculée pour chaque radionucléide ; les images $\underline{f}(\underline{x})$ représentent une distribution d'activité dans un volume source ou une distribution de densité de flux sur les directions d'incidence.

15. Procédé selon la revendication 14, dans lequel le procédé comprend en outre, en partie ou en totalité, les étapes suivantes :

• l'étalonnage des signaux de tous les voxels de détecteur en tant qu'énergie rayonnante absorbée $E$ ;

• la définition d'un système de coordonnées approprié ;

• la division de la plage de mesure pour la valeur de fonction $\sigma(E_1,E_2)$ en canaux de valeur mesurée équidistants ;

• la création d'un ou de plusieurs ensembles de données de projection $\underline{p}(\underline{y})$ qui enregistrent le nombre d'événements de coïncidence qui sont comptés pour une combinaison respective d'un premier voxel de détecteur spécifique $\underline{d}_1$ avec un second voxel de détecteur spécifique $\underline{d}_2$ à une valeur de fonction spécifique $\sigma(E_1,E_2)$, dans lequel un ensemble de données de projection distinct $\underline{p}(\underline{y})$ est créé pour chaque radionucléide ;

• la création d'une ou de plusieurs matrices système $\underline{H}$ pour chaque radionucléide à détecter ;

• la création et validation des matrices du système $\underline{H}$ par des mesures avec le système détecteur ou par des simulations de Monte Carlo ou au moyen d'un modèle théorique ;

• la transmission de toutes les matrices système $\underline{H}$ à un algorithme de reconstruction d'image, qui traite les données de projection $\underline{p}(\underline{y})$ et calcule les images $\underline{f}(\underline{x})$.

**Bildgebende Detektorsysteme**

**Typ A**

• bestehen aus Detektoren zweier Gruppen, eine Detektorgruppe ist aus Materialien mit $Z_{eff} > 30$, die andere aus Materialien mit $Z_{eff} \leq 30$ zusammengesetzt,

• jeder Detektor einer Gruppe kann mit jedem Detektor der anderen Gruppe zu einem Paar kombiniert werden,

• die Detektorpaare sind unidirektional,

• die Detektoren der Gruppe mit $Z_{eff} > 30$ bilden untereinander zusätzlich ein Detektorsystem vom Typ B

**Typ B**

• sind aus gleichartigen Detektoren mit $Z_{eff} > 30$ aufgebaut, die Materialien können gleich oder unterschiedlich sein,

• jeder Detektor kann mit jedem anderen zu einem Paar kombiniert werden,

• die Detektorpaare sind bidirektional

**Typ A *getrennt***

• beide Detektorgruppen sind räumlich voneinander getrennt

**Typ A *gemischt***

• beide Detektorgruppen sind räumlich miteinander gemischt

Abb. 1

Typ A
*getrennt*

Typ A
*gemischt*

Typ B

Abb. 2a

Abb. 2b

Abb. 2c

Abb. 3

(a)

(b)

$$\sigma = \frac{E2 - E1}{E1 + E2}$$

Abb. 4a

Abb. 4b

(a)            (b)            (c)

Abb. 5a        Abb. 5b        Abb. 5c

(a)            (b)

$$\sigma = \frac{E2 - E1}{E1 + E2}$$

|  | P1 | P2 | P3 |
|---|---|---|---|
| - 1,0 |  |  |  |
| - 0,9 | 0 | 3 | 1 |
| - 0,8 | 85 | 55 | 49 |
| - 0,7 | 137 | 119 | 107 |
| - 0,6 | 67 | 72 | 81 |
| - 0,5 | 48 | 58 | 60 |
| - 0,4 | 46 | 92 | 113 |
| - 0,3 | 24 | 147 | 114 |
| - 0,2 | 28 | 107 | 85 |
| - 0,1 | 27 | 77 | 44 |
| 0,0 | 20 | 49 | 23 |
| + 0,1 | 32 | 30 | 7 |
| + 0,2 | 31 | 10 | 2 |
| + 0,3 | 47 | 8 | 4 |
| + 0,4 | 75 | 10 | 11 |
| + 0,5 | 108 | 16 | 20 |
| + 0,6 | 181 | 26 | 32 |
| + 0,7 | 251 | 46 | 42 |
| + 0,8 | 322 | 58 | 69 |
| + 0,9 | 314 | 43 | 35 |
| + 1,0 | 28 | 5 | 0 |

Abb. 6a        Abb. 6b

(a)                    (b)                    (c)                    (d)

Abb. 7a            Abb. 7b            Abb. 7c            Abb. 7d

(a)                                        (b)

$$\sigma = \frac{E2 - E1}{E1 + E2}$$

|       | P1  | P2  | P3  | P4  |
|-------|-----|-----|-----|-----|
| - 1,0 | 4   | 8   | 10  | 23  |
| - 0,9 | 7   | 5   | 19  | 57  |
| - 0,8 | 14  | 11  | 47  | 33  |
| - 0,7 | 53  | 72  | 52  | 68  |
| - 0,6 | 41  | 58  | 60  | 71  |
| - 0,5 | 22  | 62  | 75  | 90  |
| - 0,4 | 38  | 78  | 114 | 66  |
| - 0,3 | 54  | 98  | 128 | 53  |
| - 0,2 | 66  | 77  | 148 | 58  |
| - 0,1 | 78  | 63  | 167 | 42  |
| 0,0   | 125 | 121 | 170 | 31  |
| + 0,1 | 136 | 288 | 152 | 29  |
| + 0,2 | 121 | 301 | 121 | 16  |
| + 0,3 | 134 | 450 | 110 | 22  |
| + 0,4 | 159 | 426 | 104 | 17  |
| + 0,5 | 211 | 387 | 94  | 28  |
| + 0,6 | 311 | 322 | 87  | 36  |
| + 0,7 | 371 | 239 | 69  | 24  |
| + 0,8 | 414 | 164 | 35  | 18  |
| + 0,9 | 254 | 53  | 12  | 15  |
| + 1,0 |     |     |     |     |

Abb. 8a                                    Abb. 8b

(a)

2-Ebenen Compton Kamera

(b)

Zusätzliche Detektorpaare in einem
Typ A *getrennt* Detektorsystem

Abb. 9a (Stand der Technik)

Abb.9b

Detektorsystem mit 2-dimensionaler
Detektorgruppe

(a) für halbsphärische Aufnahmen
von Strahlungsquellen

(b) für Aufnahmen von Strahlungsquellen
in der Detektorebene

Abb. 10a

Abb. 10b

Typ A

Typ B

Abb. 11a

Abb. 11b

Weitere Varianten von Typ A Detektorsystemen

(a)

(b)

(c)

Abb. 12

Weitere Varianten von Typ B Detektorsystemen

(a)

(b)

Abb. 13a

Abb. 13b

**Detektorpaar aus CeBr₃ und Plastik**

Maximale Wahrscheinlichkeit für Compton Einfachstreuung

Maximale Gesamtwahrscheinlichkeit

Optimale Szintillatordicke für CeBr3 und Plastik

Abb. 14

Abb. 15

Abb. 16a

Abb. 16b

Abb. 16c

Quelle

Abb. 17

Abb. 18

Abb. 19

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20120114100 A

- US 20120043467 A
- US 9638811 B2

**In der Beschreibung aufgeführte Nicht-Patentliteratur**

- *Nuclear Instruments & Methods*, 1973, vol. 107, 385
- *Nature*, 1974, vol. 251, 5471

- **SHEPP** ; **VARDI**. *IEEE Transactions on Medical Imaging*, 1982, vol. 1, 113